# EUROPEAN PATENT APPLICATION

(11) **EP 4 803 549 A1**
(43) Date of publication of application: **09.09.2026**
(21) Application number: 24884745.1
(22) Date of filing: 29.10.2024
(51) Int. Cl.: C07K 19/00, C07K 16/28, A61K 39/395, A61K 47/68, A61P 35/00

(54) **ANTIBODY BINDING TO BCMA AND MULTISPECIFIC ANTIBODY COMPRISING SAME**

(30) Priority: 30.10.2023 WO PCT/CN2023/127818
(71) Applicant: Leads Biolabs Inc., Dover, DE 19901 (US)
(72) Inventor: SUN, Jianming, Nanjing, Jiangsu 210019 (CN); DING, Mi, Nanjing, Jiangsu 210019 (CN); QIN, Yurong, Nanjing, Jiangsu 210019 (CN); HUANG, Xiao, Nanjing, Jiangsu 210019 (CN); LING, Hong, Nanjing, Jiangsu 210019 (CN)
(74) Representative: Bianchetti & Minoja with Trevisan & Cuonzo IPS SRL
(86) International application number: PCT/CN2024/128190
(87) International publication number: WO 2025/092742

(57) **Abstract**

Provided are a monoclonal antibody specifically binding to BCMA, a BCMA and CD3 bispecific antibody, and a BCMA/GPRC5D/CD3 trispecific antibody constructed based on the BCMA monoclonal antibody. Further provided are a nucleic acid molecule encoding the antibodies, an expression vector for use in expressing the antibodies, a host cell, and a method. Further provided are an immunoconjugate, a bispecific molecule, a trispecific molecule, and a pharmaceutical composition comprising the antibodies, as well as a diagnostic and therapeutic method using the anti-BCMA antibody, the BCMA/CD3 bispecific antibody, and the BCMA/GPRC5D/CD3 trispecific antibody of the present disclosure.

## Description

### TECHNICAL FIELD

Provided are a monoclonal antibody specifically binding to BCMA, a BCMA and CD3 bispecific antibody, and a BCMA/GPRC5D/CD3 trispecific antibody constructed based on the BCMA monoclonal antibody. Further provided are a nucleic acid molecule encoding the antibodies, an expression vector for use in expressing the antibodies, a host cell, and a method. Further provided are an immunoconjugate, a bispecific molecule, a trispecific molecule, and a pharmaceutical composition comprising the antibodies, as well as a diagnostic and therapeutic method using the anti-BCMA antibody, the BCMA/CD3 bispecific antibody, and the BCMA/GPRC5D/CD3 trispecific antibody of the present disclosure.

### BACKGROUND

B-cell maturation antigen (BCMA, also known as CD269) is a member of the TNF receptor superfamily. BCMA expression is restricted to the B-cell lineage, where it is primarily expressed in the interfollicular regions of germinal centers, as well as on differentiated plasma cells and plasmablasts. BCMA binds to two distinct ligands, a proliferation-inducing ligand (APRIL) and B-cell activating factor (BAFF, also known as BlyS, TALL-1, and THANK). The ligands of BCMA bind to two additional TNF receptors: transmembrane activator-calcium modulator-cyclophilin ligand interactor (TACI) and BAFF receptor (BAFF-R, also known as BR3). TACI binds to both APRIL and BAFF, whereas BAFF-R exhibits a restricted but high-affinity binding to BAFF. Collectively, BCMA, TACI, BAFF-R, and their corresponding ligands regulate various aspects of humoral immunity, B-cell development, and homeostasis. BCMA is nearly absent in naive and memory B cells (Novak et al., Blood, 103: 689-694 (2004)) but BCMA expression is selectively induced during plasma cell differentiation. During this process, BCMA supports humoral immunity by promoting the survival of normal plasma cells and plasmablasts (O'Connor et al., J. Exp. Med., 199: 91-98 (2004)). BCMA expression has also been reported in primary multiple myeloma (MM) samples.

G protein-coupled receptor family C group 5 member D (GPRC5D), as an orphan receptor, has been found to be highly expressed in plasma cells of patients with multiple myeloma and associated with the survival rate of patients (Atamaniuk, J., et al., (2012). "Overexpression of G protein-coupled receptor 5D in the bone marrow is associated with poor prognosis in patients with multiple myeloma." Eur J Clin Invest 42(9): 953-960.). Further studies have shown that in normal human blood cells, GPRC5D is expressed on the surface of plasma cells, while other blood cell types exhibit no surface expression of GPRC5D. CD38+CD138+ plasma cells isolated from patients with multiple myeloma were found to exhibit high surface expression of GPRC5D (Kodama, T., et al., (2019). "Anti-GPRC5D/CD3 Bispecific T-Cell-Redirecting Antibody for the Treatment of Multiple Myeloma." Mol Cancer Ther 18(9): 1555-1564.). Analysis of multiple myeloma cells from patients further revealed that the expression of GPRC5D is not correlated with the expression of BCMA (Smith, E. L., et al., (2019). "GPRC5D is a target for the immunotherapy of multiple myeloma with rationally designed CAR T cells." Sci Transl Med 11(485).) (Pillarisetti, K., et al., (2020). "A T-cell-redirecting bispecific G-protein-coupled receptor class 5 member D x CD3 antibody to treat multiple myeloma." Blood 135(15): 1232-1243.). Thus, GPRC5D can serve as a tumor-specific target for multiple myeloma. In addition, GPRC5D can also serve as a tumor-specific target for other tumors.

CD3 is a homodimeric or heterodimeric antigen expressed on T cells, which binds to the T cell receptor (TCR) complex and is required for the activation of T cells. Functional CD3 is formed by dimeric association of two of four different chains: ε, ζ, δ, and γ. The CD3 dimer arrangements include γ/ε, δ/ε, and ζ/ζ. Antibodies directed against CD3 have been shown to cluster CD3 on T cells, thereby causing the activation of T cells in a manner similar to the engagement of the TCR by peptide-loaded MHC molecules. Thus, anti-CD3 antibodies have been proposed for therapeutic purposes involving the activation of T cells. In addition, it has been proposed that bispecific antibodies capable of binding to CD3 and targeting tumor surface antigens are capable of engaging tumor cells with T cells, thereby directly activating the T cells, releasing granzymes, perforins, and cytokines to kill tumors, and then achieving the therapeutic purpose of inhibiting the tumors.

T cell-based antibody therapies play an important role in combating various cancers, including multiple myeloma (MM). Bispecific antibodies (BsAbs) are designed to target two distinct antigens: one arm binds to a relevant antigen on tumor cells, while the other arm engages the CD3 co-receptor complex on T cells. This dual-targeting design redirects cytotoxic T cells and brings them into proximity with tumor cells, facilitating tumor cell lysis. The tumor-lysing efficacy of BsAbs depends on the expression pattern and abundance of the tumor-associated antigens on tumor cells. This leads to limitations in patient selectivity and treatment with BsAbs treatment. It is therefore possible that the expression of tumor-associated antigens is down-regulated, and then the immune selection pressure is reduced, resulting in immune escape and thus disease relapse.

Since BCMA and GPRC5D are tumor-specific antigens expressed on the surface of multiple myeloma (MM) tumor cells, MM patients exhibit high levels of tumor cell heterogeneity, the expression of BCMA and GPRC5D on MM cells is relatively independent, and BCMA-positive or GPRC5D-positive MM cells are present in patients. When treated with BCMA×CD3 BsAb or GPRC5D×CD3 BsAb, MM cells negative for BCMA or GPRC5D cannot be completely eradicated, ultimately resulting in disease relapse.

Although the prior art includes antibodies targeting BCMA and bispecific or multispecific antibodies constructed based on BCMA antibodies or fragments, such as Teclistamab (US2017/0051068A1), which has demonstrated high safety and provided deep, durable remission for MM patients who have failed multiple lines of therapy, and is now in Phase III clinical trials. Other BCMA/CD3 bispecific antibodies such as AMG420 and REGN5458 are also under clinical investigation. However, known antibodies still have areas in need of improvement, such as weak ability to induce T cell killing, high cytokine release levels, low affinity for human BCMA-expressing cells, inability to effectively clear tumor cell lines with low human BCMA expression, specific induction of non-specific killing of BCMA-negative cells by T cells, or development of BCMA expression mutations and downregulation following treatment.

Therefore, there is a need for a novel BCMA antibody and a corresponding anti-GPRC5D/BCMA/CD3 trispecific antibody, which can overcome the aforementioned disadvantages, induce optimal T cell killing, and result in lower cytokine release levels.

### SUMMARY

The present disclosure provides, in a first aspect, a novel anti-BCMA antibody, such as a VHH antibody or an antibody comprising a VHH, e.g., VHH-Fc, and a bispecific antibody binding to CD3 constructed using the same.

In some embodiments, the anti-BCMA antibody specifically binds to BCMA (e.g., human BCMA) with a high affinity. In some embodiments, the anti-BCMA antibody binds to a BCMA-expressing cell, e.g., a tumor cell that endogenously expresses BCMA.

The anti-CD3/BCMA bispecific antibody of the present disclosure has one or more of the following properties:
(1) binding to human BCMA, e.g., binding to human BCMA expressed on a cell, such as binding to BCMA with a high affinity;
(2) specifically inducing T cells to kill BCMA-positive tumor cells and/or having no (e.g., significant) non-specific killing effect on BCMA-negative cells;
(3) having better cytokine induction specificity, e.g., inducing cells (e.g., T cells) to release cytokines, e.g., interferons such as IFNγ, tumor necrosis factors such as TNFα, and/or interleukins such as IL-6 only in cells expressing BCMA (e.g., BCMA-positive tumor cells);
(4) the trend of inducing cytokine release being consistent with the killing results of BCMA-positive cells, e.g., the stronger the killing effect, the higher the cytokine release; and
(5) effective in treating BCMA-related indications.

The present disclosure further provides a novel anti-GPRC5D antibody, such as a VHH antibody or an antibody comprising a VHH, e.g., VHH-Fc, and a multispecific antibody binding to BCMA and CD3 constructed using the same.

The present disclosure further provides a multispecific antibody targeting both BCMA and GPRC5D, enabling treatment of both BCMA-positive patients and GPRC5D-positive patients. Additionally, the antibody can prevent disease relapse caused by immune escape resulting from the down-regulation or loss of expression of a single target, thereby improving therapeutic efficacy and providing clinical benefits.

Therefore, a BCMA/GPRC5D/CD3 trispecific antibody is constructed in the present disclosure. By adjusting the positions of the CD3 antibody and the BCMA/GPRC5D antibody, various combinations are performed, and after screening, the trispecific antibody that induces optimal T cell killing while maintaining lower levels of cytokine release is obtained.

In some embodiments, the anti-BCMA/GPRC5D/CD3 trispecific antibody of the present disclosure has one or more of the following properties:
(1) specifically binding to human BCMA, human GPRC5D, and human CD3, e.g., binding to proteins on the surface of cells expressing human BCMA and human GPRC5D proteins (such as tumor cells endogenously expressing human BCMA and human GPRC5D), e.g., not binding to other GPRC family members other than GPRC5D, such as not binding to GPRC5A, GPRC5B, and GPRC5C, or binding to them with a very weak affinity;
(2) specifically inducing T cells to kill BCMA and/or GPRC5D-positive tumor cells;
(3) specifically activating T cells (e.g., as indicated by increased expression of CD25 and/or CD69), such as superior to known control antibodies, and promoting T cell apoptosis (e.g., as indicated by increased expression of PD1 and/or TIM3);
(4) having better cytokine induction specificity, e.g., inducing cells (e.g., T cells) to release cytokines, e.g., interferons such as IFNγ, tumor necrosis factors such as TNFα, and/or interleukins such as IL-6 only in cells expressing human BCMA and/or human GPRC5D (e.g., BCMA and/or GPRC5D-positive tumor cells);
(4) the trend of inducing cytokine release being consistent with the killing results of cells expressing human BCMA and/or GPRC5D (BCMA-positive and/or GPRC5D-positive), e.g., the stronger the killing effect, the higher the cytokine release;
(5) specifically inducing T cells to kill tumor cells expressing human BCMA and/or GPRC5D (BCMA-positive and/or GPRC5D-positive) (e.g., greater killing activity than the control) without non-specific killing against BCMA- and GPRC5D-negative cells;
(6) having no (e.g., significant) function of promoting T cell cytokine release in BCMA- and GPRC5D-negative cells;
(7) specifically binding to tumor cells expressing human BCMA and/or GPRC5D (BCMA-positive and/or GPRC5D-positive) and having greater binding activity than the control; and
(8) treating tumors *in vivo*, e.g., inhibiting tumor growth and/or not affecting animal body weight.

### BRIEF DESCRIPTION OF THE DRAWINGS

FIG. 1 shows the structural diagrams of bispecific antibodies.
FIG. 2 shows the binding of anti-human BCMA chimeric antibodies to a cell line expressing human BCMA proteins.
FIG. 3 shows the binding of anti-human BCMA humanized antibodies to a tumor cell line expressing human BCMA proteins.
FIG. 4 shows the binding of bispecific antibodies to a cell line expressing human BCMA proteins.
FIG. 5 shows the specific killing of tumor cell lines expressing human BCMA proteins by bispecific antibodies.
FIG. 6 shows the cytokine release in a killing system targeting a tumor cell line expressing human BCMA proteins by bispecific antibodies.
FIG. 7 shows the structural diagrams of trispecific antibodies.
FIG. 8 shows the binding of trispecific antibodies to tumor cell lines expressing human BCMA proteins.
FIG. 9 shows the killing of tumor cell lines expressing human BCMA proteins by trispecific antibodies.
FIG. 10 shows the detection results for the markers of the activation and exhaustion of T cells in killing systems targeting tumor cell lines expressing human BCMA proteins by trispecific antibodies.
FIG. 11 shows the cytokine release in killing systems targeting tumor cell lines expressing human BCMA proteins by trispecific antibodies.
FIG. 12 shows the results of killing targeting a multiple myeloma tumor cell line MOLP8 by trispecific antibodies as well as control diabodies alone or in combination.
FIG. 13 shows the TNFα release in killing systems targeting a multiple myeloma cell line MOLP8 by trispecific antibodies and by control diabodies alone or in combination.
FIG. 14 shows the binding of trispecific antibodies as well as control diabodies and trispecific antibodies to multiple myeloma tumor cell lines ARD and AMO-1.
FIG. 15 shows the results of killing targeting a multiple myeloma tumor cell line ARD by trispecific antibodies as well as control diabodies and trispecific antibodies.

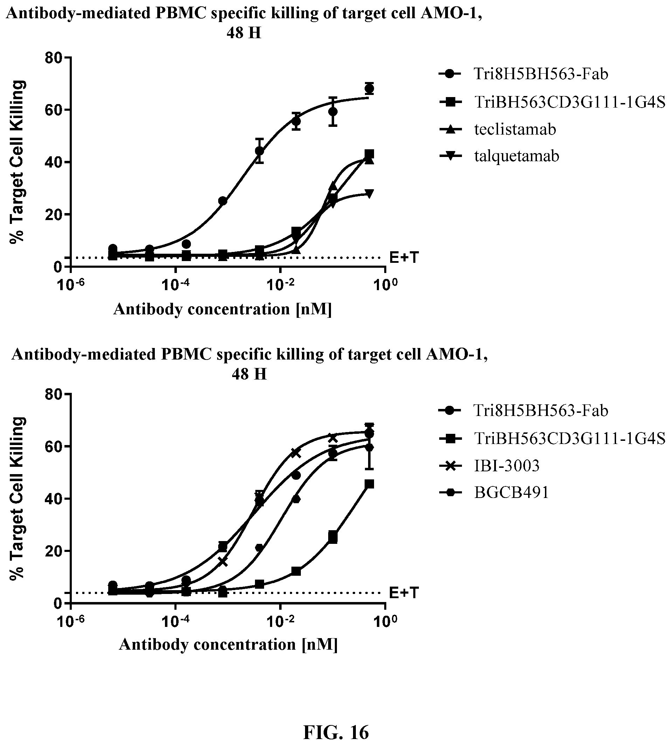

FIG. 16 shows the results of killing targeting a multiple myeloma tumor cell line AMO-1 by trispecific antibodies as well as control diabodies and trispecific antibodies.
FIG. 17 shows the TNFα release in a killing system targeting a multiple myeloma tumor cell line ARD by trispecific antibodies as well as control diabodies and trispecific antibodies.
FIG. 18 shows the TNFα release in a killing system targeting a multiple myeloma cell line AMO-1 by trispecific antibodies as well as control diabodies and trispecific antibodies.
FIG. 19 shows the tumor inhibitory effect of a trispecific antibody in an NCI-H929 tumor-bearing humanized mouse model.
FIG. 20 shows the tumor inhibitory effect of trispecific antibodies in a MOLP8 tumor-bearing humanized mouse model.
FIG. 21 shows the detection of the binding of a camelid-derived anti-GPRC5D VHH antibody to human GPRC5D proteins.
FIG. 22 shows the detection of the binding of a camelid-derived anti-GPRC5D VHH antibody to cynomolgus monkey GPRC5D proteins.
FIG. 23 shows the detection of the binding of a humanized anti-GPRC5D VHH-Fc antibody to GPRC5D.
FIG. 24 shows the detection of the binding of a humanized anti-GPRC5D VHH antibody to GPRC5A.
FIG. 25 shows the detection of the binding of a humanized anti-GPRC5D VHH antibody to GPRC5B.
FIG. 26 shows the detection of the binding of a humanized anti-GPRC5D VHH antibody to GPRC5C.

### DETAILED DESCRIPTION OF THE PRESENT DISCLOSURE

It should be understood that the present disclosure is not limited to the particular methodology, protocols, and reagents described herein, as these may vary. It should also be understood that the terminology used herein is only intended to describe specific embodiments rather than limit the scope of the present disclosure, which will be limited only by the appended claims.

### I. Definition

For the purpose of explaining this description, the following definitions will be used, and wherever appropriate, terms used in the singular form may also include the plural form, and vice versa. Unless otherwise defined, any technical and scientific term used herein has the same meaning as commonly understood by those of ordinary skill in the art to which the present disclosure belongs.

The term "about" used in combination with a numerical value is intended to encompass the numerical values in a range from a lower limit less than the specified numerical value by 5% to an upper limit greater than the specified numerical value by 5%.

As used herein, the term "and/or" refers to any one of the options or any two or more or all of the options.

As used herein, the term "comprise" or "include" is intended to mean that the elements, integers, or steps are included, but not to the exclusion of any other elements, integers, or steps. The term "comprise" or "include" used herein, unless indicated otherwise, also encompasses the situation where the entirety consists of the described elements, integers, or steps. For example, when referring to an antibody variable region "comprising" a specific sequence, it is also intended to encompass an antibody variable region consisting of the specific sequence.

When referring to "first" and "second" herein, it is only to distinguish two domains or two chains, and does not indicate the location of the two domains in any way.

The term "BCMA" as used herein refers to the tumor-associated antigen B cell maturation antigen, also known as BCMA, TR17_human, and TNFRSF17 (e.g., human BCMA protein under UniProt accession No. Q02223). In one embodiment, the human BCMA protein of the present disclosure comprises or consists of the amino acid sequence set forth in SEQ ID NO: 77 (or the sequence without a human Fc tag), or an amino acid sequence having at least 85%, 90%, 91%, 92%, 93%, 94%, 95%, 96%, 97%, 98%, or 99% identity thereto. In one embodiment, an antigen-binding region binding to BCMA in the anti-BCMA antibody or the antigen-binding fragment thereof or the trispecific antibody of the present disclosure has high-affinity binding activity for cells expressing human BCMA, e.g., has higher binding affinity than that of a control antibody (e.g., 269B094). In one embodiment, the assay is performed by flow cytometry.

The term "CD3" as used herein refers to an antigen expressed on a T cell as part of a multimolecular T cell receptor (TCR), i.e., a T-cell engaging antigen, T-cell surface glycoprotein CD3, which consists of a homodimer or heterodimer formed from two of the following four receptor chains: CD3-ε, CD3-δ, CD3-ζ, and CD3-γ. Human CD3-εn (hCD3ε) comprises an amino acid sequence described in UniProtKB/Swiss-Prot: P07766. Human CD3-δ (hCD3δ) comprises an amino acid sequence described in UniProtKB/Swiss-Prot: P04234. In some embodiments, the CD3 described herein refers to CD3 from a human or monkey (e.g., cynomolgus monkey).

The term "antibody binding to CD3" or "anti-CD3 antibody" as used herein includes an antibody and an antigen-binding fragment thereof that specifically recognize or bind to a single CD3 subunit (e.g., ε, δ, γ, or ζ), as well as an antibody and an antigen-binding fragment thereof that specifically recognize and bind to a dimeric complex of two CD3 subunits (e.g., γ/ε, δ/ε, and ζ/ζ CD3 dimers). The antibody and the antigen-binding fragment of the present disclosure may bind to soluble CD3, binding CD3, and/or CD3 expressed on the cell surface. The soluble CD3 includes native CD3 proteins and recombinant CD3 protein variants, e.g., monomeric and dimeric CD3 structures that lack a transmembrane region or otherwise do not bind to the cell membrane. In one embodiment, the antigen-binding region binding to CD3 in the multispecific antibody of the present disclosure may have relatively low binding activity to CD3 or cells expressing CD3 (e.g., T cells). The binding affinity of the antibody to CD3 may be detected by flow cytometry or bio-layer interferometry. Preferably, in the trispecific antibody molecule of the present disclosure, the antigen-binding region binding to CD3 has a binding affinity of 1-1000 nM for human or monkey (cynomolgus monkey) CD3. In some embodiments, the antigen-binding region binding to CD3 in the trispecific antibody of the present disclosure binds to human and/or monkey (e.g., cynomolgus monkey) CD3 with relatively low binding affinity, thereby enabling the activation of human and/or monkey (e.g., cynomolgus monkey) T cells.

Effector cells include effector T cells (T lymphocytes), such as CD4+ T cells, CD8+ T cells, Th1, Th2, and regulatory T cells (Tregs). The effector cells may also include natural killer cells, macrophages, granulocytes, plasma cells, or B cells (lymphocytes).

The term "GPRC5D" refers to a tumor-associated antigen, G protein-coupled receptor family C group 5 member D (e.g., human GPRC5D protein under accession number NP_061124.1 or cynomolgus monkey GPRC5D protein under accession number XP_005570249.2). In one embodiment, the human GPRC5D protein of the present disclosure comprises or consists of the amino acid sequence set forth in SEQ ID NO: 108, or an amino acid sequence having at least 85%, 90%, 91%, 92%, 93%, 94%, 95%, 96%, 97%, 98%, or 99% identity thereto. In one embodiment, an antigen-binding region binding to GPRC5D in the trispecific antibody of the present disclosure has high-affinity binding activity for cells expressing human GPRC5D, e.g., has higher binding affinity than that of a control antibody (e.g., GC5B596). In one embodiment, the assay is performed by flow cytometry. In one embodiment, the antigen-binding region binding to GPRC5D in the trispecific antibody of the present disclosure has cross-reactivity to human and monkey (e.g., cynomolgus monkey) GPRC5D, i.e., it is capable of binding to human and monkey (e.g., cynomolgus monkey) GPRC5D.

The terms "whole antibody", "full-length antibody", "complete antibody", and "intact antibody" are used interchangeably herein to refer to a naturally occurring glycoprotein comprising at least two heavy (H) chains and two light (L) chains interconnected by disulfide bonds. Each heavy chain consists of a heavy chain variable region (abbreviated herein as VH) and a heavy chain constant region. The heavy chain constant region consists of 3 domains, CH1, CH2, and CH3. Each light chain consists of a light chain variable region (abbreviated herein as VL) and a light chain constant region. The light chain constant region consists of one domain CL. The VH region and the VL region can be further divided into hypervariable regions (complementarity determining regions, or CDRs), with relatively conservative regions (framework regions, or FRs) inserted therebetween. Each VH or VL consists of three CDRs and four FRs, arranged from amino-terminus to carboxyl-terminus in the following order: FR1, CDR1, FR2, CDR2, FR3, CDR3, and FR4. The constant regions are not directly involved in the binding of antibodies to antigens, but exhibit a variety of effector functions. In some embodiments, the antibody heavy chain constant region HC of the present disclosure is a heavy chain constant region of IgG1, IgG2, IgG3 or IgG4, preferably a heavy chain constant region of IgG1. In some embodiments, the heavy chain constant region comprises an LALA mutation. In some embodiments, the heavy chain constant region comprises a D265A mutation and a P329A mutation. In some embodiments, the heavy chain constant region comprises an LALA mutation, a D265A mutation, and a P329A mutation. In some embodiments, the heavy chain constant region of the multispecific antibody molecule of the present disclosure comprises a "knob into hole" mutation. In some preferred embodiments, the antibody heavy chain constant region HC of the present disclosure
(i) comprises or consists of an amino acid sequence having at least 85%, 90%, 91%, 92%, 93%, 94%, 95%, 96%, 97%, 98%, or 99% identity to an amino acid sequence selected from SEQ ID NO: 89 or 92;
(ii) comprises or consists of an amino acid sequence selected from SEQ ID NO: 89 or 92; or
(iii) comprises or consists of an amino acid sequence having one or more (preferably not more than 20 or 10, and more preferably not more than 5, 4, 3, 2, or 1) amino acid modifications (preferably amino acid substitutions, and more preferably conservative amino acid substitutions) compared to an amino acid sequence selected from SEQ ID NO: 89 or 92.

In some embodiments, the antibody light chain constant region LC of the present disclosure is a lambda or kappa light chain constant region. In some embodiments, the antibody light chain constant region LC of the present disclosure
(i) comprises or consists of an amino acid sequence having at least 85%, 90%, 91%, 92%, 93%, 94%, 95%, 96%, 97%, 98%, or 99% identity to an amino acid sequence selected from SEQ ID NO: 91 or 93;
(ii) comprises or consists of an amino acid sequence selected from SEQ ID NO: 91 or 93; or
(iii) comprises or consists of an amino acid sequence having one or more (preferably not more than 20 or 10, and more preferably not more than 5, 4, 3, 2, or 1) amino acid modifications (preferably amino acid substitutions, and more preferably conservative amino acid substitutions) compared to an amino acid sequence selected from SEQ ID NO: 91 or 93.

The term "antibody fragment" comprises a portion of an intact antibody. In a preferred embodiment, the antibody fragment is an antigen-binding fragment.

The term "antigen-binding fragment" is a portion or segment of an intact antibody or a complete antibody that has fewer amino acid residues than the intact antibody or the complete antibody, which can bind to an antigen or compete with an intact antibody (i.e., an intact antibody from which the antigen-binding fragment is derived) for binding to an antigen. The antigen-binding fragment may be prepared by recombinant DNA techniques, or by enzymatic or chemical cleavage of an intact antibody. The antigen-binding fragments include, but are not limited to, Fab, Fab', F(ab')2, Fv, single-chain Fv, diabodies, and single-domain antibodies (sdAbs). The Fab fragment is a monovalent fragment consisting of VL, VH, CL, and CH1 domains and can be obtained, for example, by papain digestion of a complete antibody. In addition, the F(ab')2, a dimer of the Fab', is a bivalent antibody fragment produced by pepsin digestion of a portion below disulfide bonds in a hinge region of a complete antibody. The F(ab')2 can be reduced by disrupting the disulfide bonds in the hinge region under neutral conditions, and the F(ab')2 dimer is thus converted into Fab' monomers. The Fab' monomer is substantially a Fab fragment with a hinge region (for more detailed descriptions of other antibody fragments, see Fundamental Immunology, edited by W. E. Paul, Raven Press, N.Y. (1993)). The Fv fragment consists of the VL and VH domains of a single arm of an antibody. In addition, although the two domains VL and VH of the Fv fragment are encoded by separate genes, the domains can be linked, using recombinant methods, by a synthetic linker peptide capable of making these two domains produced as a single protein chain in which the VL and VH regions are paired to form a single-chain Fv (scFv). The antibody fragment can be obtained by a chemical method, a recombinant DNA method, or a protease digestion method.

The "Fab fragment" and "Fab" are used interchangeably herein to refer to an immunoglobulin fragment consisting of two polypeptide chains and comprising an immunoglobulin heavy chain variable domain VH, a heavy chain constant domain CH1, a light chain variable domain VL, and a light chain constant domain CL, wherein one polypeptide chain comprises, from N-terminus to C-terminus, a VH and one constant region selected from CH1 and CL, and the other polypeptide chain comprises, from N-terminus to C-terminus, a VL and the other constant region selected from CL and CH1, wherein the VH and VL domains are paired to form an antigen-binding site. Herein, a Fab chain comprising a heavy chain constant region CH1 is also referred to as a "Fab heavy chain"; correspondingly, a Fab chain comprising a light chain constant region CL is also referred to as a "Fab light chain".

The terms "VHH" and "VHH antibody" are used interchangeably herein and generally refer to an antibody that comprises or consists of only one heavy chain variable region and has an antigen-binding activity. The VHH generally comprises three CDRs and highly-conserved 4 framework regions, and generally has a structure of the following formula: FR1-CDR-FR2-CDR2-FR3-CDR3-FR4, wherein FR1 to FR4 refer to framework regions 1-4; CDR1 to CDR3 refer to complementarity determining regions 1-3. The CDR sequences in the VHH variable region may be determined according to any of the CDR definition schemes described in the "Definition" section, and preferably, the boundaries of the three CDRs in the variable region sequence may be defined according to IMGT. The VHH generally comprises only a heavy chain variable domain derived from a heavy-chain antibody lacking a light chain, also referred to as a nanobody. The VHH used in the present disclosure is preferably from animals of the Camelidae family, such as an alpaca, or a humanized or sequence-optimized form thereof (e.g., an affinity-matured form to increase the binding affinity). In some embodiments, the VHH of the present disclosure is a monovalent monospecific polypeptide molecule consisting of, or consisting essentially of, a single heavy chain variable region (e.g., a heavy chain variable region of a heavy-chain antibody).

The single-domain antibody or the VHH of the present disclosure may also be comprised in a larger polypeptide/protein. Examples of polypeptides/proteins comprising the VHH of the present disclosure include, but are not limited to, a heavy-chain antibody (HcAb), or a multispecific antibody, or a fusion protein.

The "heavy-chain antibody" described herein refers to an antibody having no light chain, which may comprise, for example, from the N-segment to the C-segment, VH-Fc or VH-CH2-CH3 or VH-hinge region-CH2-CH3, or may comprise VH-CH1-CH2-CH3. The heavy-chain antibody of the present disclosure may also encompass a homodimer, such as a heavy-chain dimer antibody having no light chain. The heavy-chain antibody may comprise a VH from a standard antibody or a VH from a single-domain antibody. For example, a VH in the heavy-chain antibody may be a VHH. In some embodiments, the heavy-chain antibody of the present disclosure may be a heavy-chain antibody having a framework region and/or a heavy chain constant region derived from animals of the Camelidae family (llamas, camels, particularly alpacas), a humanized form thereof or a sequence-optimized form thereof (affinity-matured form), or a fragment thereof (e.g., a fragment comprising at least a part of the constant region). The heavy-chain antibody of the present disclosure further encompasses an antibody formed by fusing the heavy chain variable region or the VHH to an Fc region (e.g., a human IgG Fc region, such as a human IgG1 or IgG4 Fc region). When reference is made to "VHH" in the context of a heavy-chain antibody or a multispecific antibody or a fusion protein, it should be understood that it is a portion of the multispecific antibody and not as a separate molecule.

The term "target" refers to the bound substance against which the binding molecule is directed. The target may be an antigen, or may be a ligand or a receptor.

The term "antigen" refers to a molecule that induces an immune response. Such an immune response may involve antibody production or activation of specific immune cells, or both. Those skilled will understand that any macromolecules, including essentially all proteins or peptides, can be used as antigens. In addition, an antigen may be derived from recombinant or genomic DNA. As used herein, the term "epitope" refers to a portion of an antigen that specifically interacts with an antibody molecule.

The term "target-binding region" as used herein refers to a portion of a multispecific binding molecule, e.g., a bispecific binding molecule, that binds to a particular target or an antigen. The target-binding region may be, for example, an antibody or immunoglobulin per se or an antibody fragment. Such target-binding region may or may not have a tertiary structure independent of the remainder of the multispecific antibody molecule, and may or may not bind to its target as a separate entity. The target-binding region may also be a receptor or a ligand, or a domain of a receptor capable of binding to a ligand. In the case of multispecific antibodies, the "target-binding region" is also referred to as the "antigen-binding region". In one embodiment, the antigen-binding region used in the multispecific antibody molecule of the present disclosure comprises a VH/VL pair consisting of a light chain variable region (VL) and a heavy chain variable region (VH) of the antibody, and the VH/VL pair may be contained in a single polypeptide chain (e.g., scFv) or in two separate polypeptide chains (e.g., contained in a Fab heavy chain and a Fab light chain, respectively). In one embodiment, the antigen-binding region used in the multispecific antibody molecule of the present disclosure may be a Fab. In one embodiment, the antigen-binding region used in the multispecific antibody molecule of the present disclosure may comprise only a VH, e.g., derived from a VHH, or be a VHH.

As used herein, the term "monospecific" antibody refers to an antibody having one or more binding regions, each of which binds to the same epitope of the same antigen. For example, the present disclosure provides a monospecific antibody against BCMA.

As used herein, the term "multispecific" antibody refers to an antibody having at least two antigen-binding regions, in which each antigen-binding site binds to a different epitope of the same antigen or a different epitope of a different antigen. The multispecific antibody is an antibody having binding specificities for at least two different antigen epitopes. In one embodiment, provided herein is a bispecific antibody having binding specificities for a first antigen and a second antigen. For example, the present disclosure provides a bispecific antibody against BCMA and CD3. In one embodiment, provided herein is a trispecific antibody having binding specificities for a first antigen, a second antigen, and a third antigen. For example, the present disclosure provides a trispecific antibody against BCMA, GPRC5D, and CD3.

The term "multispecific binding molecule" refers to a multispecific binding molecule with at least two specificities, e.g., a bispecific binding molecule, that is, the molecule comprises at least a first target-binding region and a second target-binding region, where the first target-binding region binds to one target, and the second target-binding region binds to another target. Accordingly, the multispecific binding molecule according to the present disclosure comprises specificities for binding to at least two different targets. The molecule according to the present disclosure further encompasses a multispecific molecule comprising multiple target-binding regions, e.g., a trispecific binding molecule. In some embodiments, in the case that the binding molecule is an antibody, the target is an antigen. In some embodiments, the multispecific binding molecule of the present disclosure is a bispecific antibody or a trispecific antibody.

When referring to "first antigen-binding region" in a bispecific antibody or trispecific antibody, it refers to a binding region for the first antigen, and is not intended to limit the number of such antigen-binding regions contained in the antibody. For example, a multispecific antibody may comprise one or more than one first antigen-binding regions. For example, a trispecific antibody comprises a first antigen-binding region, a second antigen-binding region, and a third antigen-binding region, but may comprise one or more than one first antigen-binding regions, one or more than one second antigen-binding regions, and one or more than one third antigen-binding regions. In some embodiments, the bispecific antibody molecule of the present disclosure comprises at least one antigen-binding region specifically binding to CD3 and at least one antigen-binding region specifically binding to BCMA. In some embodiments, the bispecific antibody molecule of the present disclosure comprises one antigen-binding region specifically binding to CD3 and one or two antigen-binding regions specifically binding to BCMA. In some embodiments, the trispecific antibody molecule of the present disclosure comprises at least one antigen-binding region specifically binding to GPRC5D, at least one antigen-binding region specifically binding to BCMA, and at least one antigen-binding region specifically binding to CD3. In some embodiments, the trispecific antibody molecule of the present disclosure comprises an antigen-binding region specifically binding to GPRC5D, an antigen-binding region specifically binding to BCMA, and an antigen-binding region specifically binding to CD3.

When referring to an "antigen-binding region derived from an antibody", it means that the binding domain constituting the antigen-binding region is or is derived from the binding domain of the antibody that specifically binds to the antigen. For example, a fragment of the antigen-binding region that specifically binds to the antigen, e.g., a Fab, is or is derived from a corresponding fragment of the antibody, e.g., a Fab; or the heavy chain variable region and/or the light chain variable region of the antigen-binding region is or is derived from the heavy chain variable region and/or the light chain variable region of the antibody; or 1, 2, 3, 4, 5, or 6 CDRs of the antigen-binding region are the CDRs of the antibody. The term "derived from" means that the fragment in the antigen-binding region is substantially identical to the fragment of the antibody from which it is derived, but has a mutation(s), such as a substitution, deletion, or addition, at one or more sites. In one specific embodiment, the mutation is not in the CDR of the antibody. In one specific embodiment, the mutation is not in the variable region of the antibody.

The term "variable region" or "variable domain" refers to a domain of a heavy chain or light chain of an antibody involved in the binding of the antibody to an antigen. Variable regions of heavy and light chains of native antibodies generally have similar structures, wherein each domain comprises four conserved framework regions (FRs) and three complementarity determining regions.

"Complementarity determining region" or "CDR region" or "CDR" is a region in an antibody variable domain that is highly variable in sequence and forms a structurally defined loop ("hypervariable loop") and/or comprises antigen-contacting residues ("antigen contact sites"). CDRs are primarily responsible for binding to antigenic epitopes. The CDRs of the heavy and light chains are generally referred to as CDR1, CDR2, and CDR3, and are numbered sequentially from the N-terminus. The CDRs located in the heavy chain variable domain of the antibody are referred to as HCDR1, HCDR2, and HCDR3, whereas the CDRs located in the light chain variable domain of the antibody are referred to as LCDR1, LCDR2, and LCDR3. In a given amino acid sequence of a light chain variable region or a heavy chain variable region, the exact amino acid sequence boundary of each CDR may be determined using any one or a combination of many well-known antibody CDR assignment schemes including, e.g., Chothia based on the three-dim*e*nsional structure of antibodies and the topology of the CDR loops (Chothia et al., (1989) Nature, 342: 877-883; Al-Lazikani et al., Standard conformations for the canonical structures of immunoglobulins, Journal of Molecular Biology, 273: 927-948 (1997)), Kabat based on antibody sequence variability (Kabat et al., Sequences of Proteins of Immunological Interest, 4th ed., U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

The following are the regional ranges of the CDRs defined using the Kabat, AbM, Chothia, Contact, and IMGT schemes.

| CDR | Kabat scheme | AbM scheme | Chothia scheme | Contact scheme | IMGT scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L51 |
| LCDR3 (Kabat and Chothia numbering systems) | L89-L97 | L89-L97 | L91-L96 | L89-L96 | L89-L97 |
| HCDR1 (Kabat numbering system) | H31-H35B | H26-H35B | H26-H32...34 | H30-H35B | H26-H35B |
| HCDR1 (Chothia numbering system) | H31-H35 | H26-H35 | H26-H32 | H30-H35 | H26-H33 |
| HCDR2 (Kabat and Chothia numbering systems) | H50-H65 | H50-H58 | H52-H56 | H47-H58 | H51-H56 |
| HCDR3 (Kabat and Chothia numbering systems) | H95-H102 | H95-H102 | H96-H101 | H93-H101 | H93-H102 |

Unless otherwise stated, the term "CDR" or "CDR sequence" used herein encompasses CDR sequences determined by any one of the schemes described above. CDRs may also be determined based on having the same Kabat numbering positions as a reference CDR sequence (e.g., any of the exemplary CDRs of the present disclosure). Unless otherwise stated, in the present disclosure, residue positions of an antibody variable region (including heavy chain variable region residues and light chain v*a*riable region residues) are determined according to the Kabat numbering system (Kabat et al., Sequences of Proteins of Immunological Interest, 5th ed., Public Health Service, National Institutes of Health, Bethesda, Md. (1991)).

In one embodiment, the HCDRs in the VHH antibody of the present disclosure are determined according to IMGT scheme.

The term "Fc domain", "Fc region", or "Fc fragment" is used herein to define a C-terminus region of an immunoglobulin heavy chain, which comprises at least a portion of a constant region. The term includes Fc regions of native sequences and variant Fc regions. A native immunoglobulin "Fc domain" comprises two or three constant domains, i.e., a CH2 domain, a CH3 domain, and an optional CH4 domain. For example, in native antibodies, an immunoglobulin Fc domain comprises the second and the third constant domains (CH2 domain and CH3 domain) derived from two heavy chains of IgG, IgA, and IgD antibodies; or comprises the second, the third, and the fourth constant domains (CH2 domain, CH3 domain, and CH4 domain) derived from two heavy chains of IgM and IgE antibodies. Unless otherwise stated herein, amino acid residues in the Fc region or the heavy chain constant region are numbered according to the EU numbering system (also known as the EU index) as described in, for example, Edelman, G.M. et al., Proc. Natl. Acad. USA, 63, 78-85 (1969) (https://pubmed.ncbi.nlm.nih.gov/5257969/), see also http://www.imgt.org/IMGTScientificChart/Numbering/Hu_IGHGnber.html. Herein, the term "Fc domain", "Fc region", or "Fc fragment" does not comprise a heavy chain variable region VH and a light chain variable region VL as well as a heavy chain constant region CH1 and a light chain constant region CL of an immunoglobulin, but in some cases, they may comprise a hinge region portion at the N-terminus of the heavy chain constant region, such as EPKSS or EPKSC. In some embodiments, the heavy chain constant region Fc suitable for use in the present disclosure is from an antibody heavy chain constant region, for example, a constant region of human IgG1, IgG2, IgG3, or IgG4, preferably from a constant region of IgG1. In some embodiments, the Fc region comprises a mutation(s) that reduces binding to an Fcγ receptor, e.g., an LALA mutation, a D265A mutation, and/or a P329A mutation, preferably an LALA mutation, a D265A mutation, and a P329A mutation. In some embodiments, the Fc comprises or consists of an amino acid sequence set forth in SEQ ID NO: 80, 90, 97, or 98, or an amino acid sequence having at least 85%, 90%, 91%, 92%, 93%, 94%, 95%, 96%, 97%, 98%, or 99% identity thereto. In some embodiments of the present disclosure, the Fc fragments form an Fc dimer by dimerization. In some embodiments, the Fc fragments form an Fc heterodimer by heterodimerization. In the case of heterodimerization of the Fc fragments into a heterodimer, the Fc fragments may comprise mutations for heterodimerization, such as a knob-into-hole mutation.

Examples of "effector functions" of immunoglobulins include: C1q binding and complement-dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake in antigen-presenting cells, down-regulation of cell surface receptors (such as B-cell receptors), and B-cell activation.

The term "chimeric antibody" is an antibody molecule in which: (a) a constant region or a portion thereof is modified, substituted, or exchanged such that antigen-binding sites are linked to constant regions of different or modified classes, effector functions, and/or species, or disparate molecules imparting new properties (e.g., enzymes, toxins, hormones, growth factors, and drugs) to chimeric antibodies, etc.; or (b) a constant region or a portion thereof is modified, substituted, or exchanged by variable regions with different or modified antigen-binding specificities. For example, a mouse antibody can be modified by substituting its constant region with a constant region from a human immunoglobulin. Due to the substitution with a human constant region, the chimeric antibody can retain its specificity for recognizing antigens, while having reduced immunogenicity in humans as compared to the original mouse antibody.

"Humanized antibody" is an antibody that retains the antigen-specific reactivity of a non-human antibody (such as a camelid-derived VHH antibody) and has lower immunogenicity when administered to humans as a therapeutic agent. This can be achieved, for example, by retaining non-human antigen-binding sites and substituting the remainder of the antibodies with their human counterparts (i.e., the portions of the variable regions not involved in binding are substituted with the corresponding parts of human antibodies).

As used herein, the term "anti", "binding", or "specific binding" means that the binding effect is selective for targets or antigens and may be distinguished from unwanted or non-specific interactions. The ability of a binding site to bind to a particular target or an antigen may be determined by flow cytometry, enzyme-linked immunosorbent assay (ELISA), or conventional binding assays known in the art, such as radioimmunoassay (RIA), bio-layer interferometry, MSD assay, or surface plasmon resonance (SPR).

"Affinity" or "binding affinity" refers to the inherent binding affinity that reflects the interaction between members of a binding pair. The affinity of molecule X for its partner Y may be generally represented by the dissociation constant (K_{D}), which is a ratio of the dissociation rate constant (K_{dis}) to the association rate constant (Kₒₙ). Affinity can be measured by common methods known in the art. One specific method for measuring affinity is the ForteBio kinetic binding assay described herein.

The "percent identity (%)" of an amino acid sequence refers to the percentage of amino acid residues in a candidate sequence that are the same as those of a specific amino acid sequence shown in this description when aligning the candidate sequence with the specific amino acid sequence shown in this description, with gaps introduced if necessary to achieve maximum percent sequence identity and without considering any conservative substitutions as part of sequence identity. In some embodiments, the present disclosure considers variants of the antibody molecule of the present disclosure that have a considerable degree of identity to the antibody molecule and sequence thereof specifically disclosed herein. For example, the identity is at least 80%, 85%, 90%, 95%, 97%, 98%, 99%, or higher. The variants may comprise conservative modifications, or be conservatively modified variants.

For polypeptide sequences, "conservative modifications" include substitutions for, deletions of, or additions to a polypeptide sequence that do not substantially change the desired functional activity of the polypeptide sequence. For example, conservative substitutions often result in the substitutions of a chemically similar amino acid for an amino acid. Conservative substitution tables providing functionally similar amino acids are well known in the art. 8 groups comprising amino acids that are conservatively substituted with each other are listed as follows: 1) alanine (A) and glycine (G); 2) aspartic acid (D) and glutamic acid (E); 3) asparagine (N) and glutamine (Q); 4) arginine (R) and lysine (K); 5) isoleucine (I), leucine (L), methionine (M), and valine (V); 6) phenylalanine (F), tyrosine (Y), and tryptophan (W); 7) serine (S) and threonine (T); and 8) cysteine (C) and methionine (M). In some embodiments, the term "conservative sequence modification" is used to refer to an amino acid modification that does not significantly affect or change the binding characteristics for an antigen of interest of the antibody molecule or binding protein molecule of the present disclosure comprising the amino acid sequence. For example, conservatively modified variants retain at least 80%, 85%, 90%, 95%, 98%, 99%, or higher, such as 100%-110% or higher, binding affinity for an antigen of interest relative to the parent antibody or binding protein.

A "knob-in-hole" mutation or "knob-into-hole" is used herein to refer to the introduction of mutations in a first Fc polypeptide and a second Fc polypeptide, respectively, using the "knob-in-hole" technique to form a protuberance ("knob") and a complementary cavity ("hole") at the interface of the first Fc polypeptide and the interface of the second Fc polypeptide. It is known in the art that the "knob-in-hole" technique enables the engineering of the interface between different chains of an antibody molecule to promote the correct association of the chains of the antibody molecule. Generally, this technique involves introducing a "protuberance/knob" at the interface of one chain, and introducing a corresponding "cavity/hole" at the interface of the other chain to be paired with, such that the protuberance can be placed at the cavity. A preferred interface comprises the CH3 domain from the heavy chain constant domains of one chain and the CH3 domain from the heavy chain constant domains of the other chain to be paired with. The protuberance can be constructed by replacing small amino acid side chains at an interface of the CH3 domain from the heavy chain constant domains of one chain with large side chains, such as tyrosine or tryptophan. The compensating cavity of the same size as, or a similar size to, the protuberance is constructed at an interface of the CH3 domain from the heavy chain constant domains of the other chain to be paired with, by replacing large amino acid side chains with small side chains, such as alanine or threonine. Another optional interface comprises a light chain CL domain and a heavy chain CH1 domain of the Fab fragment described above, and the correct heterodimerization between the two chains of the Fab fragment is promoted by constructing a protuberance-cavity interaction.

Herein, antibody constant regions or antibody constant domains, including CH1, CL, and an Fc domain as well as CH2, CH3, and optional CH4 domains that constitute the Fc domain, may be selected according to the intended function of the antibody molecule. For example, the constant region may be an IgA, IgD, IgE, IgG, or IgM region, particularly an immunoglobulin constant domain of human IgG, such as a constant domain of human IgG1, IgG2, IgG3, or IgG4, preferably a constant domain of human IgG1. The immunoglobulin constant region may have a native sequence or a variant sequence.

The term "linker" as used herein refers to any molecule that enables direct connection of different portions of a bispecific binding molecule. Examples of linkers to establish covalent linkages between different portions of a molecule include peptide linkers and non-protein polymers including, but not limited to, polyethylene glycol (PEG), polypropylene glycol, polyalkylene oxide, or copolymers of polyethylene glycol and polypropylene glycol. In some embodiments, the linker is a peptide linker (also referred to as a "linker peptide") and refers to a short amino acid sequence consisting of amino acids, such as glycine (G) and/or serine (S) and/or threonine (T) residues used alone or in combination, or a hinge region from an immunoglobulin, which is used for linking the amino acid sequence of a first portion of a binding molecule to a second portion of the binding molecule. For example, the peptide linker may link a first target-binding region of a binding molecule to a second target-binding region. For example, the peptide linker may also link one portion of an antibody to another portion of the antibody, e.g., a light chain variable region to a heavy chain variable region. Preferably, the peptide linker has a length sufficient to link two entities in a manner that maintains their conformation relative to each other without interference with the desired activities. In one embodiment, the linker peptide has a length of 5-50 amino acids, such as 10, 15, 20, 25, or 30 amino acids. In one embodiment, the linker peptide comprises amino acid sequences (GS)n, (GGS)n, (GGSGG)n, (GSGGS)n, (GGGGS)n, (GGGS)n, and (GGGGS)nG, where n is an integer equal to or greater than 1, for example, n is an integer selected from 2, 3, 4, 5, 6, 7, 8, 9, and 10. Useful linkers further include glycine-alanine polymers, alanine-serine polymers, and other flexible linkers. In some embodiments, the peptide linker is (GGGGS)n, where n = 1, 2, 3, or 4, such as the sequence set forth in SEQ ID NO: 95, 99, or 100. In some embodiments, the peptide linker is the sequence set forth in SEQ ID NO: 101.

In yet another embodiment, the linker peptide is a hinge region or a portion of a hinge region derived from an immunoglobulin, including a native hinge region or a portion thereof, or a mutated hinge region or a portion thereof. In one embodiment, the linker peptide is, for example, a hinge region or a portion thereof (e.g., EPKSC) of an immunoglobulin (e.g., IgG, such as IgG1, IgG2, IgG3, or IgG4), or it is a mutated hinge region or a portion thereof (e.g., EPKSS). Alternatively, a computer program may be used to simulate three-dimensional structures of proteins and peptides, or a suitable flexible linker peptide is rationally designed by a phage display method.

The term "host cell" refers to a cell into which an exogenous polynucleotide has been introduced, including the progeny of such cells. Host cells include "transformants" and "transformed cells", which include primary transformed cells and progeny derived therefrom. Host cells are any type of cell system that may be used to produce the antibody molecule of the present disclosure, including eukaryotic cells, e.g., mammalian cells, insect cells, and yeast cells; and prokaryotic cells, e.g., *E. coli* cells. Host cells include cultured cells, as well as cells within a transgenic animal, a transgenic plant, or a cultured plant tissue or an animal tissue.

The term "vector" used herein refers to a nucleic acid molecule capable of proliferating another nucleic acid to which it is linked. The term includes vectors that serve as self-replicating nucleic acid structures as well as vectors binding to the genome of a host cell into which they have been introduced. The term "expression vector" refers to a vector comprising a recombinant polynucleotide, which comprises an expression control sequence effectively linked to a nucleotide sequence to be expressed. Expression vectors contain sufficient cis-regulato*r*y elements for expression, and other elements for expression may be provided by a host cell or in an *in vitro* expression system. Expression vectors include all those known in the art, including cosmids, plasmids (e.g., naked or contained in liposomes), and viruses (e.g., lentiviruses, retroviruses, adenoviruses, and adeno-associated viruses) incorporated into recombinant polynucleotides.

The terms "individual" and "subject" are used interchangeably and refer to a mammal. The mammals include, but are not limited to, domesticated animals (e.g., cows, sheep, cats, dogs, and horses), primates (e.g., human and non-human primates such as monkeys), rabbits, and rodents (e.g., mice and rats). In particular, individuals are humans.

The term "treatment" (or "treat" or "treating") refers to slowing, interrupting, arresting, alleviating, stopping, lowering, or reversing the onset of symptoms, complications, or biochemical indications of a disease, or alleviating symptoms, or arresting or inhibiting the further progression of the disease, symptom, or disorder.

The term "prevention" (or "prevent" or "preventing") includes the inhibition of the development or progression of symptoms of a disease or disorder, or a specific disease or disorder. In some embodiments, subjects with a family history of cancer are candidates for preventive regimens. Generally, in the context of cancer, the term "prevention" refers to the administration of a drug prior to the onset of signs or symptoms of cancer, particularly in subjects at risk of cancer.

The term "therapeutic agent" described herein encompasses any substance that is effective in preventing or treating a tumor, e.g., cancer, including a chemotherapeutic agent, a cytokine, a cytotoxic agent, an additional antibody, a small molecule drug, or an immunomodulatory agent (e.g., an immunosuppressant).

The term "cytotoxic agent" used herein refers to a substance that inhibits or prevents cell functions and/or causes cell death or cell destruction.

"Chemotherapeutic agent" includes chemical compounds useful in the treatment of cancers or immune system diseases.

The term "small molecule drug" refers to a low molecular weight organic compound capable of regulating biological processes. "Small molecule" is defined as a molecule with a molecular weight of less than 10 kD, usually less than 2 kD, and preferably less than 1 kD. The small molecule includes but is not limited to inorganic molecules, organic molecules, organic molecules containing inorganic components, molecules containing radioactive atoms, synthetic molecules, peptide mimetics, and antibody mimetics. As therapeutic agents, small molecules penetrate cells better, are less susceptible to degradation, and are less likely to induce an immune response compared to large molecules.

The term "immunomodulatory agent" used herein refers to a natural or synthetic active agent or drug that suppresses or modulates an immune response. The immune response may be a humoral response or a cellular response. The immunomodulatory agent includes an immunosuppressant. In some embodiments, the immunomodulatory agent of the present disclosure includes an immune checkpoint inhibitor or an immune checkpoint agonist.

The term "effective amount" refers to an amount or dosage of the antibody, fragment, composition, or combination of the present disclosure which generates expected effects in a patient in need of treatment or prevention after being administered to the patient in a single dose or multiple doses.

The term "therapeutically effective amount" refers to an amount effective to achieve a desired therapeutic result at a necessary dose for a necessary period of time. The therapeutically effective amount is also such an amount that any toxic or adverse effect of the antibody, fragment thereof, composition, or combination is inferior to the therapeutically beneficial effect. The "therapeutically effective amount" preferably inhibits a measurable parameter or improves a measurable parameter by at least about 40%, and even more preferably by at least about 50%, 55%, 60%, 65%, 70%, 75%, 80%, 85%, 90%, or even 100%, relative to untreated subjects.

The term "prophylactically effective amount" refers to an amount effective to achieve a desired prophylactic result at a necessary dose for a necessary period of time. Generally, since a prophylactic dose is administered in a subject before or at an earlier stage of a disease, a prophylactically effective amount will be less than a therapeutically effective amount.

The term "tumor" refers to all neoplastic cell growth and proliferation, whether malignant or benign, and all pre-cancerous and cancerous cells and tissues. The terms "cancer", "carcinoma", and "tumor" are not mutually exclusive when referred to herein. The term "tumor" encompasses both solid tumors and hematological tumors.

The term "anti-tumor effect" or "tumor inhibitory effect" refers to a biological effect that can be demonstrated by a variety of means, including but not limited to, for example, decrease in tumor volume, decrease in the number of tumor cells, decrease in tumor cell proliferation, or decrease in tumor cell viability.

The term "pharmaceutical supplementary material" refers to diluents, adjuvants (e.g., Freund's adjuvants (complete and incomplete)), excipients, carriers, stabilizers, or the like, that are administered with the active substance.

The term "pharmaceutical composition" refers to a composition that exists in a form allowing effective biological activity of the active ingredient contained therein and does not contain additional ingredients having unacceptable toxicity to a subject to which the composition is administered.

The term "pharmaceutical combination or combination product" refers to a non-fixed combination product or a fixed combination product, including but not limited to, a kit and a pharmaceutical composition. The term "non-fixed combination" means that the active ingredients (e.g., (i) the antibody or the multispecific antibody of the present disclosure, and (ii) an additional therapeutic agent) are administered, either simultaneously or sequentially (without specific time limitation or at identical or different time intervals), to a patient as separate entities, where such administration provides two or more prophylactically or therapeutically effective active agents in the patient. The term "fixed combination" means that two or more active agents are administered to a patient simultaneously in the form of a single entity. The dose and/or time intervals of two or more active agents are preferably selected such that the combined use of the components can result in a therapeutic effect on the disease or disorder greater than that achieved by the use of either component alone. The ingredients may each take a separate formulation form and such separate formulation forms may be the same or different.

The term "combination therapy" refers to the administration of two or more therapeutic agents or modalities (e.g., radiotherapy or surgery) to treat the diseases as described herein. Such administration includes co-administration of these therapeutic agents in a substantially simultaneous manner, for example, in a single capsule with a fixed proportion of active ingredients. Alternatively, such administration includes co-administration of the active ingredients in a variety of or separate containers (such as tablets, capsules, powder, and liquid). The powder and/or liquid may be reconstituted or diluted to a desired dose before administration. In addition, such administration further includes using each type of the therapeutic agents at approximately the same time or in a sequential manner at different times. In any case, the therapeutic regimen will provide the beneficial effect of the pharmaceutical combination in the treatment of disorders or symptoms described herein.

"Subject/patient/individual sample" refers to a collection of cells or fluids obtained from a patient or a subject. The source of tissue or cell samples can be solid tissues, e.g., from fresh, frozen, and/or preserved organ or tissue samples or biopsy samples or puncture samples; blood or any blood component; body fluids such as tears, vitreous humors, cerebrospinal fluids, amniotic fluids, peritoneal fluids, or interstitial fluids; and cells from a subject at any time during pregnancy or development. In some embodiments, the tissue sample is a tumor tissue. Tissue samples may comprise compounds that are naturally not mixed with tissues, such as preservatives, anticoagulants, buffers, fixatives, nutrients, and antibiotics.

### II. Anti-BCMA Antibody

In one aspect, the present disclosure provides a BCMA antibody, which has a higher binding affinity for BCMA. In some embodiments, the BCMA antibody of the present disclosure is suitable for use in the construction of an antigen-binding region in a multispecific antibody molecule.

In some embodiments, the anti-BCMA antibody or the antigen-binding fragment thereof of the present disclosure binds to BCMA (e.g., human BCMA) with a higher affinity. In some embodiments, the anti-BCMA antibody or the antigen-binding fragment thereof of the present disclosure is capable of binding to human BCMA with a high affinity, e.g., with a K_{D} value of less than or equal to about 0.5 nM, such as less than or equal to about 5, 4, 3, 2, or 1 pM.

In some embodiments, the anti-BCMA antibody or the antigen-binding fragment thereof of the present disclosure binds to BCMA expressed by a cell. In some embodiments, the affinity of the anti-BCMA antibody for BCMA expressed by a cell is determined by flow cytometry.

### Single-domain antibody

In some embodiments, the anti-BCMA antibody of the present disclosure is a single-domain antibody, particularly a VHH antibody.

The single-domain antibody or the VHH antibody has a molecular weight of about one tenth of a human IgG molecule, and a physical diameter of only a few nanometers. Due to the small molecular size, a single-domain antibody has the following advantages over conventional four-chain antibodies: high stability and solubility, and the ability to recognize hidden antigenic sites. In addition, the single-domain antibody is also cheaper to prepare than conventional four-chain antibodies. In addition to the use thereof as an individual molecule, the single-domain antibody is also a suitable component for the construction of multispecific molecules.

In some embodiments, the anti-BCMA single-domain antibody of the present disclosure is a VHH antibody comprising or consisting of a heavy chain variable region typically having the following structure: FR1-VHH CDR1-FR2-VHH CDR2-FR3-VHH CDR3-FR4, wherein FR1 to FR4 refer to framework regions 1-4; VHH CDR1 to VHH CDR3 refer to complementarity determining regions 1-3. The CDR sequences in the VHH variable region may be determined according to any of the CDR definition schemes described in the "Definition" section, and preferably, the boundaries of the three CDRs in the VHH sequence may be defined according to IMGT.

In some embodiments, the anti-BCMA VHH antibody of the present disclosure comprises
(i) three complementarity determining regions (CDRs) contained in a VH set forth in any one of SEQ ID NOs: 4, 8, 10, 12, 14, 15, 16, 18, 21, 22, and 23, or
(ii) a sequence comprising a total of at least one and no more than 5, 4, 3, 2, or 1 amino acid modification (preferably amino acid substitutions, and more preferably conservative substitutions) in the three CDRs relative to the sequence of (i).

Preferably, the CDR sequences are defined according to IMGT.

In some embodiments, the anti-BCMA VHH antibody of the present disclosure comprises or consists of a heavy chain variable region comprising
(i) three complementarity determining regions (CDRs) contained in a VH set forth in any one of SEQ ID NOs: 4, 8, 10, 12, 14, 15, 16, 18, 21, 22, and 23, or
(ii) a sequence comprising a total of at least one and no more than 5, 4, 3, 2, or 1 amino acid modification (preferably amino acid substitutions, and more preferably conservative substitutions) in the three CDRs relative to the sequence of (i).

Preferably, the CDR sequences are defined according to IMGT.

In some embodiments, the anti-BCMA VHH antibody of the present disclosure comprises complementarity determining regions (CDRs) VHH CDR1, VHH CDR2, and VHH CDR3. In some embodiments, the anti-BCMA VHH of the present disclosure comprises or consists of a heavy chain variable region comprising complementarity determining regions (CDRs) VHH CDR1, VHH CDR2, and VHH CDR3.

In some embodiments, the VHH CDR1 comprises or consists of an amino acid sequence selected from SEQ ID NO: 1, 5, or 19, or the VHH CDR1 comprises an amino acid sequence having one, two, or three modifications (preferably amino acid substitutions, and more preferably conservative substitutions) compared to an amino acid sequence set forth in SEQ ID NO: 1, 5, or 19.

In some embodiments, the VHH CDR2 comprises or consists of an amino acid sequence set forth in SEQ ID NO: 2, 6, 9, 13, or 17, or the VHH CDR2 comprises an amino acid sequence having one, two, or three modifications (preferably amino acid substitutions, and more preferably conservative substitutions) compared to an amino acid sequence set forth in SEQ ID NO: 2, 6, 9, 13, or 17.

In some embodiments, the VHH CDR3 comprises or consists of an amino acid sequence selected from SEQ ID NO: 3, 7, or 20, or the VHH CDR3 comprises an amino acid sequence having one, two, or three modifications (preferably amino acid substitutions, and more preferably conservative substitutions) compared to an amino acid sequence set forth in SEQ ID NO: 3, 7, or 20.

In one embodiment, the anti-BCMA VHH antibody of the present disclosure comprises complementarity determining regions (CDRs) VHH CDR1, VHH CDR2, and VHH CDR3, wherein
(i) the VHH CDR1 comprises or consists of the amino acid sequence set forth in SEQ ID NO: 1, the VHH CDR2 comprises or consists of an amino acid sequence set forth in SEQ ID NO: 2, 9, or 13, and the VHH CDR3 comprises or consists of the amino acid sequence set forth in SEQ ID NO: 3;
(ii) the VHH CDR1 comprises or consists of an amino acid sequence set forth in SEQ ID NO: 1 or 5, the VHH CDR2 comprises or consists of the amino acid sequence set forth in SEQ ID NO: 6, and the VHH CDR3 comprises or consists of the amino acid sequence set forth in SEQ ID NO: 7;
(iii) the VHH CDR1 comprises or consists of the amino acid sequence set forth in SEQ ID NO: 1, the VHH CDR2 comprises or consists of the amino acid sequence set forth in SEQ ID NO: 17, and the VHH CDR3 comprises or consists of the amino acid sequence set forth in SEQ ID NO: 7; or
(iv) the VHH CDR1 comprises or consists of the amino acid sequence set forth in SEQ ID NO: 19, the VHH CDR2 comprises or consists of the amino acid sequence set forth in SEQ ID NO: 17, and the VHH CDR3 comprises or consists of the amino acid sequence set forth in SEQ ID NO: 20.

In one embodiment, the anti-BCMA VHH antibody of the present disclosure comprises or consists of a heavy chain variable region comprising complementarity determining regions (CDRs) VHH CDR1, VHH CDR2, and VHH CDR3, wherein
(i) the VHH CDR1 comprises or consists of the amino acid sequence set forth in SEQ ID NO: 1, the VHH CDR2 comprises or consists of an amino acid sequence set forth in SEQ ID NO: 2, 9, or 13, and the VHH CDR3 comprises or consists of the amino acid sequence set forth in SEQ ID NO: 3;
(ii) the VHH CDR1 comprises or consists of an amino acid sequence set forth in SEQ ID NO: 1 or 5, the VHH CDR2 comprises or consists of the amino acid sequence set forth in SEQ ID NO: 6, and the VHH CDR3 comprises or consists of the amino acid sequence set forth in SEQ ID NO: 7;
(iii) the VHH CDR1 comprises or consists of the amino acid sequence set forth in SEQ ID NO: 1, the VHH CDR2 comprises or consists of the amino acid sequence set forth in SEQ ID NO: 17, and the VHH CDR3 comprises or consists of the amino acid sequence set forth in SEQ ID NO: 7; or
(iv) the VHH CDR1 comprises or consists of the amino acid sequence set forth in SEQ ID NO: 19, the VHH CDR2 comprises or consists of the amino acid sequence set forth in SEQ ID NO: 17, and the VHH CDR3 comprises or consists of the amino acid sequence set forth in SEQ ID NO: 20.

In some embodiments, the anti-BCMA VHH antibody of the present disclosure comprises or consists of a heavy chain variable region, wherein the heavy chain variable region
(i) comprises or consists of an amino acid sequence having at least 90%, 91%, 92%, 93%, 94%, 95%, 96%, 97%, 98%, or 99% identity to an amino acid sequence selected from any one of SEQ ID NOs: 4, 8, 10, 12, 14, 15, 16, 18, 21, 22, and 23;
(ii) comprises or consists of an amino acid sequence selected from any one of SEQ ID NOs: 4, 8, 10, 12, 14, 15, 16, 18, 21, 22, and 23; or
(iii) comprises an amino acid sequence having one or more (preferably not more than 10, and more preferably not more than 5, 4, 3, 2, or 1) amino acid modifications (preferably amino acid substitutions, and more preferably conservative amino acid substitutions) compared to an amino acid sequence selected from any one of SEQ ID NOs: 4, 8, 10, 12, 14, 15, 16, 18, 21, 22, and 23, wherein preferably, the amino acid modifications do not occur in the CDRs.

In some embodiments, the anti-BCMA VHH antibody of the present disclosure comprises or consists of an amino acid sequence selected from any one of SEQ ID NOs: 4, 8, 10, 12, 14, 15, 16, 18, 21, 22, or 23.

In some embodiments, the VHH antibody of the present disclosure comprises CDR amino acid sequences and/or framework (FR) amino acid sequences derived from a Camelidae heavy-chain antibody produced by immunizing animals of the Camelidae family (e.g., an alpaca). In some embodiments, a VHH monoclonal antibody of the present disclosure derived from the Camelidae heavy-chain antibody may be engineered, for example, to comprise framework region sequences derived from human amino acid sequences (i.e., human antibodies) or other non-Camelidae mammalian species. In one embodiment, to further improve the properties (e.g., affinity) of the engineered antibody, camelid-derived amino acid residues at corresponding positions in the parent camelid-derived antibody may be introduced in the engineered antibody by back mutations at one or more positions (e.g., framework regions).

In one embodiment, the VHH antibody of the present disclosure is a humanized antibody. The humanization may be achieved by the following method: replacing one or more amino acid residues, particularly framework region sequences, of a native VHH sequence of a non-human origin (e.g., a VHH sequence derived from animals of the Camelidae family or the alpaca after immunization) with residues from the heavy chain VH of the conventional human antibody at the corresponding positions. Methods for humanizing VHHs are well known in the art, for example as described in Example 3. Generally, the humanization substitutions are made in a manner that preserves the favorable binding properties of the single-domain antibody. Assays for determining biological properties of the humanized single-domain antibody, such as binding affinity, are well known in the art to determine and select a suitable humanized residue mutation or a combination of mutations.

In some embodiments, the humanized single-domain antibody of the present disclosure may be obtained by a method comprising the following steps:
determining a CDR loop structure of a parent single-domain antibody (e.g., a camelid-derived VHH antibody screened from a phage display library);
finding the closest homologous sequence for each V/J region in a human germline sequence database as a template, for example by comparing with the IMGT human antibody heavy chain variable region germline gene database (http://www.imgt.org/3Dstructure-DB/cgi/DomainGapAlign.cgi), and selecting a heavy chain variable region germline gene with high homology to the VHH antibody as a template;
grafting the CDRs of the VHH antibody separately into the selected corresponding humanized templates to form a variable region sequence of FR1-CDR1-FR2-CDR2-FR3-CR3-FR4, wherein preferably, a framework sequence for substitution is structurally similar to a framework sequence of an antibody to be humanized, e.g., a framework sequence having at least 80%, 85%, 90%, or 95%, 96%, 97%, 98%, 99% or more sequence identity; and
back-mutating key amino acids in the FR region to amino acids corresponding to the VHH antibody as needed to ensure the original affinity, so as to obtain the humanized anti-BCMA VHH antibody, and optionally sequencing the VHH antibody.

In some embodiments, the back-mutation site is selected from one or more of the frameworks (FRs).

In some embodiments, the heavy chain variable region germline gene suitable for humanizing the VHH antibody of the present disclosure is selected from IGHV3-53*02 or IGHV3-53*03.

In some embodiments, the present disclosure further provides a functional variant of the single-domain antibody of the present disclosure (in particular the VHH antibody). The functional variant may be obtained by the methods well known to the present disclosure, for example, by introducing mutations into the encoding nucleic acid sequences of exemplary single-domain antibodies of the present disclosure, e.g., into CDR sequences and/or FR sequences, and then screening (e.g., by phage display library screening) variants that retain the desired properties, e.g., via random or site-directed mutagenesis. Generally, the functional variant retains significant sequence identity to the parent single-domain antibody (or the VHH). Preferably, the functional variant retains the desired biological properties of the parent single-domain antibody (or the VHH). For example, the variant has comparable (e.g., at least 50%, 60%, 70%, or 80%, preferably 90% or more) or improved biological activity (e.g., 110%-150% or higher) relative to the biological activity of the parent. The desired biological properties include, for example, but are not limited to, binding affinity for an antigen of interest (e.g., BCMA) (as measured by KD values), blocking activity against the binding of the antigen of interest to a receptor (as measured by IC50 values), activation activity on T cells in *in-vitro* or *in-vivo* assays (as measured by release amount of cytokines), and inhibition of tumor growth/survival in *in-vitro* or *in-vivo* assays.

In some embodiments, the present disclosure provides an affinity variant of a VHH polypeptide of the present disclosure. Preferably, the affinity variant exhibits one or more amino acid modifications in the amino acid sequence relative to the parent single-domain antibody from which it is derived, wherein the affinity variant has altered binding affinity for an antigen of interest as compared to the parent antibody.

### Heavy-chain antibody

In another aspect of the present disclosure, the present disclosure further provides a heavy-chain antibody comprising the heavy chain variable region of the VHH antibody of the present disclosure.

In some embodiments, the single-domain antibody or the VHH of the present disclosure (e.g., a camelid-derived VHH or a humanized form thereof) may be linked to a constant region of a human antibody or a portion thereof (e.g., an Fc region) to produce a heavy-chain antibody comprising VHH-constant region or VHH-CH1-Fc or VHH-Fc. In one embodiment, the heavy-chain antibody comprises the VHH antibody of the present disclosure and the Fc region at the C-terminus thereof. In some embodiments, the VHH is linked to the Fc via a hinge region or a portion thereof, for example a hinge region from an IgG (e.g., a hinge region from IgG1, IgG2, IgG3, or IgG4) or a portion thereof.

In some embodiments, the anti-BCMA heavy-chain antibody of the present disclosure comprises the VHH or the heavy chain variable region therein as defined herein, and a heavy chain constant region or an Fc region of the heavy chain constant region. In some embodiments, a linker peptide, such as an antibody hinge region or a portion thereof, e.g., a hinge region or a portion thereof from an IgG (comprising a native or mutated IgG hinge region or a portion thereof), is contained between the VHH or the heavy chain variable region thereof and the heavy chain constant region or the Fc region.

In some embodiments, the linker peptide is a hinge region from human IgG1, human IgG2, human IgG3, or human IgG4 or a portion thereof, comprising a native or mutated hinge region or a portion thereof, such as a hinge region from human IgG1. For example, the linker peptide is EPKSS (SEQ ID NO: 94) or EPKSC(SEQ ID NO: 96).

In one embodiment, the heavy-chain antibody comprises an Fc region or a portion thereof from animals of the Camelidae family (e.g., an alpaca). In one embodiment, the heavy-chain antibody is produced and isolated by immunizing animals of the Camelidae family, e.g., an alpaca. A variety of methods are known in the art for immunizing animals of the Camelidae family and isolating the VHH antibody or the heavy-chain antibody produced against the antigen of interest.

In some embodiments, the heavy-chain antibody comprises a constant region from a human or non-human primate (e.g., cynomolgus monkey) antibody, e.g., a constant region from human IgG1, human IgG2, human IgG3, or human IgG4.

In some embodiments, the heavy-chain antibody comprises an Fc portion from a human or non-human primate (e.g., cynomolgus monkey). In yet another embodiment, the heavy-chain antibody comprises a human IgG Fc region, e.g., a human IgG1, human IgG2, human IgG3, or human IgG4 Fc region, preferably a human IgG1 or human IgG4 Fc region, such as a human IgG1 Fc region.

In one embodiment, the heavy-chain antibody according to the present disclosure may be dimerized with another polypeptide chain comprising an Fc region (e.g., another heavy-chain antibody, which is the same or different) via the Fc region. Thus, in one embodiment, the present disclosure further provides a homo- or heteromultimeric protein comprising the heavy-chain antibody of the present disclosure. In one preferred embodiment, the protein preferably comprises a heavy-chain antibody formed by pairing two identical heavy-chain antibody chains.

The Fc region of the present disclosure may be mutated to obtain desired properties. Mutations for the Fc region are known in the art, as defined below.

In some embodiments, the anti-BCMA antibody or the antigen-binding fragment thereof of the present disclosure comprises a heavy chain comprising a heavy chain variable region, an Fc region, and a linker peptide linking the heavy chain variable region and the Fc region. Preferably, the linker peptide comprises or consists of an amino acid sequence set forth in SEQ ID NO: 94 or 96.

In some embodiments, the anti-BCMA antibody or the antigen-binding fragment thereof of the present disclosure comprises or consists of a heavy chain comprising or consisting of a heavy chain variable region of the VHH of the present disclosure, a linker peptide, and an Fc region, wherein the heavy chain
(i) comprises or consists of an amino acid sequence having at least 85%, 90%, 91%, 92%, 93%, 94%, 95%, 96%, 97%, 98%, or 99% identity to an amino acid sequence selected from any one of SEQ ID NOs: 44-46 and 48-55;
(ii) comprises or consists of an amino acid sequence selected from any one of SEQ ID NOs: 44-46 and 48-55; or
(iii) comprises an amino acid sequence having one or more (preferably not more than 10, and more preferably not more than 5, 4, 3, 2, or 1) amino acid modifications (preferably amino acid substitutions, and more preferably conservative amino acid substitutions) compared to an amino acid sequence selected from any one of SEQ ID NOs: 44-46 and 48-55, wherein preferably, the amino acid modifications do not occur in the CDRs.

### III. Anti-GPRC5D Antibody

The present disclosure further provides a GPRC5D antibody, which has a higher binding affinity for GPRC5D. In some embodiments, the GPRC5D antibody of the present disclosure is suitable for use in the construction of an antigen-binding region in a multispecific antibody molecule.

In some embodiments, the anti-GPRC5D antibody of the present disclosure is a single-domain antibody, particularly a VHH antibody.

The single-domain antibody or the VHH antibody has a molecular weight of about one tenth of a human IgG molecule, and a physical diameter of only a few nanometers. Due to the small molecular size, a single-domain antibody has the following advantages over conventional four-chain antibodies: high stability and solubility, and the ability to recognize hidden antigenic sites. In addition, the single-domain antibody is also cheaper to prepare than conventional four-chain antibodies. In addition to the use thereof as an individual molecule, the single-domain antibody is also a suitable component for the construction of multispecific molecules.

The single-domain antibody or the VHH antibody has a molecular weight of about one tenth of a human IgG molecule, and a physical diameter of only a few nanometers. Due to the small molecular size, a single-domain monoclonal antibody has the following advantages over conventional four-chain antibodies: high stability and solubility, and the ability to recognize hidden antigenic sites. In addition, the single-domain antibody is also cheaper to prepare than conventional four-chain antibodies. In addition to the use thereof as an individual molecule, the single-domain antibody is also a suitable component for the construction of multispecific molecules.

In some embodiments, the anti-GPRC5D single-domain antibody of the present disclosure is a VHH antibody comprising or consisting of a heavy chain variable region typically having the following structure: FR1-VHH CDR1-FR2-VHH CDR2-FR3-VHH CDR3-FR4, wherein FR1 to FR4 refer to framework regions 1-4; VHH CDR1 to VHH CDR3 refer to complementarity determining regions 1-3. The CDR sequences in the VHH variable region may be determined according to any of the CDR definition schemes described in the "Definition" section, and preferably, the boundaries of the three CDRs in the VHH sequence may be defined according to IMGT.

In some embodiments, the anti-GPRC5D VHH antibody of the present disclosure comprises
(i) three complementarity determining regions (CDRs) contained in a VH set forth in any one of SEQ ID NOs: 27 and 106, or
(ii) a sequence comprising a total of at least one and no more than 5, 4, 3, 2, or 1 amino acid modification (preferably amino acid substitutions, and more preferably conservative substitutions) in the three CDRs relative to the sequence of (i).

Preferably, the CDR sequences are defined according to IMGT.

In some embodiments, the anti-GPRC5D VHH antibody of the present disclosure comprises or consists of a heavy chain variable region comprising
(i) three complementarity determining regions (CDRs) contained in a VH set forth in any one of SEQ ID NOs: 27 and 106, or
(ii) a sequence comprising a total of at least one and no more than 5, 4, 3, 2, or 1 amino acid modification (preferably amino acid substitutions, and more preferably conservative substitutions) in the three CDRs relative to the sequence of (i).

Preferably, the CDR sequences are defined according to IMGT.

In some embodiments, the anti-GPRC5D VHH antibody of the present disclosure comprises complementarity determining regions (CDRs) VHH CDR1, VHH CDR2, and VHH CDR3. In some embodiments, the anti-GPRC5D VHH of the present disclosure comprises or consists of a heavy chain variable region comprising complementarity determining regions (CDRs) VHH CDR1, VHH CDR2, and VHH CDR3.

In some embodiments, the VHH CDR1 comprises or consists of the amino acid sequence selected from SEQ ID NO: 24, or the VHH CDR1 comprises an amino acid sequence having one, two, or three modifications (preferably amino acid substitutions, and more preferably conservative substitutions) compared to the amino acid sequence set forth in SEQ ID NO: 24.

In some embodiments, the VHH CDR2 comprises or consists of the amino acid sequence set forth in SEQ ID NO: 25, or the VHH CDR2 comprises an amino acid sequence having one, two, or three modifications (preferably amino acid substitutions, and more preferably conservative substitutions) compared to the amino acid sequence set forth in SEQ ID NO: 25.

In some embodiments, the VHH CDR3 comprises or consists of the amino acid sequence selected from SEQ ID NO: 26, or the VHH CDR3 comprises an amino acid sequence having one, two, or three modifications (preferably amino acid substitutions, and more preferably conservative substitutions) compared to the amino acid sequence set forth in SEQ ID NO: 26.

In one embodiment, the anti-GPRC5D VHH antibody of the present disclosure comprises complementarity determining regions (CDRs) VHH CDR1, VHH CDR2, and VHH CDR3, wherein the VHH CDR1 comprises or consists of the amino acid sequence set forth in SEQ ID NO: 24, the VHH CDR2 comprises or consists of the amino acid sequence set forth in SEQ ID NO: 25, and the VHH CDR3 comprises or consists of the amino acid sequence set forth in SEQ ID NO: 26.

In one embodiment, the anti-GPRC5D VHH antibody of the present disclosure comprises or consists of a heavy chain variable region comprising complementarity determining region (CDRs) VHH CDR1, VHH CDR2, and VHH CDR3, wherein the VHH CDR1 comprises or consists of the amino acid sequence set forth in SEQ ID NO: 24, the VHH CDR2 comprises or consists of the amino acid sequence set forth in SEQ ID NO: 25, and the VHH CDR3 comprises or consists of the amino acid sequence set forth in SEQ ID NO: 26.

In some embodiments, the anti-GPRC5D VHH antibody of the present disclosure comprises or consists of a heavy chain variable region, wherein the heavy chain variable region
(i) comprises or consists of an amino acid sequence having at least 90%, 91%, 92%, 93%, 94%, 95%, 96%, 97%, 98%, or 99% identity to an amino acid sequence selected from any one of SEQ ID NOs: 27 and 106;
(ii) comprises or consists of an amino acid sequence selected from any one of SEQ ID NOs: 27 and 106; or
(iii) comprises an amino acid sequence having one or more (preferably not more than 10, and more preferably not more than 5, 4, 3, 2, or 1) amino acid modifications (preferably amino acid substitutions, and more preferably conservative amino acid substitutions) compared to an amino acid sequence selected from any one of SEQ ID NOs: 27 and 106, wherein preferably, the amino acid modifications do not occur in the CDRs.

In some embodiments, the anti-GPRC5D VHH antibody of the present disclosure comprises or consists of an amino acid sequence selected from any one of SEQ ID NOs: 27 and 106.

In some embodiments, the VHH antibody of the present disclosure comprises CDR amino acid sequences and/or framework (FR) amino acid sequences derived from a Camelidae heavy-chain antibody produced by immunizing animals of the Camelidae family (e.g., an alpaca). In some embodiments, a VHH monoclonal antibody of the present disclosure derived from the Camelidae heavy-chain antibody may be engineered, for example, to comprise framework region sequences derived from human amino acid sequences (i.e., human antibodies) or other non-Camelidae mammalian species. In one embodiment, to further improve the properties (e.g., affinity) of the engineered antibody, camelid-derived amino acid residues at corresponding positions in the parent camelid-derived antibody may be introduced in the engineered antibody by back mutations at one or more positions (e.g., framework regions).

In one embodiment, the VHH antibody of the present disclosure is a humanized antibody.

In some embodiments, the present disclosure further provides a functional variant of the single-domain antibody of the present disclosure (in particular the VHH antibody). The functional variant may be obtained by the methods well known to the present disclosure, for example, by introducing mutations into the encoding nucleic acid sequences of exemplary single-domain antibodies of the present disclosure, e.g., into CDR sequences and/or FR sequences, and then screening (e.g., by phage display library screening) variants that retain the desired properties, e.g., via random or site-directed mutagenesis. Generally, the functional variant retains significant sequence identity to the parent single-domain antibody (or the VHH). Preferably, the functional variant retains the desired biological properties of the parent single-domain antibody (or the VHH). For example, the variant has comparable (e.g., at least 50%, 60%, 70%, or 80%, preferably 90% or more) or improved biological activity (e.g., 110%-150% or higher) relative to the biological activity of the parent. The desired biological properties include, for example, but are not limited to, binding affinity for an antigen of interest (e.g., GPRC5D) (as measured by KD values), blocking activity against the binding of the antigen of interest to a receptor (as measured by IC50 values), activation activity on T cells in *in-vitro* or *in-vivo* assays (as measured by release amount of cytokines), and inhibition of tumor growth/survival in *in-vitro* or *in-vivo* assays.

In some embodiments, the present disclosure provides an affinity variant of a VHH polypeptide of the present disclosure. Preferably, the affinity variant exhibits one or more amino acid modifications in the amino acid sequence relative to the parent single-domain antibody from which it is derived, wherein the affinity variant has altered binding affinity for an antigen of interest as compared to the parent antibody.

In another aspect of the present disclosure, the present disclosure further provides a heavy-chain antibody comprising the heavy chain variable region of the anti-GPRC5D VHH antibody of the present disclosure.

In some embodiments, the anti-GPRC5D single-domain antibody or the VHH of the present disclosure (e.g., a camelid-derived VHH or a humanized form thereof) may be linked to a constant region of a human antibody or a portion thereof (e.g., an Fc region) to produce a heavy-chain antibody comprising VHH-constant region or VHH-CH1-Fc or VHH-Fc. In one embodiment, the heavy-chain antibody comprises the VHH antibody of the present disclosure and the Fc region at the C-terminus thereof. In some embodiments, the VHH is linked to the Fc via a hinge region or a portion thereof, for example a hinge region from an IgG (e.g., a hinge region from IgG1, IgG2, IgG3, or IgG4) or a portion thereof.

In some embodiments, the anti-GPRC5D heavy-chain antibody of the present disclosure comprises the anti-GPRC5D VHH or the heavy chain variable region therein as defined herein, and a heavy chain constant region or an Fc region of the heavy chain constant region. In some embodiments, a linker peptide, such as an antibody hinge region or a portion thereof, e.g., a hinge region or a portion thereof from an IgG (comprising a native or mutated IgG hinge region or a portion thereof), is contained between the anti-GPRC5D VHH or the heavy chain variable region thereof and the heavy chain constant region or the Fc region.

In some embodiments, the linker peptide is a hinge region from human IgG1, human IgG2, human IgG3, or human IgG4 or a portion thereof, comprising a native or mutated hinge region or a portion thereof, such as a hinge region from human IgG1. For example, the linker peptide is EPKSS (SEQ ID NO: 94) or EPKSC(SEQ ID NO: 96).

In one embodiment, the heavy-chain antibody comprises an Fc region or a portion thereof from animals of the Camelidae family (e.g., an alpaca). In one embodiment, the heavy-chain antibody is produced and isolated by immunizing animals of the Camelidae family, e.g., an alpaca. A variety of methods are known in the art for immunizing animals of the Camelidae family and isolating the VHH antibody or the heavy-chain antibody produced against the antigen of interest.

In some embodiments, the heavy-chain antibody comprises a constant region from a human or non-human primate (e.g., cynomolgus monkey) antibody, e.g., a constant region from human IgG1, human IgG2, human IgG3, or human IgG4.

In some embodiments, the heavy-chain antibody comprises an Fc portion from a human or non-human primate (e.g., cynomolgus monkey). In yet another embodiment, the heavy-chain antibody comprises a human IgG Fc region, e.g., a human IgG1, human IgG2, human IgG3, or human IgG4 Fc region, preferably a human IgG1 or human IgG4 Fc region, such as a human IgG1 Fc region.

In one embodiment, the heavy-chain antibody according to the present disclosure may be dimerized with another polypeptide chain comprising an Fc region (e.g., another heavy-chain antibody, which is the same or different) via the Fc region. Thus, in one embodiment, the present disclosure further provides a homo- or heteromultimeric protein comprising the heavy-chain antibody of the present disclosure. In one preferred embodiment, the protein preferably comprises a heavy-chain antibody formed by pairing two identical heavy-chain antibody chains.

The Fc region of the present disclosure may be mutated to obtain desired properties. Mutations for the Fc region are known in the art, as defined below.

In some embodiments, the anti-GPRC5D antibody of the present disclosure comprises a heavy chain comprising a heavy chain variable region, an Fc region, and a linker peptide linking the heavy chain variable region and the Fc region. Preferably, the linker peptide comprises or consists of an amino acid sequence set forth in SEQ ID NO: 94 or 96.

### V. Multispecific Antibody

In some embodiments, the anti-BCMA antibody of the present disclosure is a multispecific antibody, e.g., a bispecific antibody or a trispecific antibody, and, for example, comprises one binding specificity for BCMA and additional binding specificities for one or more molecules (e.g., CD3, or, e.g., CD3 and GPRC5D).

Therefore, one aspect of the present disclosure relates to a bispecific antibody, which comprises
a first antigen-binding region and a second antigen-binding region, wherein the first antigen-binding region specifically binds to CD3, and/or the second antigen-binding region specifically binds to BCMA.

Therefore, another aspect of the present disclosure relates to a trispecific antibody, which comprises
a first antigen-binding region, a second antigen-binding region, and a third antigen-binding region, wherein the first antigen-binding region specifically binds to CD3, the second antigen-binding region specifically binds to BCMA, and the third antigen-binding region specifically binds to GPRC5D.

In some embodiments, the first antigen-binding region is from an anti-CD3 antibody, e.g., an SP34 antibody or a humanized antibody thereof, e.g., the anti-CD3 humanized antibody disclosed in PCT/CN2023/071314, e.g., a Fab fragment of an anti-CD3 antibody or e.g., an scFv fragment of an anti-CD3 antibody.

In some embodiments, the second antigen-binding region is from the anti-BCMA antibody described herein, e.g., an anti-BCMA VHH.

In some embodiments, the third antigen-binding region is from an anti-GPRC5D antibody, e.g., the anti-GPRC5D antibody disclosed in PCT/CN2023/071314, such as a Fab fragment of an anti-GPRC5D antibody. In some embodiments, the third antigen-binding region is from an anti-GPRC5D VHH antibody, e.g., the VHH antibody described herein above.

The first antigen-binding region suitable for use in the multispecific antibody of the present disclosure may comprise or consist of an anti-CD3 full-length antibody or an antigen-binding fragment thereof (e.g., the anti-CD3 humanized antibody or the antigen-binding fragment thereof disclosed in PCT/CN2023/071314) as long as it is capable of specifically binding to CD3, including, but not limited to, for example, a full-length antibody, a single-chain Fv, a Fab, a Fab', a (Fab)2, a single-domain antibody, a VHH, a heavy-chain antibody, or the like specifically binding to CD3.

The second antigen-binding region suitable for use in the multispecific antibody of the present disclosure may comprise or consist of the anti-BCMA VHH of the present disclosure as long as it is capable of specifically binding to BCMA, including, but not limited to, for example, a full-length antibody, a single-chain Fv, a Fab, a Fab', a (Fab)2, a single-domain antibody, a VHH, a heavy-chain antibody, or the like specifically binding to BCMA.

The third antigen-binding region suitable for use in the multispecific antibody of the present disclosure may comprise or consist of, for example, the anti-GPRC5D VHH of the present disclosure, or comprise or consist of an anti-GPRC5D full-length antibody or an antigen-binding fragment thereof (e.g., the anti-GPRC5D antibody or the antigen-binding fragment thereof disclosed in PCT/CN2023/071314) as long as it is capable of specifically binding to GPRC5D, including, but not limited to, for example, a full-length antibody, a single-chain Fv, a Fab, a Fab', a (Fab)2, a single-domain antibody, a VHH, a heavy-chain antibody, or the like specifically binding to GPRC5D.

In some embodiments, the bispecific antibody or the trispecific antibody of the present disclosure is an IgG-like bispecific antibody. The term "IgG-like bispecific antibody" described herein refers to a bispecific antibody or a trispecific antibody comprising an Fc dimer. Therefore, in some embodiments, the bispecific antibody or the trispecific antibody of the present disclosure comprises an Fc dimer.

In some embodiments, the bispecific antibody of the present disclosure is an IgG-like bispecific antibody comprising an scFv fragment as an antigen-binding region that specifically binds to one antigen, and a VHH as an antigen-binding region that specifically binds to another antigen. In some embodiments, the IgG-like bispecific antibody comprises an scFv specifically binding to CD3 as a first antigen-binding region, and a VHH fragment specifically binding to BCMA as a second antigen-binding region.

In one embodiment, the bispecific antibody may comprise one or more first antigen-binding regions. In one embodiment, the bispecific antibody may comprise one or more second antigen-binding regions. In one embodiment, the bispecific antibody comprises one first antigen-binding region, and one or two (e.g., tandem) second antigen-binding regions.

In some embodiments, the trispecific antibody of the present disclosure is an IgG-like trispecific antibody comprising a Fab fragment as an antigen-binding region that specifically binds to one antigen, and a VHH as an antigen binding region that specifically binds to two additional antigens, respectively. In some embodiments, the IgG-like trispecific antibody comprises a first antigen-binding region comprising or consisting of a Fab fragment specifically binding to CD3, a second antigen-binding region comprising or consisting of a VHH fragment specifically binding to BCMA, and a third antigen-binding region comprising or consisting of a VHH fragment specifically binding to GPRC5D.

In some embodiments, the trispecific antibody of the present disclosure is an IgG-like trispecific antibody comprising an scFV fragment as an antigen-binding region that specifically binds to one antigen, a VHH as an antigen-binding region that specifically binds to another antigen, and a Fab fragment as an antigen-binding region that specifically binds to a third antigen. In some embodiments, the IgG-like trispecific antibody comprises a first antigen-binding region comprising or consisting of an scFv fragment specifically binding to CD3, a second antigen-binding region comprising or consisting of a VHH fragment specifically binding to BCMA, and a third antigen-binding region comprising or consisting of a Fab fragment specifically binding to GPRC5D.

In one embodiment, the trispecific antibody may comprise one or more first antigen-binding regions. In one embodiment, the trispecific antibody may comprise one or more second antigen-binding regions. In one embodiment, the trispecific antibody may comprise one or more third antigen-binding regions. In one embodiment, the trispecific antibody comprises one first antigen-binding region, one second antigen-binding region, and one third antigen-binding region.

In some embodiments, the first antigen-binding region specifically binding to CD3 is selected from an scFv or a Fab, the second antigen-binding region specifically binding to BCMA is a VHH, and/or the third antigen-binding region specifically binding to GPRC5D is selected from a VHH or a Fab.

### ➢ Fab fragment suitable for use in multispecific antibody of the present disclosure

In some embodiments, the Fab fragment, as one of the antigen-binding regions of the multispecific antibody, consists of two polypeptide chains comprising VH, CH1 (optionally comprising a hinge region), VL, and CL domains of the antibody, wherein the VH is paired with the VL, and the CH1 is paired with the CL to form the antigen-binding region. In some embodiments, in the Fab, one chain comprises, from N-terminus to C-terminus, a VH and a CH1 (i.e., a VH-CH1), and the other chain comprises, from N-terminus to C-terminus, a VL and a CL (i.e., a VL-CL).

In some embodiments, in the multispecific antibody, the Fab may be fused to the N-terminus of the Fc domain of the antibody via the C-terminus of the chain comprising the VH, wherein the Fc domain may or may not comprise a hinge region (e.g., EPKSS or EPKSC). Preferably, the Fab comprises a VH-CH1 chain and a VL-CL chain and is fused to the Fc domain of the antibody via the C-terminus of the CH1 of the VH-CH1 chain, wherein the Fc domain may or may not comprise a hinge region (e.g., EPKSS or EPKSC). Herein, a Fab chain linked to an Fc dimer is also referred to as a Fab heavy chain, and a Fab chain not linked to an Fc dimer is also referred to as a Fab light chain. In some embodiments, the fusion is direct fusion or fusion through a linker.

In some embodiments, the CH1 in the Fab may also comprise a hinge region portion, e.g., EPKSS or EPKSC, to facilitate the formation of a stable structure, such as to facilitate the production of a multispecific antibody. Where the Fc fused to the heavy chain of the Fab does not comprise the hinge region portion, or where the Fab heavy chain is fused to a non-Fc domain, the hinge region portion may be comprised at the C-terminus of the CH1 to facilitate the formation of a stable structure. Where the Fc fused to the heavy chain of the Fab comprises the hinge region portion, the C-terminus of the CH1 of the Fab heavy chain may not comprise the hinge region portion.

In some embodiments, the Fab comprised in the multispecific antibody of the present disclosure specifically binds to CD3 or specifically binds to GPRC5D.

In some embodiments, the Fab heavy chain comprises a VH and a CH1 (and optionally comprises a hinge region portion EPKSS or EPKSC at the C-terminus of the CH1). In some embodiments, the CH1 is a CH1 from IgG1, IgG2, IgG3, or IgG4, preferably a CH1 from IgG1. In some embodiments, the CH1
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 88;
(ii) comprises or consists of the amino acid sequence selected from SEQ ID NO: 88; or
(iii) comprises or consists of an amino acid sequence having one or more (preferably not more than 10 or 10, and more preferably not more than 5, 4, 3, 2, or 1) amino acid modifications (preferably amino acid substitutions, and more preferably conservative amino acid substitutions) compared to the amino acid sequence selected from SEQ ID NO: 88.

In some embodiments, the Fab light chain comprises a VL-CL. In some embodiments, the CL is a light chain constant region from an antibody kappa or lambda light chain, preferably a light chain constant region from a kappa light chain.

### ➢ scFv suitable for use in multispecific antibody of the present disclosure

In some embodiments, the scFv fragment, as one of the binding regions of the multispecific antibody, consists of one polypeptide chain comprising VH and VL domains of the antibody, wherein the VH and the VL are linked (e.g., via a linker) for pairing to form the antigen-binding site. In some embodiments, the scFv is a trans-configuration comprising, from N-terminus to C-terminus: a VH, a linker, and a VL (VH-linker-VL). In some other embodiments, the scFv is a cis-configuration comprising, from N-terminus to C-terminus: a VL, a linker, and a VH (VL-linker-VH). In some embodiments, the linker is a peptide linker consisting of amino acid residues. Suitable peptide linkers are known to those skilled in the art. In one embodiment, the linker has a length of 5-50 amino acids, such as 5-30 amino acids, for example, 15 amino acids or 20 amino acids. In one embodiment, the linker comprises an amino acid sequence (G4S)n, where n = 1, 2, 3, 4, or 5, preferably n = 3 or 4, more preferably n = 3.

In some preferred embodiments, the scFv antigen-binding site contained in the antibody molecule of the present disclosure is a disulfide-stabilized scFv.

In some embodiments, in the multispecific antibody, the scFv may be fused via the C-terminus of the chain to the N-terminus of the Fc domain of the antibody. In some embodiments, in the multispecific antibody, the scFv is fused at the N-terminus of the chain to the C-terminus of another antigen-binding region (e.g., the Fab heavy chain) and fused at the C-terminus of the chain to the N-terminus of the Fc domain of the antibody.

In some embodiments, in the multispecific antibody, the scFv may be fused via the C-terminus of the chain comprising the VL to the N-terminus of the Fc domain of the antibody. In some embodiments, in the multispecific antibody, the scFv is fused at the N-terminus of the VH chain to the C-terminus of another antigen-binding region (e.g., the Fab heavy chain) and fused at the C-terminus of the VL chain to the N-terminus of the Fc domain of the antibody.

In some embodiments, in the multispecific antibody, the scFv may be fused via the C-terminus of the chain comprising the VH to the N-terminus of the Fc domain of the antibody. In some embodiments, in the multispecific antibody, the scFv is fused at the N-terminus of the VL chain to the C-terminus of another antigen-binding region (e.g., the Fab heavy chain) and fused at the C-terminus of the VH chain to the N-terminus of the Fc domain of the antibody.

In some embodiments, the scFv comprised in the multispecific antibody of the present disclosure specifically binds to CD3.

### ➢ Fc dimer suitable for use in multispecific antibody of the present disclosure

In one embodiment, two Fc regions in the multispecific antibody of the present disclosure are dimerized to form a dimeric Fc. Preferably, the two Fc regions form a heterodimeric Fc by heterodimerization.

In some embodiments, the first and second Fc regions are identical. In some other embodiments, the first Fc region and the second Fc region are different, and the two are paired and heterodimerized.

The Fc region fragment suitable for use in the antibody molecule of the present disclosure may be any antibody Fc region. The Fc region may comprise a native sequence Fc region and a variant Fc region. The native sequence Fc domain encompasses naturally occurring Fc sequences of various immunoglobulins, such as Fc regions of various Ig subtypes and allotypes thereof (Gestur Vidarsson et al., IgG subclasses and allotypes: from structure to effector functions, 20 October 2014, doi: 10.3389/fimmu.2014.00520). For example, the Fc region of the antibody of the present disclosure may comprise two or three constant domains, i.e., a CH2 domain, a CH3 domain, and optionally a CH4 domain. In some embodiments, the antibody Fc region may also bear an IgG hinge region or a portion of the IgG hinge region, such as an IgG1 hinge region or a portion of the IgG1 hinge region, at the N-terminus. The hinge region may comprise a mutation(s). In some embodiments, the hinge region portion may be EPKSS or EPKSC.

Preferably, the Fc region of the antibody of the present disclosure comprises CH2-CH3 from N-terminus to C-terminus, or comprises hinge region-CH2-CH3 from N-terminus to C-terminus. In some embodiments, the Fc region suitable for use in the antibody or the multispecific antibody of the present disclosure is a human IgG Fc, such as a human IgG1 Fc, a human IgG2 Fc, a human IgG3, or a human IgG4 Fc. In one embodiment, the Fc region derives from a human IgG1 Fc, e.g., comprises or consists of an amino acid sequence set forth in SEQ ID NO: 80 or 98, or an amino acid sequence having at least 90% identity, e.g., 95%, 96%, 97%, 99% or higher identity thereto.

The Fc region in the antibody or the multispecific antibody of the present disclosure may be mutated to obtain desired properties. Mutations for the Fc region are known in the art.

In one embodiment, the Fc region is modified in properties of the effector function of the Fc region (e.g., complement activation function of the Fc region). In one embodiment, the effector function has been reduced or eliminated relative to a wild-type isotype Fc region. In one embodiment, the effector function is reduced or eliminated by using a method selected from: using an Fc isotype that naturally has a reduced or an eliminated effector function, and performing Fc region modification.

In one preferred embodiment, the Fc region has a reduced effector function mediated by the Fc region, such as a reduced or an eliminated ADCC or ADCP or CDC effector function, for example, comprising a mutation(s) for achieving the above function.

As understood by those skilled in the art, according to the expected use of the antibody molecule of the present disclosure, the antibody molecule of the present disclosure may further comprise a modification in the Fc domain that alters the binding affinity to one or more Fc receptors. In one embodiment, the Fc receptor is an Fcγ receptor, in particular a human Fcγ receptor. In some embodiments, the Fc region comprises a mutation(s) that reduces binding to the Fcγ receptor. For example, in some embodiments, the Fc region used in the present disclosure has one or more of an L234A/L235A mutation, a D265A mutation, and a P329A mutation, which reduce binding to the Fcγ receptor. In some embodiments, the Fc region used in the present disclosure has an L234A/L235A mutation, a D265A mutation, and a P329A mutation, which reduce binding to the Fcγ receptor. In yet another preferred embodiment, the Fc fragment may have a mutation(s) that leads to an increased serum half-life, e.g., a mutation(s) that improves the binding of the Fc fragment to FcRn. In some embodiments, the Fc region comprising a mutation(s) that reduces binding to the Fcγ receptor comprises or consists of an amino acid sequence set forth in SEQ ID NO: 90 or 97, or an amino acid sequence having at least 90% identity, e.g., 95%, 96%, 97%, 99% or higher identity thereto. In some embodiments, the Fc region comprises an amino acid sequence having at least 90% identity, e.g., 95%, 96%, 97%, 99% or higher identity, to SEQ ID NO: 90 or 97 and comprises an L234A/L235A mutation, a D265A mutation, and a P329A mutation.

As understood by those skilled in the art, to facilitate the formation of the multispecific antibody of the present disclosure as a heterodimer, the Fc region comprised in the multispecific antibody of the present disclosure may comprise mutations that favor the heterodimerization. In one embodiment, mutations are introduced into the CH3 regions of the two Fc regions.

Methods for facilitating the heterodimerization of the Fc regions are known in the art. For example, the CH3 region of the first Fc region and the CH3 region of the second Fc region are engineered in a complementary manner, such that each CH3 region (or the heavy chain comprising the same) can be no longer homodimerized with itself but forced to be heterodimerized with other CH3 regions that are complementarily engineered (such that heterodimerization occurs between the first and second CH3 regions and no homodimers are formed between the two first CH3 regions or the two second CH3 regions).

Preferably, based on the knob-in-hole technique, a corresponding knob mutation(s) and a corresponding hole mutation(s) are introduced into the first monomeric Fc region and the second monomeric Fc region, respectively. For this technique, see, e.g., Merchant, A. M., et al., (1998). "An efficient route to human bispecific IgG." Nat Biotechnol 16(7): 677-681.

In a particular embodiment, in the CH3 region of one Fc region, the threonine residue at position 366 is substituted with a tryptophan residue (T366W) (knob mutation); while in the CH3 region of the other Fc region, the tyrosine residue at position 407 is substituted with a valine residue (Y407V) (hole mutation). Optionally, the threonine residue at position 366 is substituted with a serine residue (T366S), and the leucine residue at position 368 is substituted with an alanine residue (L368A) (numbering according to the EU index).

In yet another embodiment, in the CH3 region of one Fc region, the knob mutations comprise or consist of: a substitution of the threonine residue at position 366 with a tryptophan residue (T366W) and a substitution of the serine residue at position 354 with a cysteine residue (S354C) or a substitution of the glutamic acid residue at position 356 with a cysteine residue (E356C) (in particular, a substitution of the serine residue at position 354 with a cysteine residue); while in the CH3 region of the other Fc region, the hole mutations comprise or consist of: a substitution of the tyrosine residue at position 407 with a valine residue (Y407V), optionally a substitution of the threonine residue at position 366 with a serine residue (T366S) and a substitution of the leucine residue at position 368 with an alanine residue (L368A) (numbering according to the EU index), and optionally a substitution of the tyrosine residue at position 349 with a cysteine residue (Y349C) (numbering according to the EU index).

In one specific embodiment, one Fc region comprises amino acid substitutions S354C and T366W (knob mutations), and the other Fc region comprises amino acid substitutions Y349C, T366S, L368A, and Y407V (hole mutations) (numbering according to the EU index).

Thus, in one specific embodiment, the two Fc regions comprised in the multispecific antibody of the present disclosure are heterodimerized, wherein
a) one Fc-region polypeptide comprises a mutation T366W, while the other Fc-region polypeptide comprises mutations T366S, L368A, and Y407V, or
b) one Fc-region polypeptide comprises mutations T366W and Y349C, while the other Fc-region polypeptide comprises mutations T366S, L368A, Y407V, and S354C, or
c) one Fc-region polypeptide comprises mutations T366W and S354C, while the other Fc-region polypeptide comprises mutations T366S, L368A, Y407V, and Y349C; and
optionally, the Fc region further comprises a mutation(s) that reduces binding to an Fcγ receptor, e.g., one or more of an L234A/L235A mutation, a D265A mutation, and a P329A mutation, for example, an L234A/L235A mutation, a D265A mutation, and a P329A mutation.

In some embodiments, the Fc region further comprises additional mutations that favor the purification of the heterodimer.

In one specific embodiment, the two Fc regions in the multispecific antibody of the present disclosure are heterodimerized.

The first Fc region comprises a knob mutation(s), and comprises or consists of an amino acid sequence set forth in SEQ ID NO: 47, 86, or 87, or an amino acid sequence having at least 90% identity, e.g., 95%, 96%, 97%, 99% or higher identity thereto. In some embodiments, the Fc region comprises an amino acid sequence having at least 90% identity, e.g., 95%, 96%, 97%, 99% or higher identity, to SEQ ID NO: 47, 86, or 87 and comprises knob mutations (e.g., S354C and T366W). In some embodiments, the Fc region comprises or does not comprise a hinge region EPKSS or EPKSC.

The second Fc region comprises a hole mutation(s), and comprises or consists of an amino acid sequence set forth in SEQ ID NO: 84, 85, or 111, or an amino acid sequence having at least 90% identity, e.g., 95%, 96%, 97%, 99% or higher identity thereto. In some embodiments, the Fc region comprises an amino acid sequence having at least 90% identity, e.g., 95%, 96%, 97%, 99% or higher identity, to SEQ ID NO:84, 85, or 111 and comprises a hole mutation(s) (e.g., T366S, L368A, Y407V, and Y349C). In some embodiments, the Fc region comprises or does not comprise a hinge region EPKSS or EPKSC.

### ➢ Antigen-binding region specifically binding to CD3

In some embodiments, the multispecific antibody comprising an antigen-binding region specifically binding to CD3 of the present disclosure binds to CD3 (e.g., human CD3 or monkey CD3, such as cynomolgus monkey CD3) with a desired affinity. In some embodiments, the multispecific antibody comprising an antigen-binding region specifically binding to CD3 of the present disclosure is capable of binding to both human CD3 and monkey CD3 (e.g., cynomolgus monkey CD3). In some embodiments, the affinity of the antibody is determined by bio-layer interferometry or surface plasmon resonance.

In some embodiments, the multispecific antibody comprising an antigen-binding region specifically binding to CD3 of the present disclosure binds to human CD3 or monkey CD3 (e.g., cynomolgus monkey CD3) with an equilibrium dissociation constant (K_{D}) of about 1-1000 nM. In some embodiments, the multispecific antibody comprising an antigen-binding region specifically binding to CD3 of the present disclosure binds to monkey CD3 (e.g., cynomolgus monkey CD3) with a K_{D} of about 10-100 nM, or 20-100 nM, or 50-100 nM. In some embodiments, the multispecific antibody comprising an antigen-binding region specifically binding to CD3 of the present disclosure binds to human CD3 with a K_{D} of about 100-1000 nM (e.g., about 200-1000 nM, 300-1000 nM, 400-1000 nM, or 500-1000 nM).

In some embodiments, the multispecific antibody comprising an antigen-binding region specifically binding to CD3 of the present disclosure binds to CD3 on the surface of an effector cell. In some embodiments, the multispecific antibody comprising an antigen-binding region specifically binding to CD3 of the present disclosure is capable of activating an effector cell. In some embodiments, the effector cell is a T cell. In some embodiments, the binding is determined by flow cytometry. In some embodiments, the activation effect of the antibody is detected using a reporter gene assay system (e.g., a Jurkat/NFAT-luc reporter gene system).

In some embodiments, the multispecific antibody comprising an antigen-binding region specifically binding to CD3 of the present disclosure is capable of activating effector cells to induce the killing of tumor cells.

In some embodiments, the antigen-binding region specifically binding to CD3 of the present disclosure comprises three complementarity determining regions from a heavy chain variable region (HCDRs): HCDR1, HCDR2, and HCDR3.

In some embodiments, the antigen-binding region specifically binding to CD3 of the present disclosure comprises three complementarity determining regions from a light chain variable region (LCDRs): LCDR1, LCDR2, and LCDR3.

In some embodiments, the antigen-binding region specifically binding to CD3 of the present disclosure comprises three complementarity determining regions from a heavy chain variable region (HCDRs) and three complementarity determining regions from a light chain variable region (LCDRs).

In some aspects, the antigen-binding region specifically binding to CD3 of the present disclosure comprises a heavy chain variable region (VH). In some aspects, the antigen-binding region specifically binding to CD3 of the present disclosure comprises a light chain variable region (VH). In some aspects, the antigen-binding region specifically binding to CD3 of the present disclosure comprises a heavy chain variable region (VH) and a light chain variable region (VL). In some embodiments, the heavy chain variable region comprises three complementarity determining regions (CDRs) from the heavy chain variable region: HCDR1, HCDR2, and HCDR3. In some embodiments, the light chain variable region comprises three complementarity determining regions (CDRs) from the light chain variable region: LCDR1, LCDR2, and LCDR3.

In some embodiments, the antigen-binding region specifically binding to CD3 of the present disclosure further comprises an antibody heavy chain constant region HC. In some embodiments, the antigen-binding region specifically binding to CD3 of the present disclosure further comprises an antibody light chain constant region LC. In some embodiments, the antigen-binding region specifically binding to CD3 of the present disclosure further comprises a heavy chain constant region HC and a light chain constant region LC.

In some embodiments, the heavy chain variable region VH of the antigen-binding region specifically binding to CD3 of the present disclosure
(i) comprises or consists of an amino acid sequence having at least 90%, 91%, 92%, 93%, 94%, 95%, 96%, 97%, 98%, or 99% identity to the amino acid sequence set forth in SEQ ID NO: 34;
(ii) comprises or consists of the amino acid sequence set forth in SEQ ID NO: 34; or
(iii) comprises or consists of an amino acid sequence having one or more (preferably not more than 10, and more preferably not more than 5, 4, 3, 2, or 1) amino acid modifications (preferably amino acid substitutions, and more preferably conservative amino acid substitutions) compared to the amino acid sequence set forth in SEQ ID NO: 34, wherein preferably, the amino acid modifications do not occur in the CDRs.

In some embodiments, the light chain variable region VL of the antigen-binding region specifically binding to CD3 of the present disclosure
(i) comprises or consists of an amino acid sequence having at least 90%, 91%, 92%, 93%, 94%, 95%, 96%, 97%, 98%, or 99% identity to the amino acid sequence set forth in SEQ ID NO: 35;
(ii) comprises or consists of the amino acid sequence set forth in SEQ ID NO: 35; or
(iii) comprises or consists of an amino acid sequence having one or more (preferably not more than 10, and more preferably not more than 5, 4, 3, 2, or 1) amino acid modifications (preferably amino acid substitutions, and more preferably conservative amino acid substitutions) compared to the amino acid sequence set forth in SEQ ID NO: 35, wherein preferably, the amino acid modifications do not occur in the CDRs.

In some embodiments, the three complementarity determining regions from a heavy chain variable region (HCDRs): HCDR1, HCDR2, and HCDR3 of the antigen-binding region specifically binding to CD3 of the present disclosure
(i) are three complementarity determining regions HCDR1, HCDR2, and HCDR3 contained in a VH set forth in SEQ ID NO: 34; or
(ii) comprises a sequence having a total of at least one and no more than 5, 4, 3, 2, or 1 amino acid modification (preferably amino acid substitutions, and more preferably conservative substitutions) relative to the sequence of (i).

Preferably, the HCDRs are determined according to Kabat.

In some embodiments, the three complementarity determining regions from a light chain variable region (LCDRs): LCDR1, LCDR2, and LCDR3 of the antigen-binding region specifically binding to CD3 of the present disclosure
(i) are three complementarity determining regions LCDR1, LCDR2, and LCDR3 contained in a VL set forth in SEQ ID NO: 35; or
(ii) comprises a sequence having a total of at least one and no more than 5, 4, 3, 2, or 1 amino acid modification (preferably amino acid substitutions, and more preferably conservative substitutions) relative to the sequence of (i).

Preferably, the LCDRs are determined according to Kabat.

In some embodiments, in the antigen-binding region specifically binding to CD3 of the present disclosure,
the HCDR1 comprises or consists of the amino acid sequence set forth in SEQ ID NO: 28; the HCDR2 comprises or consists of the amino acid sequence set forth in SEQ ID NO: 29; the HCDR3 comprises or consists of the amino acid sequence set forth in SEQ ID NO: 30; the LCDR1 comprises or consists of the amino acid sequence set forth in SEQ ID NO: 31; the LCDR2 comprises or consists of the amino acid sequence set forth in SEQ ID NO: 32; and/or the LCDR3 comprises or consists of the amino acid sequence set forth in SEQ ID NO: 33.

In some specific embodiments of the present disclosure, the antigen-binding region specifically binding to CD3 of the present disclosure comprises a VH and a VL, wherein
the VH comprises or consists of an amino acid sequence set forth in SEQ ID NO: 34 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 35 or an amino acid sequence having at least 90%, 91%, 92%, 93%, 94%, 95%, 96%, 97%, 98%, or 99% identity thereto.

In some specific embodiments of the present disclosure, the antigen-binding region specifically binding to CD3 of the present disclosure comprises three complementarity determining regions HCDR1, HCDR2, and HCDR3 contained in the VH set forth in SEQ ID NO: 34, and three complementarity determining regions LCDR1, LCDR2, and LCDR3 contained in the VL set forth in SEQ ID NO: 35.

In some specific embodiments of the present disclosure, the antigen-binding region specifically binding to CD3 of the present disclosure comprises: an HCDR1 set forth in SEQ ID NO: 28, an HCDR2 set forth in SEQ ID NO: 29, an HCDR3 set forth in SEQ ID NO: 30, an LCDR1 set forth in SEQ ID NO: 31, an LCDR2 set forth in SEQ ID NO: 32, and an LCDR3 set forth in SEQ ID NO: 33.

In one embodiment of the present disclosure, the amino acid modifications described herein include amino acid substitutions, insertions, or deletions. In one preferred embodiment, the amino acid modifications described herein occur in a region outside the CDR (e.g., in FR). More preferably, the amino acid modifications described herein occur in a region outside the heavy chain variable region and/or outside the light chain variable region. Preferably, the amino acid modification described herein is an amino acid substitution, preferably a conservative substitution.

In some embodiments, the antigen-binding region specifically binding to CD3 of the present disclosure comprises a heavy chain constant region or a fragment thereof (e.g., CH1), which is a heavy chain constant region or a fragment thereof of IgG1, IgG2, IgG3, or IgG4. In some embodiments, the antigen-binding region specifically binding to CD3 of the present disclosure comprises a light chain constant region or a fragment thereof, which is a kappa or lambda light chain constant region or a fragment thereof, e.g., a lambda light chain constant region or a fragment thereof.

In one embodiment, the antigen-binding region specifically binding to CD3 of the present disclosure is an anti-CD3 antibody fragment (e.g., an antigen-binding fragment), preferably an antibody fragment selected from: a Fab, a Fab', a Fab'-SH, an Fv, a single-chain antibody (e.g., scFv), an (Fab')₂, a single-domain antibody (e.g., VHH), a domain antibody (dAb), or a linear antibody.

In one embodiment, the antigen-binding region specifically binding to CD3 of the present disclosure is a Fab comprising the heavy chain variable region VH and the light chain variable region VL described in this section.

In some embodiments, the Fab heavy chain comprises a VH and a CH1 (and optionally comprises a hinge region portion EPKSS or EPKSC at the C-terminus of the CH1), wherein the VH is the VH of an anti-CD3 antibody.

In some embodiments, the Fab light chain comprises a VL-CL, wherein the VL is the VL of an anti-CD3 antibody. In some embodiments, the CL is a light chain constant region from an antibody kappa or lambda light chain, preferably a light chain constant region from a kappa light chain.

In some embodiments, the Fab heavy chain
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 11;
(ii) comprises or consists of the amino acid sequence selected from SEQ ID NO: 11; or
(iii) comprises or consists of an amino acid sequence having one or more (preferably not more than 10 or 10, and more preferably not more than 5, 4, 3, 2, or 1) amino acid modifications (preferably amino acid substitutions, and more preferably conservative amino acid substitutions) compared to the amino acid sequence selected from SEQ ID NO: 11.

In some embodiments, the Fab light chain
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 60;
(ii) comprises or consists of the amino acid sequence selected from SEQ ID NO: 60; or
(iii) comprises or consists of an amino acid sequence having one or more (preferably not more than 10 or 10, and more preferably not more than 5, 4, 3, 2, or 1) amino acid modifications (preferably amino acid substitutions, and more preferably conservative amino acid substitutions) compared to the amino acid sequence selected from SEQ ID NO: 60.

In one specific embodiment, the antigen-binding region specifically binding to CD3 of the present disclosure is an scFv comprising the heavy chain variable region VH and the light chain variable region VL described above. In some embodiments, the scFv comprises, from N-terminus to C-terminus, the heavy chain variable region VH described in this section, a linker, and the light chain variable region described in this section. In some embodiments, the scFv comprises the sequence set forth in SEQ ID NO: 109 or a sequence having at least 90%, 91%, 92%, 93%, 94%, 95%, 96%, 97%, 98%, 99% or more sequence identity and the same CDRs compared to the sequence set forth in SEQ ID NO: 109.

### ➢ Antigen-binding region specifically binding to GPRC5D

### Antigen-binding region specifically binding to GPRC5D from an anti-GPRC5D single-domain antibody

In some embodiments, the antigen-binding region is derived from a VHH antibody specifically binding to GPRC5D, e.g., the anti-GPRC5D VHH described herein.

### Antigen-binding region from an anti-GPRC5D full-length antibody or an antigen-binding fragment thereof

In some embodiments, the antigen-binding region is derived from an antibody specifically binding to GPRC5D, e.g., the antibody or the antigen-binding fragment thereof specifically binding to GPRC5D disclosed in PCT/CN2023/071314.

In some embodiments, the antibody specifically binding to GPRC5D comprises three complementarity determining regions from a heavy chain variable region (HCDRs): HCDR1, HCDR2, and HCDR3, wherein the HCDR1, HCDR2, and HCDR3 are HCDR1, HCDR2, and HCDR3 of the heavy chain variable region set forth in SEQ ID NO: 42.

In some embodiments, the antibody specifically binding to GPRC5D comprises three complementarity determining regions from a light chain variable region (LCDRs): LCDR1, LCDR2, and LCDR3, wherein the LCDR1, LCDR2, and LCDR3 are LCDR1, LCDR2, and LCDR3 of the light chain variable region set forth in SEQ ID NO: 43.

In some embodiments, in the antibody specifically binding to GPRC5D, the HCDR1 comprises or consists of the amino acid sequence set forth in SEQ ID NO: 36, the HCDR2 comprises or consists of the amino acid sequence set forth in SEQ ID NO: 37, the HCDR3 comprises or consists of the amino acid sequence set forth in SEQ ID NO: 38, the LCDR1 comprises or consists of the amino acid sequence set forth in SEQ ID NO: 39, the LCDR2 comprises or consists of the amino acid sequence set forth in SEQ ID NO: 40, and/or the LCDR3 comprises or consists of the amino acid sequence set forth in SEQ ID NO: 41.

In some embodiments, the antibody specifically binding to GPRC5D comprises three complementarity determining regions from a heavy chain variable region (HCDRs): HCDR1, HCDR2, and HCDR3 and three complementarity determining regions from a light chain variable region (LCDRs): LCDR1, LCDR2, and LCDR3, wherein the HCDR1 comprises or consists of the amino acid sequence set forth in SEQ ID NO: 36, the HCDR2 comprises or consists of the amino acid sequence set forth in SEQ ID NO: 37, the HCDR3 comprises or consists of the amino acid sequence set forth in SEQ ID NO: 38, the LCDR1 comprises or consists of the amino acid sequence set forth in SEQ ID NO: 39, the LCDR2 comprises or consists of the amino acid sequence set forth in SEQ ID NO: 40, and the LCDR3 comprises or consists of the amino acid sequence set forth in SEQ ID NO: 41.

In some aspects, the antibody specifically binding to GPRC5D comprises a heavy chain variable region (VH), wherein the VH
(i) comprises or consists of an amino acid sequence having at least 90%, 91%, 92%, 93%, 94%, 95%, 96%, 97%, 98%, or 99% identity to the amino acid sequence set forth in SEQ ID NO: 42;
(ii) comprises or consists of the amino acid sequence set forth in SEQ ID NO: 42; or
(iii) comprises or consists of an amino acid sequence having one or more (preferably not more than 10, and more preferably not more than 5, 4, 3, 2, or 1) amino acid modifications (preferably amino acid substitutions, and more preferably conservative amino acid substitutions) compared to the amino acid sequence set forth in SEQ ID NO: 42, wherein preferably, the amino acid modifications do not occur in the CDRs.

In some aspects, the antibody specifically binding to GPRC5D comprises a light chain variable region (VL), wherein the VL
(i) comprises or consists of an amino acid sequence having at least 90%, 91%, 92%, 93%, 94%, 95%, 96%, 97%, 98%, or 99% identity to the amino acid sequence set forth in SEQ ID NO: 43;
(ii) comprises or consists of the amino acid sequence set forth in SEQ ID NO: 43; or
(iii) comprises or consists of an amino acid sequence having one or more (preferably not more than 10, and more preferably not more than 5, 4, 3, 2, or 1) amino acid modifications (preferably amino acid substitutions, and more preferably conservative amino acid substitutions) compared to the amino acid sequence set forth in SEQ ID NO: 43, wherein preferably, the amino acid modifications do not occur in the CDRs.

In some embodiments, the antibody specifically binding to GPRC5D comprises a VH and a VL, wherein the VH comprises or consists of the sequence set forth in SEQ ID NO: 42, and/or the VL comprises or consists of the sequence set forth in SEQ ID NO: 43.

In some embodiments, the antigen-binding region comprises 1, 2, 3, 4, 5, or 6 CDRs of a known antibody specifically binding to GPRC5D (e.g., the antibody specifically binding to GPRC5D disclosed in PCT/CN2023/071314) or of the antibody specifically binding to GPRC5D described above.

In some embodiments, the antigen-binding region comprises 1, 2, and 3 heavy chain variable region CDRs, i.e., HCDR1, HCDR2, and HCDR3, of a known antibody specifically binding to GPRC5D (e.g., the antibody specifically binding to GPRC5D disclosed in PCT/CN2023/071314) or of the antibody specifically binding to GPRC5D described above.

In some embodiments, the antigen-binding region comprises 1, 2, and 3 light chain variable region CDRs, i.e., LCDR1, LCDR2, and LCDR3, of a known antibody specifically binding to GPRC5D (e.g., the antibody specifically binding to GPRC5D disclosed in PCT/CN2023/071314) or of the antibody specifically binding to GPRC5D described above.

In some embodiments, the antigen-binding region comprises 3 heavy chain variable region CDRs and 3 light chain variable region CDRs of a known antibody specifically binding to GPRC5D (e.g., the antibody specifically binding to GPRC5D disclosed in PCT/CN2023/071314) or of the antibody specifically binding to GPRC5D described above.

In some embodiments, the antigen-binding region comprises the heavy chain variable region and the light chain variable region of a known antibody specifically binding to GPRC5D (e.g., the antibody specifically binding to GPRC5D disclosed in PCT/CN2023/071314) or of the antibody specifically binding to GPRC5D described above, as well as the mutations described herein.

Some antigen-binding regions comprise the Fab of a known antibody specifically binding to GPRC5D (e.g., the antibody specifically binding to GPRC5D disclosed in PCT/CN2023/071314) or of the antibody specifically binding to GPRC5D described above.

In one embodiment, the antigen-binding region specifically binding to GPRC5D of the present disclosure is a Fab comprising the heavy chain variable region VH and the light chain variable region VL described in this section.

In some embodiments, the Fab heavy chain comprises a VH and a CH1 (and optionally comprises a hinge region portion EPKSS or EPKSC at the C-terminus of the CH1), wherein the VH is the VH of an anti-GPRC5D antibody.

In some embodiments, the Fab light chain comprises a VL-CL, wherein the VL is the VL of an anti-GPRC5D antibody. In some embodiments, the CL is a light chain constant region from an antibody kappa or lambda light chain, preferably a light chain constant region from a kappa light chain.

In some embodiments, the Fab heavy chain
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 110;
(ii) comprises or consists of the amino acid sequence selected from SEQ ID NO: 110; or
(iii) comprises or consists of an amino acid sequence having one or more (preferably not more than 10 or 10, and more preferably not more than 5, 4, 3, 2, or 1) amino acid modifications (preferably amino acid substitutions, and more preferably conservative amino acid substitutions) compared to the amino acid sequence selected from SEQ ID NO: 110.

In some embodiments, the Fab light chain
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 62;
(ii) comprises or consists of the amino acid sequence selected from SEQ ID NO: 62; or
(iii) comprises or consists of an amino acid sequence having one or more (preferably not more than 10 or 10, and more preferably not more than 5, 4, 3, 2, or 1) amino acid modifications (preferably amino acid substitutions, and more preferably conservative amino acid substitutions) compared to the amino acid sequence selected from SEQ ID NO: 62.

### ➢ Exemplary bispecific antibody molecule

In some preferred embodiments, the present disclosure provides a bispecific antibody comprising a VHH fragment specifically binding to BCMA and an antigen-binding region specifically binding to CD3, and optionally an Fc region.

In some embodiments, the present disclosure provides a bispecific antibody comprising a first antigen-binding region which is a Fab fragment or an scFv fragment specifically binding to CD3, a second antigen-binding region which is a VHH specifically binding to BCMA, and an Fc dimer.

Therefore, in some embodiments, the present disclosure relates to a bispecific antibody which is an IgG-like bispecific antibody comprising one or two anti-BCMA VHHs, one anti-CD3 scFv, and an Fc heterodimer, wherein
the scFv comprises a VH-VL (optionally the VH and VL are fused via a linker), and the VHH comprises or consists of a heavy chain variable region,
wherein the C-terminus of the VL of the scFv is fused to a CH2 or a hinge region of a first Fc region (e.g., comprising a knob mutation(s) or comprising a hole mutation(s)) to form a first heavy chain; and
the C-terminus of the one anti-BCMA VHH or of the two anti-BCMA VHHs in tandem is fused to a second Fc region (e.g., comprising a hole mutation(s) or comprising a knob mutation(s)) to form a second heavy chain (e.g., the C-terminus of the VHH is fused to a CH2 or a hinge region of the second Fc region).

In some specific embodiments, the present disclosure relates to a bispecific antibody which is an IgG-like bispecific antibody comprising one or two anti-BCMA VHHs, one anti-CD3 scFv, and an Fc heterodimer, wherein
the scFv comprises a VH-VL (optionally the VH and VL are fused via a linker), and the VHH comprises or consists of a heavy chain variable region,
wherein the C-terminus of the VL of the scFv is fused to a CH2 or a hinge region of a first Fc region comprising a knob mutation(s) to form a first heavy chain; and
the C-terminus of the one anti-BCMA VHH or of the two anti-BCMA VHHs in tandem is fused to a second Fc region comprising a hole mutation(s) to form a second heavy chain (e.g., the C-terminus of the VHH is fused to a CH2 or a hinge region of the second Fc region).

In some embodiments, the present disclosure relates to a bispecific antibody which is an IgG-like bispecific antibody comprising one anti-BCMA VHH or two anti-BCMA VHHs in tandem, one anti-CD3 antibody Fab, and an Fc heterodimer.

The Fab fragment comprises a VH-CH1 and a VL-CL, and the VHH comprises a heavy chain variable region.

The C-terminus of the CH1 of the Fab fragment is fused to a CH2 or a hinge region of a first Fc region (e.g., comprising a knob mutation(s) or comprising a hole mutation(s)) to form a first heavy chain; and
the C-terminus of the one anti-BCMA VHH or of the two anti-BCMA VHHs in tandem is fused to a second Fc region (e.g., comprising a hole mutation(s) or comprising a knob mutation(s)) to form a second heavy chain (e.g., the C-terminus of the VHH is fused to a CH2 or a hinge region of the second Fc region).

In some embodiments, the Fc region comprises or does not comprise a hinge region. In some embodiments, the hinge region is selected from EPKSS or EPKSC. In some embodiments, the fusion is direct fusion or fusion via a linker or a linker peptide. In some embodiments, the linker peptide is selected from (GGGGS)n, where n =1, 2, 3, or 4.

In some embodiments, the first heavy chain comprises or consists of the amino acid sequence set forth in SEQ ID NO: 57, or an amino acid sequence having at least 85%, 90%, 91%, 92%, 93%, 94%, 95%, 96%, 97%, 98%, or 99% identity thereto; and/or
the second heavy chain comprises or consists of an amino acid sequence set forth in any one of SEQ ID NOs: 56, 102, 103, and 104, or an amino acid sequence having at least 85%, 90%, 91%, 92%, 93%, 94%, 95%, 96%, 97%, 98%, or 99% identity thereto.

In some embodiments, the bispecific antibody comprises or consists of the first heavy chain and the second heavy chain described herein.

### ➢ Exemplary trispecific antibody

In some embodiments, the present disclosure provides a trispecific antibody comprising a first antigen-binding region specifically binding to CD3, a second antigen-binding region specifically binding to BCMA, and a third antigen-binding region specifically binding to GPRC5D, and an Fc dimer, e.g., an Fc heterodimer.

In some embodiments, the first antigen-binding region specifically binding to CD3 comprises or consists of a Fab fragment or an scFv fragment of an anti-CD3 antibody.

In some embodiments, the second antigen-binding region specifically binding to BCMA comprises or consists of an anti-BCMA VHH antibody.

In some embodiments, the third antigen-binding region specifically binding to GPRC5D comprises or consists of a GPRC5D VHH antibody, or comprises or consists of an anti-GPRC5D Fab fragment.

In some embodiments, the present disclosure provides a trispecific antibody comprising one anti-GPRC5D VHH, one anti-BCMA VHH, one anti-CD3 Fab fragment, and an Fc dimer.

The Fab fragment comprises a VH-CH1 (optionally comprising a hinge region portion EPKSS or EPKSC) and a VL-CL, and the VHH comprises a heavy chain variable region. The trispecific antibody comprises or consists of a first heavy chain, a second heavy chain, and a light chain.

The first heavy chain comprises, from N-terminus to C-terminus: the anti-GPRC5D VHH, the anti-BCMA VHH, and a first Fc region (e.g., comprising a hole or knob mutation(s)), which are fused with or without a linker(s); or the anti-BCMA VHH, the anti-GPRC5D VHH, and a first Fc region (e.g., comprising a hole or knob mutation(s)), which are fused with or without a linker(s);
the second heavy chain comprises, from N-terminus to C-terminus: the heavy chain of the anti-CD3 Fab fragment and a second Fc region (e.g., comprising a knob or hole mutation(s)), which are fused with or without a linker; and
the light chain comprises: the light chain of the anti-CD3 Fab fragment.

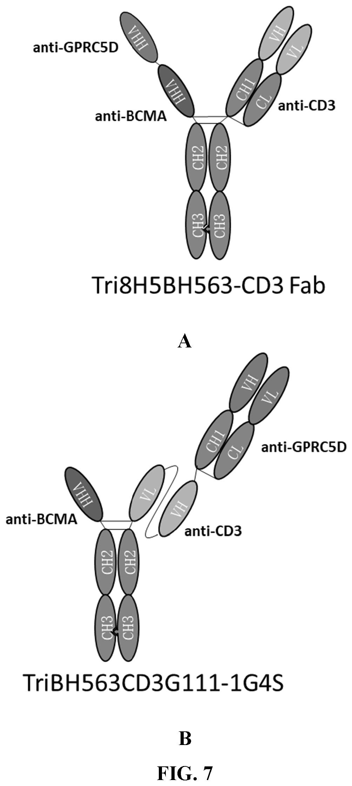

For example, the antibody has the structure shown in FIG. 7A.

In some embodiments, the trispecific antibody comprises or consists of a first heavy chain, a second heavy chain, and a light chain, wherein
the first heavy chain comprises or consists of the amino acid sequence set forth in SEQ ID NO: 58, or an amino acid sequence having at least 85%, 90%, 91%, 92%, 93%, 94%, 95%, 96%, 97%, 98%, or 99% identity thereto;
the second heavy chain comprises or consists of the amino acid sequence set forth in SEQ ID NO: 59, or an amino acid sequence having at least 85%, 90%, 91%, 92%, 93%, 94%, 95%, 96%, 97%, 98% or 99% identity thereto; and/or
the light chain comprises or consists of the amino acid sequence set forth in SEQ ID NO: 60, or an amino acid sequence having at least 85%, 90%, 91%, 92%, 93%, 94%, 95%, 96%, 97%, 98%, or 99% identity thereto.

In some embodiments, the present disclosure provides a trispecific antibody comprising one anti-GPRC5D Fab fragment, one anti-BCMA VHH, one anti-CD3 scFv, and an Fc dimer.

The Fab fragment comprises a VH-CH1 (optionally comprising a hinge region portion EPKSS or EPKSC) and a VL-CL, the VHH comprises a heavy chain variable region, and the scFv comprises a VH-VL. The trispecific antibody comprises or consists of a first heavy chain, a second heavy chain, and a light chain.

The first heavy chain comprises, from N-terminus to C-terminus: the anti-BCMA VHH and a first Fc region (e.g., comprising a hole or knob mutation(s)), which are fused with or without a linker; and
the second heavy chain comprises, from N-terminus to C-terminus: the heavy chain of the anti-GPRC5D Fab fragment, the anti-CD3 scFv, and a second Fc region (e.g., comprising a knob or hole mutation(s)), which are fused with or without a linker(s); and
the light chain comprises: the light chain of the anti-GPRC5D Fab fragment.

For example, the antibody has the structure shown in FIG. 7B.

In some embodiments, the trispecific antibody comprises or consists of a first heavy chain, a second heavy chain, and a light chain, wherein
the first heavy chain comprises or consists of the amino acid sequence set forth in SEQ ID NO: 56, or an amino acid sequence having at least 85%, 90%, 91%, 92%, 93%, 94%, 95%, 96%, 97%, 98%, or 99% identity thereto;
the second heavy chain comprises or consists of an amino acid sequence set forth in SEQ ID NO: 61, or an amino acid sequence having at least 85%, 90%, 91%, 92%, 93%, 94%, 95%, 96%, 97%, 98%, or 99% identity thereto; and/or
the light chain comprises or consists of the amino acid sequence set forth in SEQ ID NO: 62, or an amino acid sequence having at least 85%, 90%, 91%, 92%, 93%, 94%, 95%, 96%, 97%, 98%, or 99% identity thereto.

In some embodiments, the Fc region comprises or does not comprise a hinge region. In some embodiments, the hinge region is selected from EPKSS or EPKSC. In some embodiments, the fusion is direct fusion or fusion via a linker or a linker peptide. When the CH1 comprises a hinge region portion EPKSS or EPKSC and is linked to the Fc region, the Fc region need not comprise the hinge region portion.

In some embodiments, the linker peptide is selected from (GGGGS)n or (GGSGG)n, where n = 1, 2, 3, or 4.

In some embodiments, the first Fc region comprises a knob mutation(s), and the second Fc region comprises a hole mutation(s). In some embodiments, the first Fc region comprises a hole mutation(s), and the second Fc region comprises a knob mutation(s).

In some embodiments, one or both of the first and second Fc regions comprise L234A and L235A mutations and/or D265A and/or P329A. In some embodiments, the first Fc region comprises a knob mutation(s), L234A and L235A mutations, D265A, and P329A, and the second Fc region comprises a hole mutation(s), L234A and L235A mutations, D265A, and P329A. In some embodiments, the first Fc region comprises a hole mutation(s), L234A and L235A mutations, D265A, and P329A, and the second Fc region comprises a knob mutation(s), L234A and L235A mutations, D265A, and P329A.

### VI. Nucleic Acid Encoding Antibody and Host Cell Comprising the Same

In one aspect, the present disclosure provides a nucleic acid encoding any of the above anti-BCMA antibodies or multispecific antibodies or any one of the chains thereof.

For example, the nucleic acid of the present disclosure comprises a nucleic acid encoding an amino acid sequence selected from any one of SEQ ID NOs: 4, 8, 10, 12, 14, 15, 16, 18, 21, 22, 23, 44-46, 48-55, 56, 58, 59, 61, and 102-104, or a nucleic acid encoding an amino acid sequence having at least 85%, 90%, 91%, 92%, 93%, 94%, 95%, 96%, 97%, 98%, or 99% identity to an amino acid sequence selected from any one of SEQ ID NOs: 4, 8, 10, 12, 14, 15, 16, 18, 21, 22, 23, 44-46, 48-55, 56, 58, 59, 61, and 102-104.

As will be understood by those skilled in the art, each antibody or polypeptide amino acid sequence may be encoded by a variety of nucleic acid sequences because of codon degeneracy. Nucleic acid sequences encoding the molecules of the present disclosure may be produced using methods well known in the art, for example by de novo solid-phase DNA synthesis, or by PCR amplification.

In one aspect, the present disclosure provides a nucleic acid encoding any of the above antibodies or any of the chains of the antibodies. When expressed in a suitable expression vector, a polypeptide encoded by the nucleic acid is capable of exhibiting binding capacity to human BCMA antigen.

In a further aspect, the present disclosure provides a nucleic acid encoding a multispecific antibody. When expressed in a suitable expression vector, a polypeptide encoded by the nucleic acid is capable of exhibiting binding capacity to human or monkey (e.g., cynomolgus monkey) CD3 and human BCMA antigen, and optionally human or monkey (e.g., cynomolgus monkey) GPRC5D antigen. In one embodiment, the nucleic acids encoding the chains of the multispecific antibody may be in the same vector or in different vectors. In yet another embodiment, the nucleic acids encoding the chains of the multispecific antibody may be introduced into the same or different host cells for expression. Thus, in some embodiments, the method for producing the multispecific antibody of the present disclosure comprises a step of: culturing a host cell comprising a nucleic acid encoding each chain of the molecule under a condition suitable for expressing the chain to produce the multispecific antibody of the present disclosure.

In one embodiment, provided is a vector comprising the nucleic acid. In one embodiment, the vector is an expression vector, e.g., a eukaryotic expression vector. The vector includes, but is not limited to, a virus, a plasmid, a cosmid, a λ phage, or a yeast artificial chromosome (YAC). In one embodiment, the vector is, for example, a pcDNA vector, such as pcDNA3.1.

In one embodiment, provided is a host cell comprising the nucleic acid or the vector, e.g., for cloning or expressing a vector encoding the anti-BCMA antibody or the multispecific antibody. In one embodiment, the host cell is eukaryotic. In another embodiment, the host cell is selected from a yeast cell, a mammalian cell (e.g., a CHO cell (e.g., CHO-S, such as ExpiCHO-S) or 293 cell (e.g., 293F or HEK293 cell)), or other cells suitable for preparing an antibody or a fragment thereof. In one embodiment, the host cell is prokaryotic, e.g., a bacterium, such as *E. coli*.

In one embodiment, the host cell is eukaryotic. In another embodiment, the host cell is selected from yeast cells, mammalian cells, or other cells suitable for preparing an antibody or a fragment thereof. For example, eukaryotic microorganisms, such as filamentous fungi or yeast, are suitable cloning or expression hosts for the vector encoding the antibody. For example, fungus and yeast strains in which a glycosylation pathway has been "humanized" produce antibodies having a partial or full human glycosylation pattern. Host cells suitable for expressing a glycosylated antibody are also derived from multicellular organisms (invertebrates and vertebrates). Vertebrate cells may also be used as hosts. For example, a mammalian cell line engineered to be suitable for suspension growth may be used. Other examples of useful mammalian host cell lines are monkey kidney CV1 line (COS-7) transformed with SV40, human embryonic kidney line (HEK293, 293F, or 293T cells), and the like. Other useful mammalian host cell lines include Chinese hamster ovary (CHO) cells, including DHFR-CHO cells, CHO-S cells, ExpiCHO, and the like; and myeloma cell lines such as Y0, NS0, and Sp2/0. Mammalian host cell lines suitable for producing antibodies are known in the art.

### VII. Production and Purification of Anti-BCMA Antibody or Multispecific Antibody of the Present Disclosure

In one embodiment, provided is a method for preparing the anti-BCMA antibody or the multispecific antibody of the present disclosure, wherein the method comprises culturing a host cell comprising a nucleic acid encoding the anti-BCMA antibody or the multispecific antibody (e.g., any one and/or more polypeptide chains) or an expression vector comprising the nucleic acid, as provided above, under a condition suitable for expressing the anti-BCMA antibody or the multispecific antibody or the chain thereof, and optionally recovering the anti-BCMA antibody or the multispecific antibody from the host cell (or the host cell medium).

Polynucleotides encoding the polypeptide chains of the anti-BCMA antibody or the multispecific antibody of the present disclosure may be inserted into one or more vectors for further cloning and/or expression in host cells. Methods known to those skilled in the art can be used to construct expression vectors. Once the expression vector comprising one or more nucleic acid molecules of the present disclosure has been prepared for expression, the expression vector may be transfected or introduced into suitable host cells. Various techniques may be used for this purpose, for example, protoplast fusion, calcium phosphate precipitation, electroporation, retroviral transduction, viral transfection, biolistics, liposome-based transfection, or other conventional techniques.

The anti-BCMA antibody or the multispecific antibody prepared as described herein may be purified by known prior art such as high-performance liquid chromatography, ion exchange chromatography, gel electrophoresis, affinity chromatography, and size exclusion chromatography. The actual conditions used for purifying a particular protein also depend on factors such as net charge, hydrophobicity, and hydrophilicity, and these will be apparent to those skilled in the art.

The purity of the antibody molecule of the present disclosure may be determined by any one of a variety of well-known analytical methods including size exclusion chromatography, gel electrophoresis, high performance liquid chromatography, and the like.

### VIII. Assay Method for Anti-BCMA Antibody or Multispecific Antibody

The anti-BCMA antibody or the multispecific antibody provided herein may be identified, screened, or characterized for physical/chemical properties and/or biological activities thereof through a variety of assays known in the art.

In one aspect, the anti-BCMA antibody or the multispecific antibody of the present disclosure are assayed for their binding activity to a target (e.g., an antigen, e.g., a free antigen or an antigen expressed on a cell), for example, by known methods such as bio-layer interferometry, ELISA, and flow cytometry. The binding to CD3 and/or BCMA and/or GPRC5D (or CD3 and/or BCMA and/or GPRC5D expressed on a cell) can be assayed using methods known in the art, and exemplary methods are disclosed herein. In some embodiments, the binding is determined by radioimmunoassay (RIA), bio-layer interferometry (BLI), electrochemiluminescence (ECL), surface plasmon resonance (SPR), or flow cytometry (FACS).

The present disclosure further provides an assay method for identifying the biological activity of the anti-BCMA antibody or the multispecific antibody. The biological activity is selected from the properties of the anti-BCMA antibody or the multispecific antibody of the present disclosure.

For example, the binding activity of the antibody molecules of the present disclosure to a cell expressing BCMA and/or GPRC5D may be determined by methods known in the art, such as fluorescent reporter molecule assay and flow cytometry, or by the exemplary methods disclosed in the examples herein, such as determining the binding of the antibody molecules of the present disclosure to BCMA and/or GPRC5D expressed on the cell.

For example, the activation activity of the antibody molecule of the present disclosure on a T cell may be determined by methods known in the art, such as a T cell activation assay system (e.g., an NFAT-luc reporter gene system such as a Jurkat/NFAT-luc reporter gene system). For example, the activation activity may be determined by the methods shown in the examples, through detecting a CD3 signaling pathway in the T cell or through detecting the release of cytokines (e.g., interferons such as IFNγ; tumor necrosis factors such as TNFα and/or interleukins such as IL-6) following activation of the T cell.

For example, the inhibitory activity or structural safety of the antibody molecule of the present disclosure against a tumor may be determined by methods known in the art, such as a tumor inhibition experiment conducted on a mouse tumor model.

The cells for use in any of the above *in-vitro* assays are primary cells or cell lines, including cells that naturally express or overexpress BCMA (e.g., human or monkey (e.g., cynomolgus monkey)) or GPRC5D (e.g., human or monkey (e.g., cynomolgus monkey) GPRC5D), for example, cells that overexpress BCMA or GPRC5D, e.g., BCMA- and GPRC5D-positive cells, such as NCI-H929, MM.1R, or MOLP-8.

It will be understood that any of the assays described above may be performed using a combination of the antibody of the present disclosure and other active agents.

### IX. Immunoconjugate, Pharmaceutical Composition, Pharmaceutical Combination Product, and Kit of Anti-BCMA Antibody or Multispecific Antibody of the Present Disclosure

In some embodiments, the present disclosure provides an immunoconjugate comprising any of the anti-BCMA antibody or the multispecific antibody described herein. Preferably, the immunoconjugate comprises one or more additional therapeutic agents (e.g., cytotoxins or small molecule compounds) or markers.

In some embodiments, the present disclosure provides a composition or a medicament or a formulation comprising any of the anti-BCMA antibody or the multispecific antibody described herein, and preferably the composition is a pharmaceutical composition.

In one embodiment, the composition further comprises a pharmaceutical supplementary material. In one embodiment, the composition, e.g., the pharmaceutical composition, comprises the anti-BCMA antibody or the multispecific antibody of the present disclosure, and a combination of one or more additional therapeutic agents.

The composition or the medicament or the formulation of the present disclosure may further comprise a suitable pharmaceutical supplementary material, such as a pharmaceutical carrier and a pharmaceutical excipient known in the art, including buffers.

As used herein, the "pharmaceutical carrier" includes any and all solvents, dispersion media, isotonic agents and absorption delaying agents, and the like that are physiologically compatible.

For use and application of the pharmaceutical supplementary materials, see Handbook of Pharmaceutical Excipients, 8th ed., R. C. Rowe, P. J. Seskey and S. C. Owen, Pharmaceutical Press, London, Chicago.

The composition or the medicament or the formulation of the present disclosure may be in a variety of forms. These forms include, for example, liquid, semi-solid, and solid dosage forms, such as liquid solutions (e.g., injectable solutions and eye drops), pulvis or suspensions, liposomes, and suppositories. The preferred form depends on the intended mode of administration and therapeutic use.

The medicament or the formulation comprising the anti-BCMA antibody or the multispecific antibody described herein may be prepared by mixing the anti-BCMA antibody or the multispecific antibody of the present disclosure having a desired purity with one or more optional pharmaceutical supplementary materials, e.g. in the form of a lyophilized formulation or an aqueous solution.

The composition or the medicament or the formulation of the present disclosure may further comprise more than one active ingredient required by a particular indication treated, preferably those having complementarity activity without adversely affecting one another. For example, it is desirable to further provide additional therapeutic agents.

The present disclosure further provides a pharmaceutical combination or a pharmaceutical combination product comprising the anti-BCMA antibody or the multispecific antibody of the present disclosure and one or more additional therapeutic agents.

The present disclosure further provides a kit of parts, comprising the pharmaceutical combination, wherein, for example, the kit of parts comprises in the same package:
- a first container containing a pharmaceutical composition comprising the anti-BCMA antibody or the multispecific antibody of the present disclosure; and
- a second container containing a pharmaceutical composition comprising an additional therapeutic agent.

In some embodiments, the additional therapeutic agent is, for example, a chemotherapeutic agent, a cytokine, a cytotoxic agent, an additional antibody, a small molecule drug, or an immunomodulatory agent (e.g., an immunosuppressive agent).

### X. Use of Anti-BCMA Antibody or Multispecific Antibody and Methods for Using the Same

In one aspect, the present disclosure provides a method for preventing or treating a disease in a subject, comprising administering to the subject the anti-BCMA antibody or the multispecific antibody, or the immunoconjugate, the composition or the medicament or the formulation comprising the same of the present disclosure. In some embodiments, the present disclosure provides a method for specifically activating a T cell in a subject, comprising administering to the subject the antibody or multispecific antibody, or the immunoconjugate, the composition or the medicament or the formulation comprising the same of the present disclosure.

In some embodiments, the disease is, for example, a tumor, e.g., a cancer. The cancer may be at an early, intermediate, or advanced stage, or may be a metastatic cancer. In some embodiments, the tumor may be a solid tumor or a hematological tumor. In some embodiments, the tumor is a myeloma, e.g., multiple myeloma such as metastatic multiple myeloma, colon cancer, rectal cancer, or colorectal cancer.

In some embodiments, the treatment of the disease will benefit from activation of a CD3 signaling pathway and/or activation of T cells.

In some embodiments, the tumor or cancer is a BCMA-positive tumor or cancer. In some embodiments, the BCMA-positive tumor or cancer is characterized by the presence of, or elevated levels of BCMA protein and/or nucleic acids in a patient with the tumor or cancer (e.g., in the tissues or cells of the patient's tumor or cancer) (e.g., compared to the BCMA protein and/or nucleic acid levels in the same tissue of a healthy individual, or compared to the BCMA protein and/or nucleic acid levels in the adjacent healthy tissue of the patient). For example, the tumor cells of the tumor or cancer may have or exhibit elevated levels of BCMA protein and/or nucleic acids (e.g., compared to the BCMA protein and/or nucleic acid levels in the cells of the same tissue of a healthy individual, or compared to the BCMA protein and/or nucleic acid levels in the healthy cells of the same tissue or in the cells of the adjacent healthy tissue of the patient).

In some embodiments, the tumor or cancer is a GRPC5D-positive tumor or cancer. In some embodiments, the GPRC5D-positive tumor or cancer is characterized by the presence of, or elevated levels of GPRC5D protein and/or nucleic acids in a patient with the tumor or cancer (e.g., in the tissues or cells of the patient's tumor or cancer) (e.g., compared to the GPRC5D protein and/or nucleic acid levels in the same tissue of a healthy individual, or compared to the GPRC5D protein and/or nucleic acid levels in the adjacent healthy tissue of the patient). For example, the tumor cells of the tumor or cancer may have or exhibit elevated levels of GPRC5D protein and/or nucleic acids (e.g., compared to the GPRC5D protein and/or nucleic acid levels in the cells of the same tissue of a healthy individual, or compared to the GPRC5D protein and/or nucleic acid levels in the healthy cells of the same tissue or in the cells of the adjacent healthy tissue of the patient).

In some embodiments, the tumor or cancer is a BCMA-positive and GPRC5D-positive tumor or cancer.

The anti-BCMA antibody or the multispecific antibody of the present disclosure (as well as the immunoconjugate, the composition, the pharmaceutical composition, the formulation, the combination product or the like comprising the same) may be administered by any suitable method, including parenteral administration, intrapulmonary administration, intranasal administration, and, if required by locoregional treatment, intralesional administration. Parenteral injection or infusion includes intramuscular, intravenous, intra-arterial, intraperitoneal or subcutaneous injection or infusion. The administration may be performed by any suitable route, such as injection, e.g., intravenous or subcutaneous injection, to some extent depending on short-term or long-term treatment. Various administration schedules are encompassed herein, including, but not limited to, single administration or multiple administrations at multiple time points, bolus injection, and pulse infusion.

In order to prevent or treat a disease, the appropriate dosage of the anti-BCMA antibody or the multispecific antibody (as well as the immunoconjugate, the composition, the pharmaceutical composition, the formulation, the combination product or the like comprising the same) of the present disclosure (when used alone or in combination with one or more additional therapeutic agents) will depend on the type of the disease to be treated, the type of the antibody, the severity and progression of the disease, purpose of administration (prophylactic or therapeutic), previous therapies, clinical histories of patients, responses to the antibody, and the discretion of an attending physician. The antibody is suitably administered to a patient through a single treatment or through a series of treatments. In other aspects, the present disclosure provides use of the anti-BCMA antibody or the multispecific antibody of the present disclosure, or the immunoconjugate or the composition or the combination product comprising the same in producing or preparing a medicament for the use described herein, e.g., for use in the prevention or treatment of the related disease or disorder mentioned herein.

In some embodiments, the anti-BCMA antibody or the multispecific antibody (as well as the immunoconjugate, the composition, the pharmaceutical composition, the formulation or the like comprising the same) may also be administered in combination with one or more additional therapies, e.g., therapeutic modalities and/or additional therapeutic agents, for the use described herein, e.g., for use in the prevention and/or treatment of the related disease or disorder mentioned herein.

In some embodiments, the therapeutic modality is, for example, surgical therapy or radiotherapy.

In some embodiments, the additional therapeutic agent is, for example, a chemotherapeutic agent, a cytokine, a cytotoxic agent, an additional antibody, a small molecule drug, or an immunomodulatory agent (e.g., an immunosuppressive agent).

### XI. Diagnosis and Detection

In one aspect, the present disclosure further relates to methods for diagnosis and detection (e.g., for diagnostic or non-diagnostic purposes) of the anti-BCMA antibody or the multispecific antibody, and a composition comprising the same for diagnosis and detection.

In certain embodiments, the anti-BCMA antibody provided herein may be used to detect the presence of BCMA in a biological sample. In certain embodiments, the bispecific antibody provided herein may be used to detect the presence of CD3 and/or BCMA in a biological sample. In certain embodiments, the trispecific antibody provided herein may be used to detect the presence of CD3 and/or BCMA and/or GPRC5D in a biological sample.

The term "detection" used herein includes quantitative and qualitative detections, and exemplary detections may involve immunohistochemistry, immunocytochemistry, flow cytometry (e.g., FACS), magnetic beads complexed with antibody molecules, ELISA, and PCR techniques (e.g., RT-PCR). In certain embodiments, the biological sample is a body fluid, such as blood, serum, or plasma.

In certain embodiments, the method comprises contacting a biological sample with the anti-BCMA antibody or the multispecific antibody described herein under a condition that allows the binding thereof to BCMA, and detecting whether a complex is formed between the anti-BCMA antibody or the multispecific antibody and BCMA. The formation of the complex indicates the presence of BCMA. The method may be an *in-vitro* or *in-vivo* method.

In certain embodiments, a labeled anti-BCMA antibody or multispecific antibody is provided. The label includes, but is not limited to, a label or moiety that is detected directly (such as a fluorescent label, a chromophoric label, an electron-dense label, a chemiluminescent label, and a radioactive label), and a moiety that is detected indirectly, such as an enzyme or a ligand, for example, by enzymatic reaction or molecular interaction. In some embodiments, the label is a marker such as biotin or hFc.

In some embodiments provided herein, the sample is obtained prior to treatment with the anti-BCMA antibody or the multispecific antibody of the present disclosure. In some embodiments, the sample is obtained prior to application of additional therapies. In some embodiments, the sample is obtained during treatment with additional therapies, or after treatment with other therapies.

In some embodiments, BCMA and/or GPRC5D is detected prior to treatment, e.g., prior to an initial treatment or prior to a certain treatment following a treatment interval.

### XII. Detailed Description

In some aspects, the present disclosure relates to the following specific embodiments:
1. A VHH antibody specifically binding to BCMA, comprising:
   three complementarity determining regions (CDRs) contained in a VHH set forth in any one of SEQ ID NOs: 4, 8, 10, 12, 14, 15, 16, 18, 21, 22, and 23, wherein
   preferably, sequences of the CDRs are defined according to IMGT.
2. The VHH antibody according to Embodiment 1, comprising complementarity determining regions (CDRs) VHH CDR1, VHH CDR2, and VHH CDR3, wherein
   (i) the VHH CDR1 comprises or consists of the amino acid sequence set forth in SEQ ID NO: 1, the VHH CDR2 comprises or consists of an amino acid sequence set forth in SEQ ID NO: 2, 9, or 13, and the VHH CDR3 comprises or consists of the amino acid sequence set forth in SEQ ID NO: 3;
   (ii) the VHH CDR1 comprises or consists of an amino acid sequence set forth in SEQ ID NO: 1 or 5, the VHH CDR2 comprises or consists of the amino acid sequence set forth in SEQ ID NO: 6, and the VHH CDR3 comprises or consists of the amino acid sequence set forth in SEQ ID NO: 7; or
   (iii) the VHH CDR1 comprises or consists of the amino acid sequence set forth in SEQ ID NO: 1, the VHH CDR2 comprises or consists of the amino acid sequence set forth in SEQ ID NO: 17, and the VHH CDR3 comprises or consists of the amino acid sequence set forth in SEQ ID NO: 7.
3. The VHH antibody according to Embodiment 1, comprising or consisting of a heavy chain variable region, wherein the heavy chain variable region
   (i) comprises or consists of an amino acid sequence having at least 90%, 91%, 92%, 93%, 94%, 95%, 96%, 97%, 98%, or 99% identity to an amino acid sequence selected from any one of SEQ ID NOs: 4, 8, 10, 12, 14, 15, 16, 18, 21, 22, and 23;
   (ii) comprises or consists of an amino acid sequence selected from any one of SEQ ID NOs: 4, 8, 10, 12, 14, 15, 16, 18, 21, 22, and 23; or
   (iii) comprises an amino acid sequence having one or more (preferably not more than 10, and more preferably not more than 5, 4, 3, 2, or 1) amino acid modifications (preferably amino acid substitutions, and more preferably conservative amino acid substitutions) compared to an amino acid sequence selected from any one of SEQ ID NOs: 4, 8, 10, 12, 14, 15, 16, 18, 21, 22, and 23, wherein preferably, the amino acid modifications do not occur in the CDRs.
4. A heavy-chain antibody specifically binding to BCMA, comprising the VHH antibody according to any one of Embodiments 1-3.
5. The heavy-chain antibody according to Embodiment 4, comprising the VHH antibody according to any one of Embodiments 1-3 linked to an antibody constant region or an Fc region, wherein preferably, the antibody constant region or the Fc region is from human IgG1, human IgG2, human IgG3, or human IgG4, optionally, the VHH antibody is linked to the Fc region by a hinge region or a portion thereof, and optionally, an amino acid sequence of the hinge region portion is EPKSS (SEQ ID NO: 94) or EPKSC (SEQ ID NO: 96).
6. The heavy-chain antibody according to Embodiment 4, comprising the VHH antibody according to any one of Embodiments 1-3 linked to an antibody Fc region, wherein the Fc region is an Fc region from human IgG1, IgG2, IgG3, or IgG4, optionally the Fc region comprises an L234A/L235A mutation, a D265A mutation and a P329A mutation, and preferably the Fc region
   (i) comprises or consists of an amino acid sequence having at least 85%, 90%, 91%, 92%, 93%, 94%, 95%, 96%, 97%, 98%, or 99% identity to an amino acid sequence set forth in SEQ ID NO: 80, 90, 97, or 98;
   (ii) comprises or consists of an amino acid sequence set forth in SEQ ID NO: 80, 90, 97, or 98; or
   (iii) comprises an amino acid sequence having one or more (preferably not more than 10, and more preferably not more than 5, 4, 3, 2, or 1) amino acid modifications (preferably amino acid substitutions, and more preferably conservative amino acid substitutions) compared to an amino acid sequence set forth in SEQ ID NO: 80, 90, 97, or 98.
7. The heavy-chain antibody according to Embodiment 4,
   (i) comprising or consisting of an amino acid sequence having at least 85%, 90%, 91%, 92%, 93%, 94%, 95%, 96%, 97%, 98%, or 99% identity to an amino acid sequence selected from any one of SEQ ID NOs: 44-46 and 48-55;
   (ii) comprising or consisting of an amino acid sequence selected from any one of SEQ ID NOs: 44-46 and 48-55; or
   (iii) comprising an amino acid sequence having one or more (preferably not more than 10, and more preferably not more than 5, 4, 3, 2, or 1) amino acid modifications (preferably amino acid substitutions, and more preferably conservative amino acid substitutions) compared to an amino acid sequence selected from any one of SEQ ID NOs: 44-46 and 48-55, wherein preferably, the amino acid modifications do not occur in the CDRs.
8. The VHH antibody according to any one of Embodiments 1-3 or the heavy-chain antibody according to any one of Embodiments 4-7, wherein the antibody is a chimeric antibody or a humanized antibody.
9. A multispecific antibody, comprising a first antigen-binding region and a second antigen-binding region, and optionally a third antigen-binding region, wherein the second antigen-binding region specifically binds to BCMA, and comprises the VHH antibody according to any one of Embodiments 1-3 and 8, or the heavy-chain antibody according to any one of Embodiments 4-8, and preferably the multispecific antibody being a bispecific antibody or a trispecific antibody.
10. The multispecific antibody according to Embodiment 9, wherein the first antigen-binding region specifically binds to CD3.
11. The multispecific antibody according to Embodiment 10, wherein the first antigen-binding region comprises a VH and a VL, wherein the VH comprises three complementarity determining regions from a heavy chain variable region (HCDRs): HCDR1, HCDR2, and HCDR3, and the VL comprises three complementarity determining regions from a light chain variable region (LCDRs): LCDR1, LCDR2, and LCDR3, wherein
   (i) the HCDR1, the HCDR2, and the HCDR3 are three complementarity determining regions HCDR1, HCDR2, and HCDR3 contained in a VH set forth in SEQ ID NO: 34, respectively; and the LCDR1, the LCDR2, and the LCDR3 are three complementarity determining regions LCDR1, LCDR2, and LCDR3 contained in a VL set forth in SEQ ID NO: 35, respectively; or
   (ii) the HCDR1 consists of the amino acid sequence set forth in SEQ ID NO: 28, the HCDR2 consists of the amino acid sequence set forth in SEQ ID NO: 29, the HCDR3 consists of the amino acid sequence set forth in SEQ ID NO: 30, the LCDR1 consists of the amino acid sequence set forth in SEQ ID NO: 31, the LCDR2 consists of the amino acid sequence set forth in SEQ ID NO: 32, and the LCDR3 consists of the amino acid sequence set forth in SEQ ID NO: 33.
12. The multispecific antibody according to Embodiment 11, wherein the first antigen-binding region comprises a VH and a VL, wherein
   the VH comprises or consists of the amino acid sequence set forth in SEQ ID NO: 34, or an amino acid sequence having at least 90%, 91%, 92%, 93%, 94%, 95%, 96%, 97%, 98%, or 99% identity thereto, and/or
   the VL comprises or consists of the amino acid sequence set forth in SEQ ID NO: 35, or an amino acid sequence having at least 90%, 91%, 92%, 93%, 94%, 95%, 96%, 97%, 98%, or 99% identity thereto.
13. The multispecific antibody according to any one of Embodiments 10-12, wherein the first antigen-binding region is a Fab or an scFv specifically binding to CD3.
14. The multispecific antibody according to Embodiment 13, wherein the Fab comprises a VH and a CH1 of the first antigen-binding region, and optionally a hinge region portion (e.g., EPKSS and EPKSC), wherein the CH1 is a CH1 from IgG1, IgG2, IgG3, or IgG4, preferably a CH1 from IgG1.
15. The multispecific antibody according to Embodiment 14, wherein the CH1
   (i) comprises or consists of an amino acid sequence having at least 85%, 90%, 91%, 92%, 93%, 94%, 95%, 96%, 97%, 98%, or 99% identity to the amino acid sequence selected from SEQ ID NO: 88; or
   (ii) comprises or consists of the amino acid sequence selected from SEQ ID NO: 88.
16. The multispecific antibody according to Embodiment 15, wherein the Fab heavy chain of the first antigen-binding region
   (i) comprises or consists of an amino acid sequence having at least 85%, 90%, 91%, 92%, 93%, 94%, 95%, 96%, 97%, 98%, or 99% identity to the amino acid sequence selected from SEQ ID NO: 11;
   (ii) comprises or consists of the amino acid sequence selected from SEQ ID NO: 11; or
   (iii) comprises or consists of an amino acid sequence having one or more (preferably not more than 10 or 10, and more preferably not more than 5, 4, 3, 2, or 1) amino acid modifications (preferably amino acid substitutions, and more preferably conservative amino acid substitutions) compared to the amino acid sequence selected from SEQ ID NO: 11; and/or
   the Fab light chain of the first antigen-binding region
   (i) comprises or consists of an amino acid sequence having at least 85%, 90%, 91%, 92%, 93%, 94%, 95%, 96%, 97%, 98%, or 99% identity to the amino acid sequence selected from SEQ ID NO: 60;
   (ii) comprises or consists of the amino acid sequence selected from SEQ ID NO: 60; or
   (iii) comprises or consists of an amino acid sequence having one or more (preferably not more than 10 or 10, and more preferably not more than 5, 4, 3, 2, or 1) amino acid modifications (preferably amino acid substitutions, and more preferably conservative amino acid substitutions) compared to the amino acid sequence selected from SEQ ID NO: 60.
17. The multispecific antibody according to Embodiment 13, wherein the scFv comprises, from N-terminus to C-terminus, a heavy chain variable region VH of the first antigen-binding region, a linker, and a light chain variable region of the first antigen-binding region.
18. The multispecific antibody according to Embodiment 17, wherein the scFv comprises the sequence set forth in SEQ ID NO: 109 or a sequence having at least 90%, 91%, 92%, 93%, 94%, 95%, 96%, 97%, 98%, 99% or more sequence identity and the same CDRs as compared to SEQ ID NO: 109.
19. The multispecific antibody according to any one of Embodiments 9-18, wherein the second antigen-binding region is the VHH according to any one of Embodiments 1-3 and 8.
20. The multispecific antibody according to any one of Embodiments 9-19, wherein the multispecific antibody is a trispecific antibody and comprises a third antigen-binding region specifically binding to GPRC5D.
21. The multispecific antibody according to Embodiment 20, wherein the third antigen-binding region comprises or consists of an anti-GPRC5D VHH.
22. The multispecific antibody according to Embodiment 21, wherein the anti-GPRC5D VHH comprises or consists of a heavy chain variable region comprising
   (i) three complementarity determining regions (CDRs) contained in a VH set forth in any one of SEQ ID NOs: 27 and 106, or
   (ii) complementarity determining regions (CDRs) VHH CDR1, VHH CDR2, and VHH CDR3, wherein the VHH CDR1 comprises or consists of the amino acid sequence set forth in SEQ ID NO: 24, the VHH CDR2 comprises or consists of the amino acid sequence set forth in SEQ ID NO: 25, and the VHH CDR3 comprises or consists of the amino acid sequence set forth in SEQ ID NO: 26.
23. The multispecific antibody according to Embodiment 22, wherein the heavy chain variable region
   (i) comprises or consists of an amino acid sequence having at least 90%, 91%, 92%, 93%, 94%, 95%, 96%, 97%, 98%, or 99% identity to an amino acid sequence selected from any one of SEQ ID NOs: 27 and 106;
   (ii) comprises or consists of an amino acid sequence selected from any one of SEQ ID NOs: 27 and 106; or
   (iii) comprises an amino acid sequence having one or more (preferably not more than 10, and more preferably not more than 5, 4, 3, 2, or 1) amino acid modifications (preferably amino acid substitutions, and more preferably conservative amino acid substitutions) compared to an amino acid sequence selected from any one of SEQ ID NOs: 27 and 106, wherein preferably, the amino acid modifications do not occur in the CDRs.
24. The multispecific antibody according to Embodiment 20, wherein the third antigen-binding region comprises a VH and a VL, wherein the VH comprises three complementarity determining regions from a heavy chain variable region (HCDRs): HCDR1, HCDR2, and HCDR3, and the VL comprises three complementarity determining regions from a light chain variable region (LCDRs): LCDR1, LCDR2, and LCDR3, wherein
   (i) the HCDR1, the HCDR2, and the HCDR3 are three complementarity determining regions HCDR1, HCDR2, and HCDR3 contained in a VH set forth in SEQ ID NO: 42, respectively; and the LCDR1, the LCDR2, and the LCDR3 are three complementarity determining regions LCDR1, LCDR2, and LCDR3 contained in a VL set forth in SEQ ID NO: 43, respectively; or
   (ii) the HCDR1 comprises or consists of the amino acid sequence set forth in SEQ ID NO: 36, the HCDR2 comprises or consists of the amino acid sequence set forth in SEQ ID NO: 37, the HCDR3 comprises or consists of the amino acid sequence set forth in SEQ ID NO: 38, the LCDR1 comprises or consists of the amino acid sequence set forth in SEQ ID NO: 39, the LCDR2 comprises or consists of the amino acid sequence set forth in SEQ ID NO: 40, and the LCDR3 comprises or consists of the amino acid sequence set forth in SEQ ID NO: 41.
25. The multispecific antibody according to Embodiment 24, wherein the third antigen-binding region comprises a VH and a VL, wherein
   the VH comprises or consists of the amino acid sequence set forth in SEQ ID NO: 42, or an amino acid sequence having at least 90%, 91%, 92%, 93%, 94%, 95%, 96%, 97%, 98%, or 99% identity thereto, and/or
   the VL comprises or consists of the amino acid sequence set forth in SEQ ID NO: 43, or an amino acid sequence having at least 90%, 91%, 92%, 93%, 94%, 95%, 96%, 97%, 98%, or 99% identity thereto.
26. The multispecific antibody according to Embodiment 24 or 25, wherein the third antigen-binding region is a Fab specifically binding to GPRC5D.
27. The multispecific antibody according to Embodiment 26, wherein the Fab comprises a VH and a CH1 of the third antigen-binding region, and optionally the CH1 comprises a hinge region portion (e.g., EPKSS and EPKSC), wherein the CH1 is a CH1 from IgG1, IgG2, IgG3, or IgG4, preferably a CH1 from IgG1.
28. The multispecific antibody according to Embodiment 27, wherein the CH1
   (i) comprises or consists of an amino acid sequence having at least 85%, 90%, 91%, 92%, 93%, 94%, 95%, 96%, 97%, 98%, or 99% identity to the amino acid sequence selected from SEQ ID NO: 88; or
   (ii) comprises or consists of the amino acid sequence selected from SEQ ID NO: 88.
29. The multispecific antibody according to Embodiment 28, wherein the Fab heavy chain of the third antigen-binding region
   (i) comprises or consists of an amino acid sequence having at least 85%, 90%, 91%, 92%, 93%, 94%, 95%, 96%, 97%, 98%, or 99% identity to the amino acid sequence selected from SEQ ID NO: 110;
   (ii) comprises or consists of the amino acid sequence selected from SEQ ID NO: 110; or
   (iii) comprises or consists of an amino acid sequence having one or more (preferably not more than 10 or 10, and more preferably not more than 5, 4, 3, 2, or 1) amino acid modifications (preferably amino acid substitutions, and more preferably conservative amino acid substitutions) compared to the amino acid sequence selected from SEQ ID NO: 110; and/or
   the Fab light chain of the third antigen-binding region
   (i) comprises or consists of an amino acid sequence having at least 85%, 90%, 91%, 92%, 93%, 94%, 95%, 96%, 97%, 98%, or 99% identity to the amino acid sequence selected from SEQ ID NO: 62;
   (ii) comprises or consists of the amino acid sequence selected from SEQ ID NO: 62; or
   (iii) comprises or consists of an amino acid sequence having one or more (preferably not more than 10 or 10, and more preferably not more than 5, 4, 3, 2, or 1) amino acid modifications (preferably amino acid substitutions, and more preferably conservative amino acid substitutions) compared to the amino acid sequence selected from SEQ ID NO: 62.
30. The multispecific antibody according to any one of Embodiments 9-29, wherein the multispecific antibody is an IgG-like bispecific antibody comprising an Fc dimer, wherein two Fc regions constituting the Fc dimer are identical or different.
31. The multispecific antibody according to Embodiment 30, wherein the two Fc regions are different, and preferably, a corresponding knob mutation(s) and a corresponding hole mutation(s) are introduced into the two Fc regions, respectively.
32. The multispecific antibody according to Embodiment 31, wherein
   a) one Fc-region polypeptide comprises a knob mutation T366W, and the other Fc-region polypeptide comprises hole mutations T366S, L368A, and Y407V, or
   b) one Fc-region polypeptide comprises knob mutations T366W and Y349C, and the other Fc-region polypeptide comprises hole mutations T366S, L368A, Y407V, and S354C, or
   c) one Fc-region polypeptide comprises knob mutations T366W and S354C, and the other Fc-region polypeptide comprises hole mutations T366S, L368A, Y407V, and Y349C; and
   optionally, the Fc region further comprises a mutation(s) that reduces binding to an Fcγ receptor, e.g., one or more of an L234A/L235A mutation, a D265A mutation, and a P329A mutation, for example, an L234A/L235A mutation, a D265A mutation, and a P329A mutation.
33. The bispecific antibody according to any one of Embodiments 30-32, wherein one or both of the Fc regions comprise a hinge region, e.g., EPKSS (SEQ ID NO: 94) or EPKSC (SEQ ID NO: 96).
34. The multispecific antibody according to Embodiment 32, wherein
   (i) the Fc region comprising a knob mutation(s)
      a) comprises or consists of an amino acid sequence set forth in SEQ ID NO: 47, 86, or 87; or
      b) comprises or consists of an amino acid sequence having at least 90% identity, e.g., 95%, 96%, 97%, 99% or higher identity, to SEQ ID NO: 47, 86, or 87 and comprising a knob mutation(s) (e.g., S354C and T366W); and/or
   (ii) the Fc region comprising a hole mutation(s)
      a) comprises or consists of an amino acid sequence set forth in SEQ ID NO: 84, 85, or 111; or
      b) comprises or consists of an amino acid sequence having at least 90% identity, e.g., 95%, 96%, 97%, 99% or higher identity, to SEQ ID NO: 84, 85, or 111 and comprising a hole mutation(s) (e.g., Y349C, T366S, L368A, and Y407V).
35. The multispecific antibody according to any one of Embodiments 9-34, being a bispecific antibody and comprising a first antigen-binding region, a second antigen-binding region, and an Fc dimer, wherein the first antigen-binding region is an scFv fragment specifically binding to CD3, and the second antigen-binding region is a VHH specifically binding to BCMA, e.g., the VHH according to any one of Embodiments 1-3 and 8.
36. The multispecific antibody according to Embodiment 35, comprising one or two VHHs specifically binding to BCMA, one anti-CD3 scFv, and an Fc heterodimer, wherein
   the scFv comprises a VH-VL (optionally the VH and VL are fused via a linker), and the VHH comprises or consists of a heavy chain variable region,
   wherein the C-terminus of the VL of the scFv is fused to a CH2 or a hinge region of a first Fc region (e.g., comprising a knob mutation(s) or comprising a hole mutation(s)) to form a first heavy chain; and
   the C-terminus of the one anti-BCMA VHH or of the two anti-BCMA VHHs in tandem is fused to a second Fc region (e.g., comprising a hole mutation(s) or comprising a knob mutation(s)) to form a second heavy chain (e.g., the C-terminus of the VHH is fused to a CH2 or a hinge region of the second Fc region).
37. The multispecific antibody according to Embodiment 36, wherein
   the first heavy chain comprises or consists of the amino acid sequence set forth in SEQ ID NO: 57, or an amino acid sequence having at least 85%, 90%, 91%, 92%, 93%, 94%, 95%, 96%, 97%, 98%, or 99% identity thereto; and/or
   the second heavy chain comprises or consists of an amino acid sequence set forth in any one of SEQ ID NOs: 56, 102, 103, and 104, or an amino acid sequence having at least 85%, 90%, 91%, 92%, 93%, 94%, 95%, 96%, 97%, 98%, or 99% identity thereto.
38. The multispecific antibody according to any one of Embodiments 9-34, being a trispecific antibody and comprising a first antigen-binding region, a second antigen-binding region, a third antigen-binding region, and an Fc dimer, wherein the first antigen-binding region is an scFv fragment or a Fab fragment specifically binding to CD3, the second antigen-binding region is a VHH specifically binding to BCMA, e.g., the VHH according to any one of Embodiments 1-3 and 8, and the third antigen-binding region is a VHH or a Fab fragment specifically binding to GPRC5D.
39. The multispecific antibody according to Embodiment 38, comprising one anti-GPRC5D VHH, one anti-BCMA VHH, one anti-CD3 Fab fragment, and an Fc dimer, wherein
   the Fab fragment comprises a VH-CH1 and a VL-CL, the VHH comprises a heavy chain variable region, and the trispecific antibody comprises or consists of a first heavy chain, a second heavy chain, and a light chain, wherein
   the first heavy chain comprises, from N-terminus to C-terminus: the anti-GPRC5D VHH, the anti-BCMA VHH, and a first Fc region, which are fused with or without a linker(s); or the anti-BCMA VHH, the anti-GPRC5D VHH, and a first Fc region, which are fused with or without a linker(s);
   the second heavy chain comprises, from N-terminus to C-terminus: the heavy chain of the anti-CD3 Fab fragment and a second Fc region, which are fused with or without a linker; and
   the light chain comprises: the light chain of the anti-CD3 Fab fragment.
40. The multispecific antibody according to Embodiment 39, wherein
   the first heavy chain comprises or consists of the amino acid sequence set forth in SEQ ID NO: 58, or an amino acid sequence having at least 85%, 90%, 91%, 92%, 93%, 94%, 95%, 96%, 97%, 98%, or 99% identity thereto;
   the second heavy chain comprises or consists of the amino acid sequence set forth in SEQ ID NO: 59, or an amino acid sequence having at least 85%, 90%, 91%, 92%, 93%, 94%, 95%, 96%, 97%, 98% or 99% identity thereto; and/or
   the light chain comprises or consists of the amino acid sequence set forth in SEQ ID NO: 60, or an amino acid sequence having at least 85%, 90%, 91%, 92%, 93%, 94%, 95%, 96%, 97%, 98%, or 99% identity thereto.
41. The multispecific antibody according to Embodiment 38, comprising one anti-GPRC5D Fab fragment, one anti-BCMA VHH, one anti-CD3 scFv, and an Fc dimer, wherein
   the Fab fragment comprises a VH-CH1 and a VL-CL, the VHH comprises a heavy chain variable region, the scFv comprises a VH-VL, and the trispecific antibody comprises or consists of a first heavy chain, a second heavy chain, and a light chain, wherein
   the first heavy chain comprises, from N-terminus to C-terminus: the anti-BCMA VHH and a first Fc region, which are fused with or without a linker;
   the second heavy chain comprises, from N-terminus to C-terminus: the heavy chain of the anti-GPRC5D Fab fragment, the anti-CD3 scFv, and a second Fc region, which are fused with or without a linker(s); and
   the light chain comprises: the light chain of the anti-GPRC5D Fab fragment.
42. The multispecific antibody according to Embodiment 41, wherein
   the first heavy chain comprises or consists of the amino acid sequence set forth in SEQ ID NO: 56, or an amino acid sequence having at least 85%, 90%, 91%, 92%, 93%, 94%, 95%, 96%, 97%, 98%, or 99% identity thereto;
   the second heavy chain comprises or consists of an amino acid sequence set forth in SEQ ID NO: 61, or an amino acid sequence having at least 85%, 90%, 91%, 92%, 93%, 94%, 95%, 96%, 97%, 98%, or 99% identity thereto; and/or
   the light chain comprises or consists of the amino acid sequence set forth in SEQ ID NO: 62, or an amino acid sequence having at least 85%, 90%, 91%, 92%, 93%, 94%, 95%, 96%, 97%, 98%, or 99% identity thereto.
43. A nucleic acid molecule, encoding the VHH antibody according to any one of Embodiments 1-3 and 8, the heavy-chain antibody according to any one of Embodiments 4-8 or any chain of the multispecific antibody according to any one of Embodiments 9-42, or consisting of a nucleic acid sequence.
44. An expression vector, comprising the nucleic acid molecule according to Embodiment 43.
45. A host cell, comprising the nucleic acid molecule according to Embodiment 43 or the expression vector according to Embodiment 44, wherein preferably, the host cell is prokaryotic or eukaryotic, e.g., a 293 cell or a CHO cell, such as a 293F cell or a 293T cell or a CHO-S cell.
46. A method for preparing the VHH antibody according to any one of Embodiments 1-3 and 8, or the heavy-chain antibody according to any one of Embodiments 4-8, or the multispecific antibody according to any one of Embodiments 9-42, the method comprising culturing a host cell comprising the nucleic acid molecule according to Embodiment 43 or the expression vector according to Embodiment 44 under a condition suitable for expressing the chains of the antibody, and optionally recovering the antibody from the host cell (or a host cell culture medium).
47. An immunoconjugate, comprising the VHH antibody according to any one of Embodiments 1-3 and 8, or the heavy-chain antibody according to any one of Embodiments 4-8, or the multispecific antibody according to any one of Embodiments 9-42.
48. A pharmaceutical composition or a medicament or a formulation, comprising the VHH antibody according to any one of Embodiments 1-3 and 8, or the heavy-chain antibody according to any one of Embodiments 4-8, or the multispecific antibody according to any one of Embodiments 9-42, or the immunoconjugate according to Embodiment 47, and optionally a pharmaceutical supplementary material.
49. A pharmaceutical combination product, comprising the VHH antibody according to any one of Embodiments 1-3 and 8, or the heavy-chain antibody according to any one of Embodiments 4-8, or the multispecific antibody according to any one of Embodiments 9-42, or the immunoconjugate according to Embodiment 47, and one or more additional therapeutic agents (e.g., a chemotherapeutic agent, a cytokine, a cytotoxic agent, an additional antibody, a small molecule drug, or an immunomodulatory agent).
50. A method for preventing or treating a cancer in a subject, comprising administering to the subject an effective amount of the VHH antibody according to any one of Embodiments 1-3 and 8, or the heavy-chain antibody according to any one of Embodiments 4-8, or the bispecific antibody according to any one of Embodiments 9-42, or the immunoconjugate according to Embodiment 47, or the pharmaceutical composition or the medicament or the formulation according to Embodiment 48, or the pharmaceutical combination product according to Embodiment 49.
51. The method according to Embodiment 50, wherein tumor cells of the cancer have an elevated protein level and/or nucleic acid level (e.g., elevated expression) of BCMA.
52. The method according to Embodiment 50 or 51, wherein the cancer is a solid tumor or a hematological tumor, such as myeloma, for example, multiple myeloma (e.g., metastatic multiple myeloma), colon cancer, rectal cancer, or colorectal cancer.
53. The method according to any one of Embodiments 50-52, further comprising administering in combination with an additional therapy such as a therapeutic modality (e.g., surgical therapy or radiotherapy) and/or an additional therapeutic agent (e.g., a chemotherapeutic agent, a cytokine, a cytotoxic agent, an additional antibody, a small molecule drug, or an immunomodulatory agent).
54. A method for detecting the presence of BCMA in a biological sample, comprising
   (i) contacting the biological sample with the VHH antibody according to any one of Embodiments 1-3 and 8, or the heavy-chain antibody according to any one of Embodiments 4-8, or the multispecific antibody according to any one of Embodiments 9-42 under a condition which allows the antibody to bind to BCMA, and
   (ii) detecting whether a complex is formed by the antibody or the bispecific antibody and the BCMA, wherein the formation of the complex indicates the presence of BCMA.

### Examples

### Example 1: Production of BCMA antibodies through alpaca immunization

### 1.1. Alpaca immune library construction and screening

### 1.1.1. Animal immunization

Human BCMA recombinant protein (ACRO, Cat#: BCA-H522y) and cynomolgus monkey BCMA-human IgG₁ Fc recombinant protein (ACRO, Cat#: BCA-C5253) were mixed at a volume ratio of 1:1 (equal concentrations, 0.5 mg each) and used to immunize two alpacas (Chengdu NBbiolab, Co. Ltd.). The alpacas received four rounds of immunizations at 21-day intervals (Table 1). On day 10 after the third immunization and day 10 after the fourth immunization, venous blood was collected from the alpacas. ELISA was performed using human and cynomolgus monkey BCMA recombinant proteins to evaluate the immune serum of the alpacas. One alpaca was selected for blood collection and library construction.

**Table 1. Alpaca immunization procedure**

| Procedure | Route of administration | Dose |
|---|---|---|
| 1^{st} Immunization | Subcutaneous injection | 0.5 mg protein/alpaca |
| 2^{nd} Immunization | Subcutaneous injection | 0.25 mg protein/alpaca |
| 3^{rd} Immunization | Subcutaneous injection | 0.25 mg protein/alpaca |
| 4^{th} Immunization | Subcutaneous injection | 0.25 mg protein/alpaca |

### 1.1.2. Alpaca immune library construction and screening

Total RNA was extracted from the peripheral blood of the alpaca using the Trizol method, and reverse transcription was performed using a PrimeScript II 1st Strand cDNA Synthesis Kit (Takara, Cat#: 6210A) to obtain cDNA. The cDNA was subjected to nested PCR amplification, and 750 bp products containing nanobodies were recovered. A second round of PCR was performed to amplify and purify the VHHs. The VHHs were ligated into a phage vector using enzyme digestion and ligation techniques, followed by electroporation, transformation, and plating. On the next day, the library capacity was determined, and the library capacity of the constructed library was found to be 4.49 × 10e9 CFU, with an insertion rate of 100%. Two rounds of solid-phase screening were performed using human BCMA recombinant protein, and the positive clones obtained were sequenced and identified. The partial amino acid sequences obtained are shown in Table 2.

**Table 2. Amino acid sequence of camelid-derived VHH of anti-BCMA antibody**

| Antibody name | Variable region sequence | HCDR1 | HCDR2 | HCDR3 |
|---|---|---|---|---|
| BH56 | SEQ ID NO: 4 | PSAMS (SEQ ID NO:1) | GIYGDGRTTYADSVKG (SEQ ID NO:2) | GIRLLSESWAQAL (SEQ ID NO:3) |
| HA68 | SEQ ID NO: 8 | PSAMN (SEQ ID NO:5) | GIYGDGKAEYADSVKG (SEQ ID NO:6) | GIRPTDAPWAASL (SEQ ID NO:7) |

### Example 2: Characterization of anti-human BCMA chimeric antibodies

### 2.1. Synthesis and expression of anti-human BCMA chimeric antibodies

The amino acid sequences of the camelid-derived antibody VHHs in Table 2 were fused with the hinge region (*EPKSS* (SEQ ID NO: 94)) and then inserted into an expression vector pcDNA3.1(+) containing a sequence encoding the human Fc constant region (SEQ ID NO: 90) to obtain plasmids encoding the respective chimeric antibodies.

The specific chimeric antibody sequences obtained were as follows:

| Antibody name | Heavy chain sequence | Variable region sequence | HCDR1 | HCDR2 | HCDR3 |
|---|---|---|---|---|---|
| BH56-IgG | SEQ ID NO: 44 | SEQ ID NO:4 | SEQ ID NO:1 | SEQ ID NO:2 | SEQ ID NO:3 |
| HA68-IgG | SEQ ID NO: 45 | SEQ ID NO:8 | SEQ ID NO:5 | SEQ ID NO:6 | SEQ ID NO:7 |

The plasmids encoding the respective anti-BCMA antibodies were transfected into ExpiCHO-S cells for the expression of camelid-derived chimeric antibodies. The 269B094 (a fully human monoclonal antibody targeting BCMA) (heavy chain amino acid sequence: SEQ ID NO: 76), derived from CN2017/096938, was used as the control antibody. Similarly, the control antibody was expressed. The antibodies were purified accordingly.

Specifically, the plasmids encoding the anti-human BCMA chimeric antibodies were transfected into ExpiCHO-S cells using an ExpiCHO^{™} expression system (ThermoFisher, Cat#: A29133) to similarly express the control antibody 269B094 monoclonal antibody, according to the manufacturer's product instructions. The cells were cultured for 10-12 days after transfection. When the cell viability decreased to 60%-70%, the supernatant was collected, and the antibodies expressed and secreted in the supernatant were purified using a MabSelect Sure protein A affinity chromatography system (GE healthcare). The purified antibodies were concentrated, and subjected to sterile filtration. The purity of the antibody protein was detected by SDS-PAGE and size exclusion chromatography. The results indicated that the purities of the antibodies were greater than 90%, and the antibodies could be used in the next step.

### 2.2. Binding of anti-human BCMA chimeric antibodies to engineered cells expressing human BCMA protein

A sequence encoding human BCMA (Accession#: Q02223-1) was cloned into a PiggyBac Dual promoter (SBI, Cat#: PB513-B1) expression vector, and transfected into CHO-K1 cells (ATCC, Cat#: CCL-61^{™}) by electroporation. The transfected cells were then screened using 4 µg/mL puromycin (Gibco, Cat#: A1113802) to obtain CHO-K1 cells highly expressing human BCMA (hereinafter referred to as CHO-K1-huBCMA).

Whether the anti-BCMA humanized antibodies in the present disclosure could bind to the human BCMA protein stably expressed on the cell surface was determined by cell binding experiments.

The CHOK1-huBCMA cell line was subjected to enzymatic digestion to obtain a single-cell suspension of CHOK1-huBCMA cells. The single-cell suspension was centrifuged at room temperature at 400× g, and the culture medium was then discarded. The cell pellet was washed once with PBS and then resuspended in the serially diluted anti-human BCMA chimeric antibodies and the control antibody 269B094 (at an initial concentration of 20 µg/mL, and serially 4-fold diluted to obtain 8 concentration points in total), and the cells were incubated at 4 °C for 30 min. The cells were washed once with PBS, and then fluorescent secondary antibody R-PE-conjugated AffiniPure Goat Anti-Human IgG, Fcγ Fragment Specific (Jackson ImmunoResearch, Cat#: 109-116-098) diluted in a 1:200 ratio was added. The cells were incubated in the dark at 4 °C for 30 min. The cells were washed twice with PBS and then resuspended, and finally, two-channel fluorescence signals were detected using a Beckman Cytoflex flow cytometer.

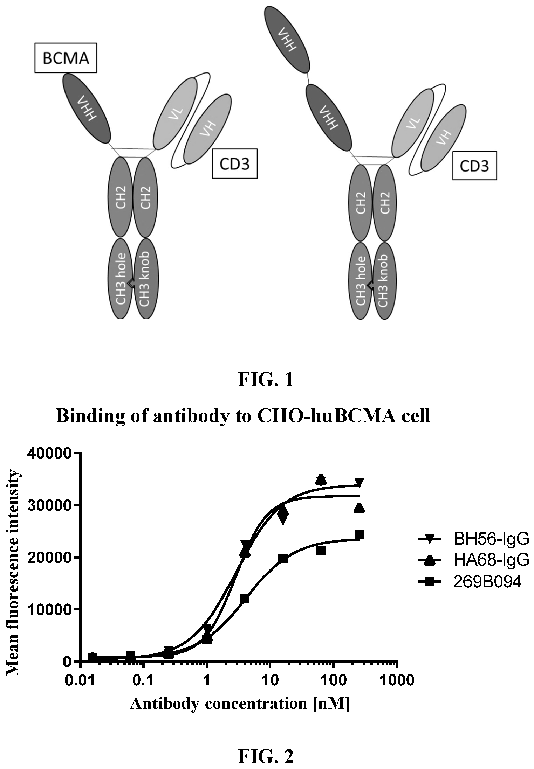

As shown in the results in FIG. 2, all the test samples exhibited better binding activity to the engineered CHOK1-huBCMA cells expressing human BCMA protein than the control sample.

### Example 3: Humanization of alpaca-derived anti-human BCMA antibodies and characterization thereof

### 3.1. Humanization of alpaca-derived anti-human BCMA antibodies

The antibodies obtained in Example 2.1 were humanized. Specifically, the sequences of antibodies BH56 and HA68 were searched and aligned in the IMGT database, thereby obtaining a human germline gene sequence IGHV3-53*03 with high homology to the variable regions of the antibody BH56, and a human germline gene sequence IGHV3-53*02 with high homology to the variable regions of the antibody HA68, respectively, for use as humanized frameworks of the heavy chain variable region. The CDRs of the heavy chain variable regions of the antibodies BH56 and HA68 were grafted into the corresponding humanized frameworks to form humanized antibodies. In order to maintain the affinity of the humanized antibodies for human BCMA, the humanized antibody framework regions obtained were subjected to back mutation.

Therefore, the sequences of the humanized heavy chain variable regions were obtained. The amino acid sequences of the humanized variable regions are listed in Table 3. The variable region amino acid sequences were sent to Biointron Biological Inc. for codon optimization and gene synthesis.

**Table 3. Heavy chain and variable region sequence of humanized antibody**

| Heavy chain name and sequence | Heavy chain variable region | HCDR1 | HCDR2 | HCDR3 |
|---|---|---|---|---|
| huBH56-1-IgG (SEQ ID NO:46) | SEQ ID NO:10 | SEQ ID NO:1 | SEQ ID NO:9 | SEQ ID NO:3 |
| huBH56-3 (SEQ ID NO: 48) | SEQ ID NO:12 | SEQ ID NO:1 | SEQ ID NO:2 | SEQ ID NO:3 |
| huBH56-4-IgG (SEQ ID NO: 49) | SEQ ID NO:14 | SEQ ID NO:1 | SEQ ID NO:13 | SEQ ID NO:3 |
| huBH56-5-IgG (SEQ ID NO:50) | SEQ ID NO:15 | SEQ ID NO:1 | SEQ ID NO:2 | SEQ ID NO:3 |
| huBH56-6-IgG (SEQ ID NO:51) | SEQ ID NO:16 | SEQ ID NO:1 | SEQ ID NO:13 | SEQ ID NO:3 |
| huHA68-1-IgG (SEQ ID NO:52) | SEQ ID NO:18 | SEQ ID NO:1 | SEQ ID NO:17 | SEQ ID NO:7 |
| huHA68-2-IgG (SEQ ID NO:53) | SEQ ID NO:21 | SEQ ID NO:1 | SEQ ID NO:17 | SEQ ID NO:7 |
| huHA68-3-IgG (SEQ ID NO:54) | SEQ ID NO:22 | SEQ ID NO:1 | SEQ ID NO:6 | SEQ ID NO:7 |
| huHA68-4-IgG (SEQ ID NO:55) | SEQ ID NO:23 | SEQ ID NO:1 | SEQ ID NO:6 | SEQ ID NO:7 |

### 3.2. Expression and purification of anti-human BCMA humanized antibodies

Similarly as described in Example 2.1, the plasmids encoding the full length of anti-human BCMA humanized antibodies were transfected into ExpiCHO-S cells to express the respective anti-human BCMA humanized antibodies, and the corresponding purification was performed as described in Example 2.1. The purified anti-human BCMA humanized antibodies were concentrated, and subjected to sterile filtration. The purities of the anti-human BCMA humanized antibodies were detected by SDS-PAGE and size exclusion chromatography (SEC).

### 3.3. Physicochemical analysis of anti-human BCMA humanized antibodies

The purities of the anti-human BCMA humanized antibodies obtained were determined by size exclusion chromatography. Specifically, 20 µg of the humanized anti-human BCMA antibodies in Table 3 were injected onto a TSK G3000SWXL column using 100 mM sodium phosphate + 100 mM Na2SO4 (pH 7.0) as a running buffer for 30 min. The collected effluent was measured using Agilent 1220 HPLC and the data were analyzed using OpenLAB software. According to the results, humanized antibodies with a purity of greater than 90% were selected and used in the next step.

### 3.4. Physicochemical analysis of anti-human BCMA humanized antibodies

In this study, the binding affinity of the above humanized antibodies to human BCMA-His protein (ACRO, Cat#: BCA-H522y) was detected using ForteBio Octet RED96e according to the manufacturer's instructions. Briefly, an AHC sensor (ForteBio, Cat#: 18-5060) was pre-equilibrated in a running buffer (1× PBS, XiGene, Cat#: XG3650, containing 0.02% Tween20, 0.1% BSA, pH 7.0) at room temperature for 10 min. In a 96-well plate, the BCMA kinetic assay was performed according to the follows steps: a) equilibrating the baseline with the running buffer for 180 s; b) adding the antibodies diluted with the running buffer at a final concentration of 5 µg/mL, and immobilizing for 200 s; c) equilibrating the baseline with the running buffer for 180 s; d) adding 100 nM human BCMA protein diluted with the running buffer at the following concentrations to each well. binding for 200 s, and dissociating for 180 s; and e) applying a regeneration solution (0.01 M Gly-HCl, pH 1.5) for 30 s. The experimental data were fitted and calculated using a Fortebio Data Analysis software 1:1 binding model. The results are shown in the table below.

**Table 4. Binding affinity of anti-human BCMA humanized antibody to human BCMA protein**

| Antibody | Antigen | KD (M) | Kon (1/Ms) | Kdis (1/s) |
|---|---|---|---|---|
| BH56-IgG | Human BCMA protein | <1.0E-12 | 1E+06 | <1.0E-07 |
| huBH56-3-IgG | | <1.0E-12 | 1E+06 | <1.0E-07 |
| huBH56-4-IgG | | <1.0E-12 | 1E+06 | <1.0E-07 |
| huBH56-5-IgG | | <1.0E-12 | 1E+06 | <1.0E-07 |
| huBH56-6-IgG | | <1.0E-12 | 978600 | <1.0E-07 |
| HA68-IgG | | <1.0E-12 | 1E+06 | <1.0E-07 |
| huHA68-3-IgG | | <1.0E-12 | 1E+06 | <1.0E-07 |
| huHA68-4-IgG | | <1.0E-12 | 1E+06 | <1.0E-07 |
| 269B094 | | 1.51E-09 | 1E+06 | 0.001552 |

The results in Table 4 indicated that compared to the chimeric antibodies, the humanized antibodies bound to human BCMA protein with a high affinity.

### 3.5. Binding of anti-human BCMA humanized antibodies to tumor cell lines

Whether the anti-BCMA humanized specific antibodies in the present disclosure could bind to the human BCMA protein expressed on the tumor cell surface was determined by cell binding experiments. The target cells in the detection system were NCI-H929 (Nanjing Cobioer Biosciences Co., Ltd., CBP60243) tumor cells endogenously expressing human BCMA.

The NCI-H929 cells were collected and centrifuged at 400× g at room temperature, and the culture medium was then discarded. The cell pellet was washed once with PBS and then resuspended in the serially diluted anti-BCMA humanized antibodies and the control antibody 269B094 (at an initial concentration of 20 µg/mL, and serially 4-fold diluted to obtain 8 concentration points in total), and the cells were incubated at 4 °C for 30 min. The cells were washed once with PBS, and then fluorescent secondary antibody R-PE-conjugated AffiniPure Goat Anti-Human IgG, Fcγ Fragment Specific (Jackson ImmunoResearch, Cat#: 109-116-098) diluted in a 1:200 ratio was added. The cells were incubated in the dark at 4 °C for 30 min. The cells were washed twice with PBS and then resuspended, and finally, two-channel fluorescence signals were detected using a Beckman Cytoflex flow cytometer.

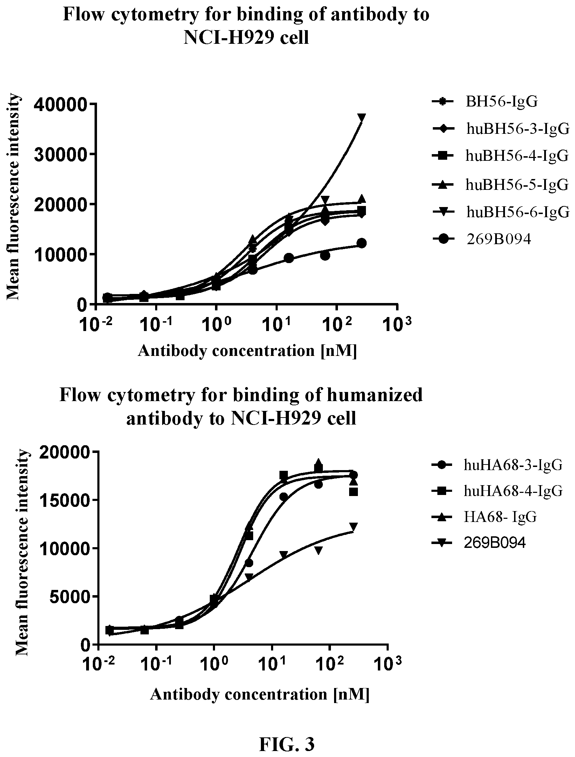

As shown in the results in FIG. 3, the binding activity of huHA68-3 to NCI-H929 cells was lower than that of HA68-IgG, and the activity of all the humanized antibodies was superior to that of the control antibody.

### Example 4: Preparation of anti-human BCMA×CD3 bispecific antibodies and characterization thereof

### 4.1. Construction of bispecific antibodies

In the present disclosure, two different bispecific antibodies with asymmetric structures were constructed as shown in FIG. 1. The anti-human BCMA moiety of each bispecific antibody was derived from the humanized antibodies huBH56-3 and huHA68-4 described above, and the anti-human CD3 moiety was derived from a humanized CD3 antibody sp34 (PCT/CN2023/071314). The constant regions of the bispecific antibodies each comprised a knob-in-hole structure (KIH) (Merchant, A. M., et al. (1998). "An efficient route to human bispecific IgG." Nat Biotechnol 16(7): 677-681.). Such bispecific antibodies are also referred to herein as "anti-CD3/BCMA bispecific antibodies" or "anti-CD3/anti-BCMA bispecific antibodies", sometimes simply referred to as "bispecific antibody molecules" or "diabodies". In the anti-CD3/BCMA bispecific antibodies, the anti-BCMA moiety targeted cells expressing BCMA, and the anti-CD3 moiety activated T cells. The diabodies simultaneously bound to BCMA on tumor cells and CD3 on T cells to facilitate targeted killing of the tumor cells by the activated T cells.

In the present application, two types of anti-CD3/BCMA bispecific antibodies with the structures shown in FIG. 1 were obtained using standard construction methods:
huBH56-3-hole: The variable region sequence of the anti-BCMA humanized antibody huBH56-3 was cloned into a pcDNA3.1(+) vector containing Fc (hole) for expression to obtain a huBH56-3-hole molecule.

2× huBH56-3-hole: The sequence encoding the huBH56-3 variable region was amplified using a PCR technique, and subsequently the two sequences encoding the huBH56-3 variable region were sequentially ligated in tandem using an overlapping PCR technique (with a linker peptide GGGGS (SEQ ID NO: 99) between the two encoding fragments). The ligated sequence was then cloned into a pcDNA3.1 (+) vector containing Fc (hole) for expression to obtain a 2× huBH56-3-hole molecule. huHA68-4-hole: The variable region sequence of the anti-BCMA humanized antibody huHA68-4 was cloned into a pcDNA3.1(+) vector containing Fc (hole) for expression to obtain a huHA68-4-hole molecule.

2× huHA68-4-hole: The sequence encoding the huHA68-4 variable region was amplified using a PCR technique, and subsequently the two sequences encoding the huHA68-4 variable region were sequentially ligated in tandem using an overlapping PCR technique (with a linker GGGGS between the two encoding fragments). The ligated sequence was then cloned into a pcDNA3.1 (+) vector containing Fc (hole) for expression to obtain a 2× huHA68-4-hole molecule.

CD3 ScFv-knob: The nucleotide sequence encoding the anti-CD3 single-chain antibody 23L2 was cloned into a pcDNA3.1 (+) vector with Fc (knob) for expression to obtain a CD3 ScFv-knob molecule.

Teclistamab, a fully human bispecific antibody targeting BCMA/CD3 (heavy chain amino acid sequence: SEQ ID NO: 63 or SEQ ID NO: 65; light chain amino acid sequence: SEQ ID NO: 64 or SEQ ID NO: 66, with the sequences derived from US2017/0051068A1), was used as the control antibody. Similarly, the control antibody was expressed. The antibodies were purified accordingly.

The bispecific antibodies in FIG. 1 were obtained by accordingly combining the expression vectors described above according to the specific composition shown in Table 5 and expressing them under appropriate conditions.

The construction, expression, purification, and preliminary analysis steps of the bispecific antibodies were the same as those in Example 2.1.

**Table 5. Humanized bispecific antibody**

| Clone | Chain | Structure | Amino acid sequence |
|---|---|---|---|
| huBH56-3-CD3ScFv | HC1: huBH56-3-hole SEQ ID NO: 56 | huBH56-3 VH | SEQ ID NO:12 |
| | | Linker peptide (hinge region portion) | SEQ ID NO:94 |
| | | Fc(hole) (CH2-CH3 hole) | SEQ ID NO:84 |
| | HC2:CD3ScFv-knob SEQ ID NO:57 | CD3-VH | SEQ ID NO: 34 |
| | | Linker peptide | SEQ ID NO:100 |
| | | CD3-VL | SEQ ID NO:35 |
| | | Linker peptide (hinge region portion) | SEQ ID NO: 94 |
| | | Fc(knob) (CH2-CH3 knob) | SEQ ID NO:87 |
| 2xhuBH56-3-CD3ScFv | HC1: 2xhuBH56-3-hole SEQ ID NO:104 | huBH56-3 VH | SEQ ID NO:12 |
| | | Linker peptide | SEQ ID NO:99 |
| | | huBH56-3 | SEQ ID NO:12 |
| | | Linker peptide (hinge region portion) | SEQ ID NO:94 |
| | | Fc(hole) (CH2-CH3 hole) | SEQ ID NO:84 |
| | HC2:CD3ScFv-knob SEQ ID NO: 57 | CD3-VH | SEQ ID NO:34 |
| | | Linker peptide | SEQ ID NO:100 |
| | | CD3-VL | SEQ ID NO:35 |
| | | Linker peptide | SEQ ID NO:94 |
| | | Fc(knob) (CH2-CH3 knob) | SEQ ID NO:87 |
| huHA68-4-CD3ScFv | HC1: huHA68-4-hole SEQ ID NO: 102 | huHA68-4 | SEQ ID NO: 23 |
| | | Linker peptide | SEQ ID NO:94 |
| | | Fc(hole) (CH2-CH3 hole) | SEQ ID NO:84 |
| | HC2:CD3ScFv-knob SEQ ID NO:57 | CD3-VH | SEQ ID NO:34 |
| | | Linker peptide | SEQ ID NO:100 |
| | | CD3-VL | SEQ ID NO:35 |
| | | Linker peptide | SEQ ID NO:94 |
| | | Fc(knob) (CH2-CH3 knob) | SEQ ID NO:87 |
| 2xhuHA68-4-CD3ScFv | HC1:2xhuHA68-4-hole SEQ ID NO: 103 | huHA68-4 | SEQ ID NO:23 |
| | | Linker peptide | SEQ ID NO:99 |
| | | huHA68-4 | SEQ ID NO:23 |
| | | Linker peptide | SEQ ID NO:94 |
| | | Fc(hole) (CH2-CH3 hole) | SEQ ID NO:84 |
| | HC2:CD3ScFv-knob SEQ ID NO:57 | CD3-VH | SEQ ID NO:34 |
| | | Linker peptide | SEQ ID NO:100 |
| | | CD3-VL | SEQ ID NO:35 |
| | | Linker peptide | SEQ ID NO:94 |
| | | Fc(knob) (CH2-CH3 knob) | SEQ ID NO:87 |

The heavy and light chain expression vectors obtained (see Table 5) were co-transfected into ExpiCHO-S cells for expression under appropriate conditions to obtain bispecific antibody proteins, and the expression, purification, and preliminary analysis steps were the same as those in Example 2.1.

### 4.2. Binding of anti-human BCMA×CD3 diabodies to engineered cells expressing human BCMA protein

Whether the anti-BCMA×CD3 humanized diabodies in the present disclosure could bind to the human BCMA protein stably expressed on the cell surface was determined by cell binding experiments.

The CHOK1-huBCMA cell line was subjected to enzymatic digestion to obtain a single-cell suspension of CHOK1-huBCMA cells. The single-cell suspension was centrifuged at room temperature at 400× g, and the culture medium was then discarded. The cell pellet was washed once with PBS and then resuspended in the serially diluted anti-BCMA humanized diabodies and the Teclistamab analog control antibody (at an initial concentration of 20 µg/mL, and serially 4-fold diluted to obtain 8 concentration points in total), and the cells were incubated at 4 °C for 30 min. The cells were washed once with PBS, and then fluorescent secondary antibody R-PE-conjugated AffiniPure Goat Anti-Human IgG, Fcγ Fragment Specific (Jackson ImmunoResearch, Cat#: 109-116-098) diluted in a 1:200 ratio was added. The cells were incubated in the dark at 4 °C for 30 min. The cells were washed twice with PBS and then resuspended, and finally, two-channel fluorescence signals were detected using a Beckman Cytoflex flow cytometer.

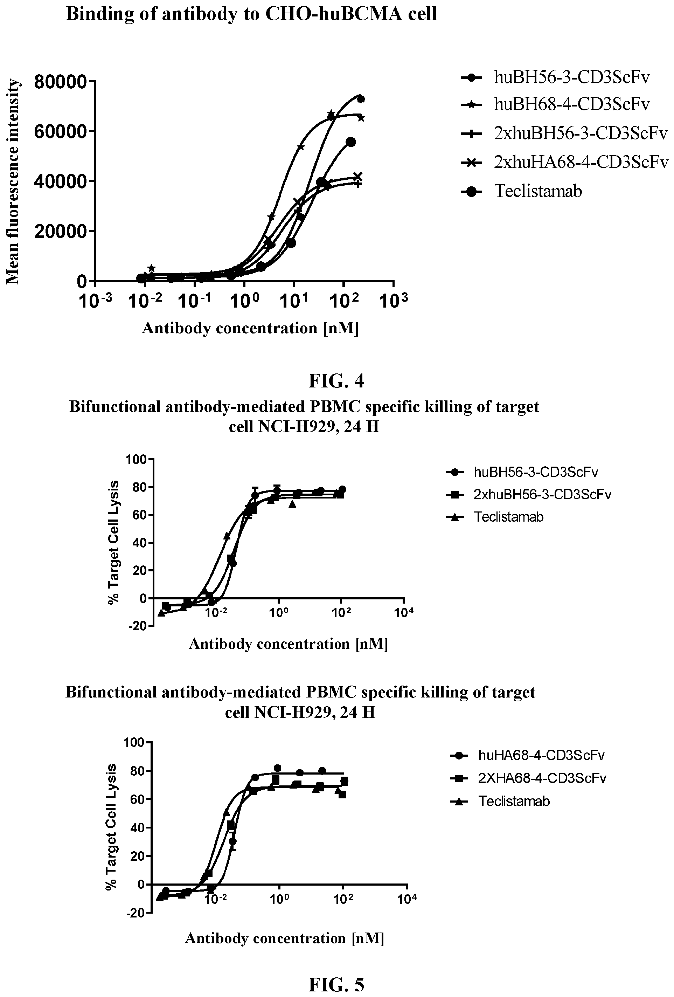

As shown in the results in FIG. 4, all the test samples exhibited better binding activity to the engineered cells than the control sample.

### 4.3. Killing activity of anti-BCMA×CD3 bispecific antibodies targeting tumor cells

To detect the activity of the anti-CD3/BCMA bispecific antibodies in mediating T cell killing of tumor cells, we established a primary T cell-dependent cytotoxicity assay system using RPMI-8226 (Cell Bank of Type Culture Collection Committee of the Chinese Academy of Sciences, TCHu234), NCI-H929 cells (Nanjing Cobioer Biosciences Co., Ltd., CBP60243), and 293T (Cell Bank of Type Culture Collection Committee of the Chinese Academy of Sciences, GNHu17) negative cell lines endogenously expressing human BCMA as the target cells, and human peripheral blood mononuclear cells (PBMCs) (obtained from healthy human blood) as the effector cells.

The PBMCs were thawed, and adjusted to a cell density of 5-10 × 10⁶ cells/mL using a 1640 complete medium (supplemented with 10% FBS). The cells were activated overnight with IL2 (Jiangsu Kingsley Pharmaceutical Co., Ltd.) at a final concentration of 100 IU/mL. The target cells RPMI-8226, NCI-H929, and BCMA-negative 293T cells were collected by centrifugation at 400× g for 5 min, and the supernatant was discarded. The target cells were adjusted to a density of 2 × 10⁵ cells/mL using an MEM-α (Gibco, Cat#: 41061-029) assay buffer containing 1% FBS (Gibco, Cat#: 10099-141) and 100 IU/mL IL2, and seeded into a 96-well plate (Corning, Cat#: 3599) at 50 µL/well (i.e., 10,000 cells/well).

A serially diluted solution of the test antibodies (the maximum assay concentration of the test antibodies was 1 µg/mL, and the test antibodies were serially 5-fold diluted to obtain 9 concentration points in total) was prepared, and 50 µL of the prepared antibody solution was added to each well. In this study, the target cell maximal killing (2% Triton100 lysis solution was added to the target cells), minimal killing (the assay buffer was added to the target cells), and natural killing (the effector cells were added to the target cells) controls were set at 50 µL/well. The ratio of the effector cells E to the target cells T was finally 10:1, and then the cells were incubated in a cell incubator for another 24 h. After the incubation was completed, the experimental plate was taken out and centrifuged at 400× g for 3 min to allow all cells to settle to the bottom of the plate. 50 µL of supernatant was carefully pipetted into a new 96-well plate, 50 µL of LDH detection solution (Roche, Cat#: 11644793001) was added to each well, and the plate was incubated at room temperature. When the color changed, the plate was read on an F50 microplate reader. The detection wavelength was 492 nm, the reference wavelength was 650 nm, and the detection analysis was performed when the OD492 value of the maximum killing well was between 0.4 and 1.0.

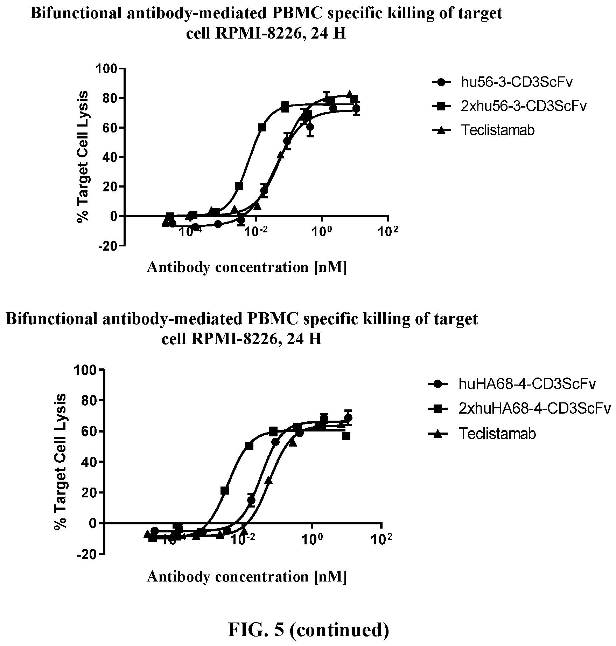

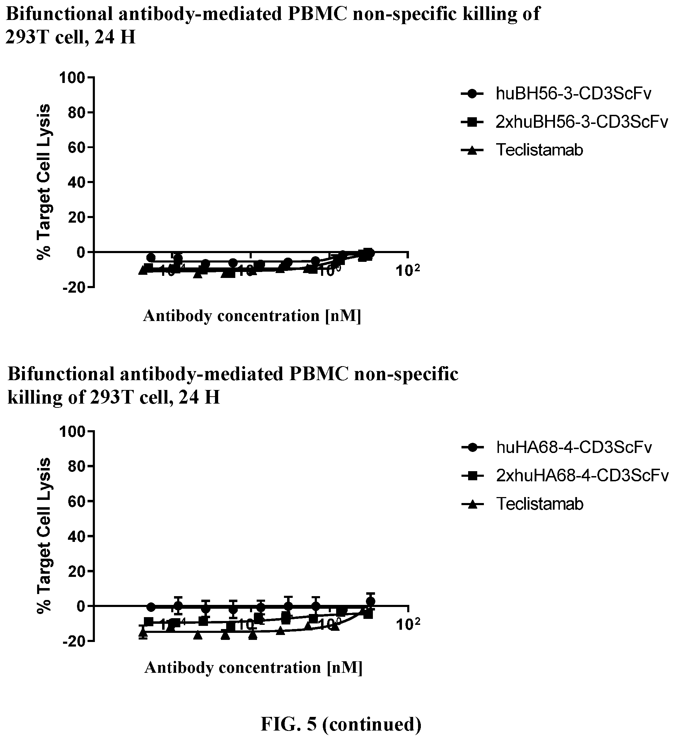

The results, shown in FIG. 5, indicated that the anti-CD3/BCMA bispecific antibodies were capable of specifically inducing T cell killing of RPMI-8226 and NCI-H929 tumor cells, exhibiting higher killing activity compared to the control antibody. However, no killing effect was observed on BCMA-negative 293T cells. Therefore, the bispecific antibody of the present disclosure has no significant visible non-specific killing activity.

### 4.4. Induction of cytokine release after activation of T cells by anti-BCMA×CD3 bispecific antibodies

To detect the induction of cytokine secretion by the **anti-BCMA×CD3** bispecific antibodies while mediating T cell killing of tumor cells, we established a primary T cell-dependent cytotoxicity assay system using NCI-H929 cells endogenously expressing human BCMA and GPRC5D as the target cells, and human peripheral blood mononuclear cells (PBMCs) as the effector cells. The killing and incubation were performed as shown in 4.3. At the endpoint of the killing, the NCI-H929 supernatant was collected for detection of IL-6 and IFN-γ in the system.

After the killing and incubation were completed, the experimental plate was taken out and centrifuged at 500× g for 5 min to allow all cells to settle to the bottom of the plate. 100 µL of the supernatant was carefully pipetted into a new 96-well plate, and the levels of human IFNy (detection kit: Cisbio, Cat#: 62HIFNGPEH), human TNFα (detection kit: R&D, Cat#: DY210), and human IL-6 (detection kit: R&D, Cat#: DY206) in the supernatant were detected, separately.

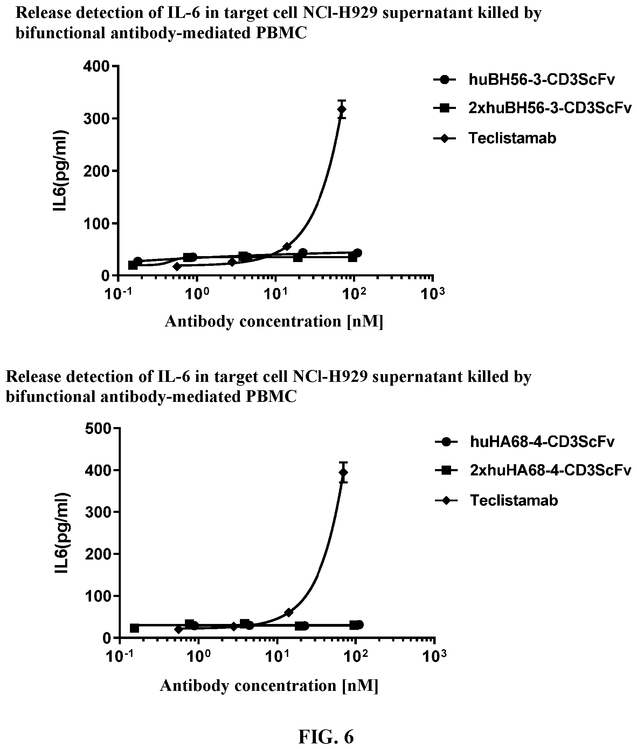

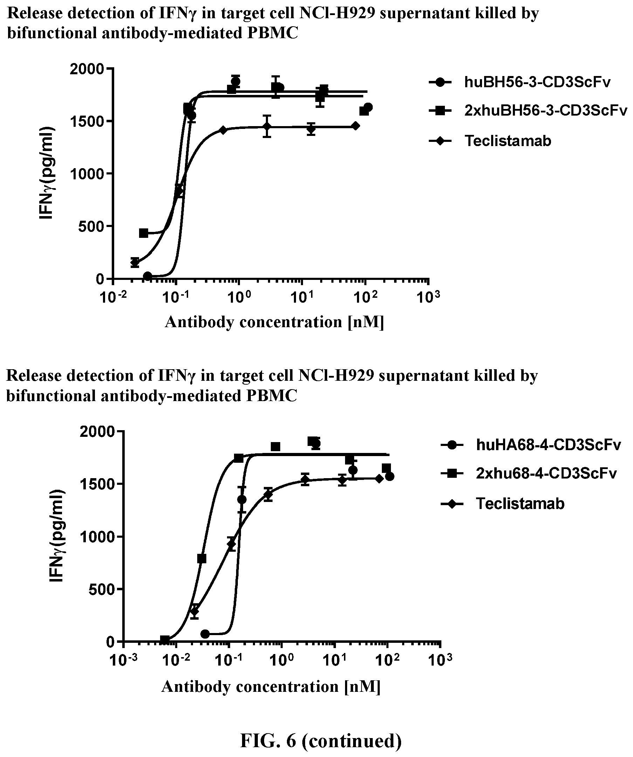

The results, shown in FIG. 6, indicated that the **anti-BCMA×CD3** bispecific antibodies were capable of inducing T cells to release human IFNy (FIG. 6) and human IL-6 (FIG. 6) cytokines in the NCI-H929 system, and the cytokine release trend was consistent with the killing results: the stronger the killing effect, the higher the cytokine release level.

### Example 5: Preparation and validation of GPRC5D VHH antibody

### 5.1. The following camelid-derived GPRC5D VHH antibody VHH8 was obtained through alpaca immunization and phage library display

The gene sequence of the human GPRC5D protein (NP_061124.1(NCBI) or sp|Q9NZD1 (Uniprot)) was subjected to codon optimization and gene synthesis by General Biology System (Anhui) Co., Ltd, and then cloned into a stable expression vector. The gene sequence encoding the human GPRC5D protein (SEQ ID NO: 108) was transfected into CHO-K1 (ATCC, Cat#: CCL-61) and 293T (Cell Bank of Type Culture Collection Committee of the Chinese Academy of Sciences, Cat#: SCSP-502) cells, respectively, using a Lipofectamine^{™} 2000 (Invitrogen, Cat#: 11668019) transfection reagent. The transfected cells were screened using 0.3 µg/mL puromycin (Gibco, Cat#: A1113802) and subjected to monoclonal seeding to obtain CHO-K1 (CHO-K1-huGPRC5D) and 293T engineered monoclonal cells (293T-huGPRC5D) highly expressing human GPRC5D.

| Cell name | Protein | Cell line | Promoter | Resistance |
|---|---|---|---|---|
| CHO-K1-HuGPRC5D | Human GPRC5D | CHO-K1 | CMV | Puromycin |
| 293T-HuGPRC5D | Human GPRC5D | HEK293T | CMV | Puromycin |

**Table 6: Variable region amino acid sequence of camelid-derived anti-GPRC5D VHH antibody**

| Name | Amino acid sequence |
|---|---|
| 8-VHH | SEQ ID NO:106 |
| 8-HCDR1 | SEQ ID NO:24 |
| 8-HCDR2 | SEQ ID NO:25 |
| 8-HCDR3 | SEQ ID NO:26 |

The DNA sequences of the VHH antibody in Table 6 were subjected to codon optimization and gene synthesis by General Biology System (Anhui) Co., Ltd. The VHH gene encoding the antibody was inserted into an expression vector pcDNA3.1(+) containing the gene encoding the heavy chain constant region Fc (SEQ ID NO: 80) of human IgG1 to obtain a plasmid encoding a camelid-derived anti-GPRC5D VHH antibody comprising the heavy chain constant region Fc of human IgG1. The amino acid sequence of the antibody VHH-Fc comprising the human IgG1 heavy chain constant region Fc is set forth in SEQ ID NO: 107.

The plasmid encoding the camelid-derived anti-GPRC5D VHH-Fc antibody (containing the human IgG1 heavy chain constant region Fc) described in Table 6 was transfected into ExpiCHO-S cells for antibody expression, and corresponding purification was performed (see Example 2.1 for details). The purified antibodies were concentrated, and subjected to sterile filtration. The purity of the antibody protein was detected by SDS-PAGE and size exclusion chromatography. The results indicated that the purities of the antibodies were greater than 90% and met the requirements, and the antibodies could be used in the next step.

### 5.2. Binding of camelid-derived anti-GPRC5D VHH antibody to human GPRC5D protein or rhesus GPRC5D protein expressed on surface of expression cell

Whether the camelid-derived anti-GPRC5D VHH antibody in the present disclosure could bind to the human GPRC5D protein or cynomolgus monkey GPRC5D protein stably expressed on the 293T cell surface was determined by cell binding experiments.

After enzymatic digestion, single-cell suspensions of the two cells were obtained. The single-cell suspensions were centrifuged at room temperature at 400× g, and the culture medium was then discarded. The cell pellet was washed once with PBS and centrifuged, and the supernatant was discarded. The cell pellet was then resuspended in the serially diluted camelid-derived anti-GPRC5D VHH-Fc antibody obtained in the present application (at an initial concentration of 25 µg/mL, and serially 4-fold down-diluted to 8 concentration points in total: 6.25 µg/mL, 1.563 µg/mL, 0.391 µg/mL, 0.0977 µg/mL, 0.0244 µg/mL, 0.0061 µg/mL, and 0.00153 µg/mL), and the cells were incubated at 4 °C for 30 min. The cells were washed once with PBS, and then fluorescent secondary antibody R-PE-conjugated AffiniPure Goat Anti-Human IgG, Fcγ Fragment Specific (Jackson ImmunoResearch, Cat#: 109-116-098) diluted in a 1:200 ratio was added. The cells were incubated in the dark at 4 °C for 30 min. The cells were washed twice with PBS and then resuspended, and finally, PE-channel fluorescence signals were detected using a Beckman CytoFlex (Beckman) flow cytometer.

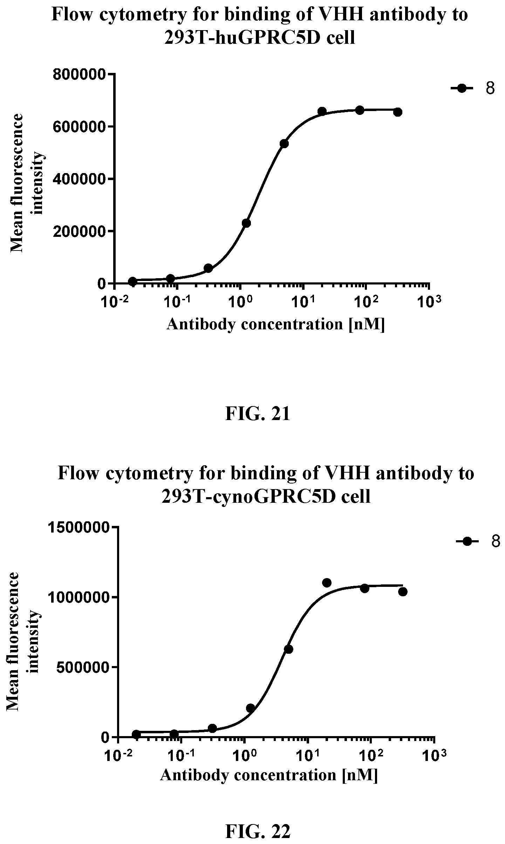

The results, shown in FIGs. 21 and 22, indicated that the camelid-derived anti-GPRC5D VHH-Fc antibody could bind to cells expressing human GPRC5D protein or cynomolgus monkey GPRC5D protein.

### 5.3. Humanization of camelid-derived anti-GPRC5D antibody

The camelid-derived antibody 8 was humanized as described in Example 3.

A humanized VHH antibody 8H5 was thus obtained with its VHH sequence set forth in SEQ ID NO: 27. According to the IMGT numbering scheme, the HCDR1, HCDR2, and HCDR3 were identified as SEQ ID NO: 24, 25, and 26, respectively. The variable region amino acid sequences were sent to General Biology System (Anhui) Co., Ltd. for codon optimization and gene synthesis. The genes encoding the VHH region of the antibody were sequentially inserted into an expression vector pcDNA3.1(+) containing a encoding gene of the human IgG1 heavy chain constant region Fc to obtain a plasmid expressing the full-length heavy chain VHH-Fc of the anti-GPRC5D humanized antibody. The specific procedure is described in 5.1, and the amino acid sequence of the VHH-Fc obtained is set forth in SEQ ID NO: 105.

The plasmid combination encoding the full-length heavy chain VHH-Fc of the GPRC5D humanized antibody was transfected into ExpiCHO-S cells for expression of anti-GPRC5D humanized antibody, and corresponding purification was performed (see Example 2.1 for details). The purified antibody was concentrated, and subjected to sterile filtration. The purity of the protein was detected by SDS-PAGE and size exclusion chromatography (SEC).

### 5. 4. Physicochemical analysis of humanized anti-GPRC5D antibody

The purity of the humanized anti-GPRC5D VHH-Fc antibody obtained was determined by size exclusion chromatography. Specifically, 20 µg of sample was injected onto a TSK G3000SWXL column using 100 mM sodium phosphate + 100 mM Na2SO4 (pH 7.0) as a running buffer for 30 min. The collected effluent was measured using Agilent 1220 HPLC and the data were analyzed using OpenLAB software.

**Table 7. Purity of humanized antibody**

| mAb ID | Purity (%) |
|---|---|
| 8H5 | 96.1 |

### 5.5. Binding of humanized anti-GPRC5D VHH-Fc antibody to human GPRC5D protein expressed on cell surface

Whether the humanized anti-GPRC5D VHH-Fc antibody in the present disclosure could bind to the human GPRC5D protein stably expressed on the surface of 293T cells was determined by cell binding experiments.

After enzymatic digestion, a single-cell suspension of 293T-huGPRC5D cells (constructed as described in Example 5.1) was obtained. The single-cell suspension was centrifuged at room temperature at 400× g, and the culture medium was then discarded. The cell pellet was washed once with PBS and centrifuged, and the supernatant was discarded. The cell pellet was then resuspended in the serially diluted humanized GPRC5D VHH-Fc antibody obtained in the present application (at an initial concentration of 25 µg/mL, and serially 4-fold down-diluted to 8 concentration points in total: 6.25 µg/mL, 1.563 µg/mL, 0.391 µg/mL, 0.0977 µg/mL, 0.0244 µg/mL, 0.0061 µg/mL, and 0.00153 µg/mL), and the cells were incubated at 4 °C for 30 min. The cells were washed once with PBS, and then fluorescent secondary antibody R-PE-conjugated AffiniPure Goat Anti-Human IgG, Fcy Fragment Specific (Jackson ImmunoResearch, Cat#: 109-116-098) diluted in a 1:200 ratio was added. The cells were incubated in the dark at 4 °C for 30 min. The cells were washed twice with PBS and then resuspended, and finally, PE-channel fluorescence signals were detected using a Beckman CytoFlex (Beckman) flow cytometer.

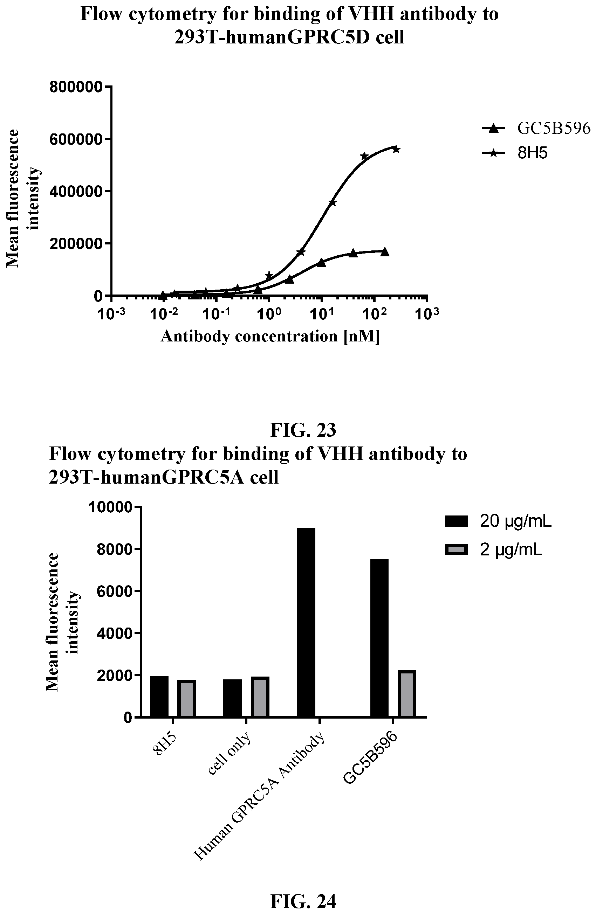

As shown in the results in FIG. 23, the humanized GPRC5D VHH-Fc antibody could bind to cells expressing human GPRC5D protein, and the binding capacity of certain clones to the cells was superior to that of the control antibody.

### 5.6. Binding assay of humanized anti-GPRC5D VHH antibody to 293T engineered cells expressing proteins of the same family:

### 5.6.1. Construction and identification of 293T engineered cells expressing proteins of the same family:

Transfected cell lines expressing GPRC5A, GPRC5B, and GPRC5C were produced using standard methods for the characterization study (Table 8).

The gene vector of the human GPRC5A protein (Q8NFJ5, SEQ ID NO: 81) was constructed by Nanjing Leadsbiolabs Co., Ltd., using cDNA (sino, Cat#: HG12833 UT) as a template to amplify a GPRC5A protein target fragment, which was subsequently cloned into a stable expression vector PiggyBac. The gene sequence of human GPRC5B protein (Q9NZH0, SEQ ID NO: 82) and human GPRC5C protein (Q9NQ84, SEQ ID NO: 83) were subjected to cell-preferred codon optimization and gene synthesis by General Biology System (Anhui) Co., Ltd., and then cloned into a stable expression vector PiggyBac. The gene sequences encoding the human GPRC5A, GPRC5B, and GPRC5C proteins were separately transfected into 293T (Cell Bank of Type Culture Collection Committee of the Chinese Academy of Sciences, Cat#: SCSP-502) cells, using a Lipofectamine^{™} 2000 (Invitrogen, Cat#: 11668019) transfection reagent. The transfected cells were subjected to pressurized screening with 0.3 µg/mL puromycin (Gibco, Cat#: A1113802) for more than one week. The cells after pressurized screening were subjected to enzymatic digestion to obtain single-cell suspensions, and the single-cell suspensions were incubated with 10 µg/mL diluted solutions of commercial positive antibodies human GPRC5A antibody (R&D, MAB5239), human GPRC5B antibody (R&D, MAB10253), and human GPRC5C antibody (R&D, MAB6594), respectively. After incubation at 4 °C for 3 min, the cell pellet was washed once with PBS and centrifuged, and the supernatant was discarded. The cell pellet was resuspended in R-Phycoerythrin AffiniPure Goat AntiMouse IgG (subclasses 1+2a+2b+3), Fcy Fragment Specific (min X Hu, Bov, Rb Sr Prot) (Jackson ImmunoResearch, 115-115-164) diluted in a 1:200 ratio, and the cells were incubated in the dark at 4 °C for 30 min. After the cells were washed twice with PBS, the positive rate of the cells was detected using flow cytometry. The flow cytometry assay showed that 293T engineered polyclonal cells expressing human GPRC5A, GPRC5B, and GPRC5C were successfully obtained through transfection (Table 8).

**Table 8: Cell line expressing GPRC5A, GPRC5B, and GPRC5C**

| Name of cell line constructed | Protein | Cell line | Promoter | Resistance |
|---|---|---|---|---|
| 293 T-humanGPRC5A | HuGPRC5A | 293T | CMV | Puromycin |
| 293T-humanGPRC5B | HuGPRC5B | 293T | CMV | Puromycin |
| 293T-humanGPRC5C | HuGPRC5C | 293T | CMV | Puromycin |

### 5.6.2. Binding of humanized anti-GPRC5D VHH antibody to 293T engineered cells expressing proteins of the same family

The three types of 293T engineered cells expressing proteins of the same family (293T-humanGPRC5A, 293T-humanGPRC5B, and 293T-humanGPRC5C) described above were subjected to enzymatic digestion to obtain single-cell suspensions. The cell pellet was resuspended in the anti-human GPRC5D VHH-Fc antibody and the control antibody GC5B596 (HC: SEQ ID NO: 67; LC: SEQ ID NO: 68) diluted with PBS to concentrations of 20 µg/mL and 2 µg/mL, and the cells were incubated in a refrigerator at 4 °C for 30 min. Meanwhile, cells without antibody treatment served as the blank control group. After washing once with PBS, the cells were resuspended in R-Phycoerythrin AffiniPure Goat Anti-Human IgG, Fcγ fragment specific (min X Bov, Hrs, Ms Sr Prot) diluted in a 1:200 ratio, and incubated in the dark at 4 °C for 30 min. After washing twice with PBS, the cells were resuspended in 100 µL of PBS, and the binding of the antibody to the cells was then detected using a Beckman CytoFlex flow cytometer. To ensure the accuracy of protein expression in the cells, flow cytometry was also performed using commercial positive antibodies human GPRC5A antibody (R&D, MAB5239), human GPRC5B antibody (R&D, MAB10253), and human GPRC5C antibody (R&D, MAB6594), following the same method for the construction of the engineered 293T cells expressing proteins of the same family.

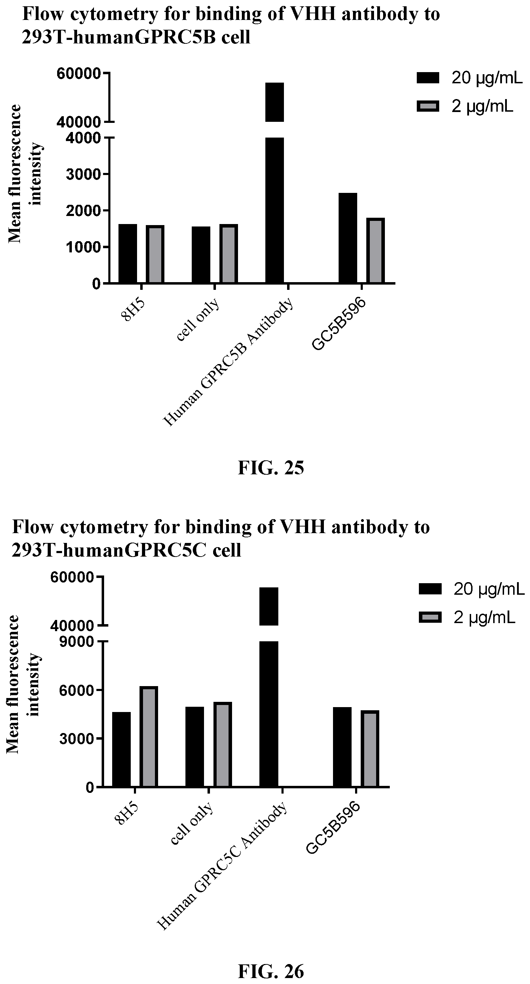

The flow cytometry assay results, shown in FIGs. 24, 25, and 26, indicated that the anti-human GPRC5D VHH antibody exhibited relatively weak binding to 293T engineered cells expressing GPRC5A, GPRC5B, and GPRC5C proteins of the same family.

### Example 6: Preparation of anti-human BCMA×GPPRC5D×CD3 trispecific antibodies and characterization thereof

### 6.1. Construction of trispecific antibodies

In the present disclosure, two different trispecific antibodies with asymmetric structures were constructed as shown in FIG. 7. The anti-human BCMA moiety of each trispecific antibody was derived from the humanized antibody huBH56-3 described above, the anti-GPRC5D moiety was derived from humanized antibodies hu8H5 and hu29H6 (the hu29H6 antibody was derived from PCT/CN2023/071314), and the anti-CD3 moiety was derived from a humanized CD3 antibody (PCT/CN2023/071314). The constant regions of the bispecific antibodies each comprised a knob-in-hole structure (KIH) (Merchant, A. M., et al. (1998). "An efficient route to human bispecific IgG." Nat Biotechnol 16(7): 677-681.). Such trispecific antibodies are also referred to herein as "anti-CD3/BCMA/GPRC5D trispecific antibodies" or "anti-CD3/anti-BCMA/anti-GPRC5D trispecific antibodies", sometimes simply referred to as "trispecific antibody molecules" or "trispecific antibodies". In the anti-CD3/BCMA/GPRC5D trispecific antibodies, the anti-BCMA moiety targeted cells expressing BCMA, the anti-GPRC5D moiety targeted cells expressing GPRC5D, or the anti-BCMA moiety and the anti-GPRC5D moiety targeted tumor cells expressing both BCMA and GPRC5D, and the anti-CD3 moiety activated T cells. The trispecific antibodies simultaneously bound to BCMA and GPRC5D on tumor cells and CD3 on T cells to facilitate targeted killing of the tumor cells by the activated T cells.

In the present application, two types of anti-CD3/BCMA/GPRC5D trispecific antibodies with the structures shown in FIG. 7 were obtained using standard construction methods:
CD3Fab heavy chain-knob: The sequence encoding the heavy chain variable region of the anti-CD3 humanized monoclonal antibody was cloned into a pcDNA3.1(+) vector containing IgG1 heavy chain constant region with Fc (knob) for expression to obtain a CD3Fab heavy chain-knob molecule.
CD3Fab light chain: The sequence encoding the light chain variable region of the anti-CD3 humanized monoclonal antibody H34L-2 was cloned into a pcDNA3.1 (+) vector containing IgG1 light chain lamda constant region for expression to obtain a CD3-LC molecule.
huBH56-3-hole: The variable region sequence of the anti-BCMA humanized antibody huBH56-3 was cloned into a pcDNA3.1(+) vector containing Fc (hole) for expression to obtain a huBH56-3-hole molecule.
hu8H5-huBH56-3-hole: The sequence encoding the hu8H5 variable region and the sequence encoding the huBH56-3 variable region were sequentially ligated in tandem using an overlapping PCR technique (with a linker GGSGG between the two encoding fragments). The ligated sequence was then cloned into a pcDNA3.1(+) vector containing Fc (hole) for expression to obtain a hu8H5-huBH56-3-hole molecule.
29H6-CD3ScFv-knob: The sequence encoding the heavy chain variable region of the anti-GPRC5D antibody 29H6 and the nucleotide sequence encoding the anti-CD3 single-chain antibody 23L2 were sequentially ligated in tandem using an overlapping PCR technique (with a linker GGGGS between the two encoding fragments). The ligated sequence was then cloned into a pcDNA3.1(+) vector containing Fc (knob) for expression to obtain a 29H6-CD3ScFv-knob molecule.
29H6Fab light chain: The sequence encoding the light chain variable region of the 29H6 was cloned into a pcDNA3.1(+) vector containing IgG1 light chain kappa constant region for expression to obtain a 29H6Fab light chain molecule.

The trispecific antibodies in FIG. 7 were obtained by accordingly combining the expression vectors described above according to the specific composition shown in Table 9 and expressing them under appropriate conditions.

The construction, expression, purification, and preliminary analysis steps of the trispecific antibodies were the same as those in Example 2.1.

**Table 9. Trispecific antibody**

| Clone | Chain | Structure | Amino acid sequence |
|---|---|---|---|
| Tri8H5BH563-CD3 Fab | HC1: hu8H5-huBH56-3-hole SEQ ID NO:58 | Hu8H5-VH | SEQ ID NO:27 |
| | | Linker peptide | SEQ ID NO:101 |
| | | huBH56-3-VH | SEQ ID NO:12 |
| | | Linker peptide (hinge region portion) | SEQ ID NO:94 |
| | | Fc (hole) (hinge region-CH2-CH3 hole) | SEQ ID NO:84 |
| | HC2: CD3-Fab HC-knob SEQ ID NO:59 CD3-Fab-HC:SEQ ID NO:11 | HuCD3-VH | SEQ ID NO:34 |
| | | CH1 | SEQ ID NO:88 |
| | | Hinge region | SEQ ID NO:96 |
| | | Fc(knob) (CH2-CH3knob) | SEQ ID NO:87 |
| | LC1: CD3Fab-LC SEQ ID NO:60 | CD3-VL | SEQ ID NO:35 |
| | | Light chain constant region (lambda) | SEQ ID NO:91 |
| TriBH563CD3G1 11-1G4S | HC1: huBH56-3-hole SEQ ID NO:56 | HuBH56-3 VH | SEQ ID NO:12 |
| | | Linker peptide (hinge region portion) | SEQ ID NO:94 |
| | | Fc(hole) (CH2-CH3knob) | SEQ ID NO:84 |
| | HC2: 29H6-CD3ScFv-knob SEQ ID NO: 61 29H6-Fab-HC (29H6-VH-CH1-hinge region): SEQ ID NO: 110 | 29H6-VH | SEQ ID NO:42 |
| | | CH1 | SEQ ID NO:88 |
| | | Hinge region | SEQ ID NO:96 |
| | | Linker peptide | SEQ ID ND:99 |
| | | CD3-ScFv | SEQ ID NO: 109 |
| | | Fc (knob) (hinge region-CH2-CH3 knob) | SEQ ID NO:47 |
| | LC1: 29H6Fab-LC SEQ ID NO:62 | 29H6-VL | SEQ ID NO:43 |
| | | Light chain constant region | SEQ ID NO:93 |

The heavy and light chain expression vectors obtained (see Table 9) were co-transfected into ExpiCHO-S cells for expression under appropriate conditions to obtain trispecific antibody proteins, and the expression, purification, and preliminary analysis steps were the same as those in Example 2.1.

The IBI3003 trispecific antibody was prepared according to the method disclosed in WO2022/174813/A1.

The BGCB491 trispecific antibody was prepared according to the method disclosed in WO2022/175255 A2.

### 6.2. Binding experiment of anti-human BCMA×GPPRC5×CD3 trispecific antibodies to tumor cells endogenously expressing human BCMA and human GPRC5D proteins

Whether the trispecific antibodies of the present disclosure could bind to proteins on the surface of tumor cells endogenously expressing human BCMA and human GPRC5D proteins was determined by cell binding experiments.

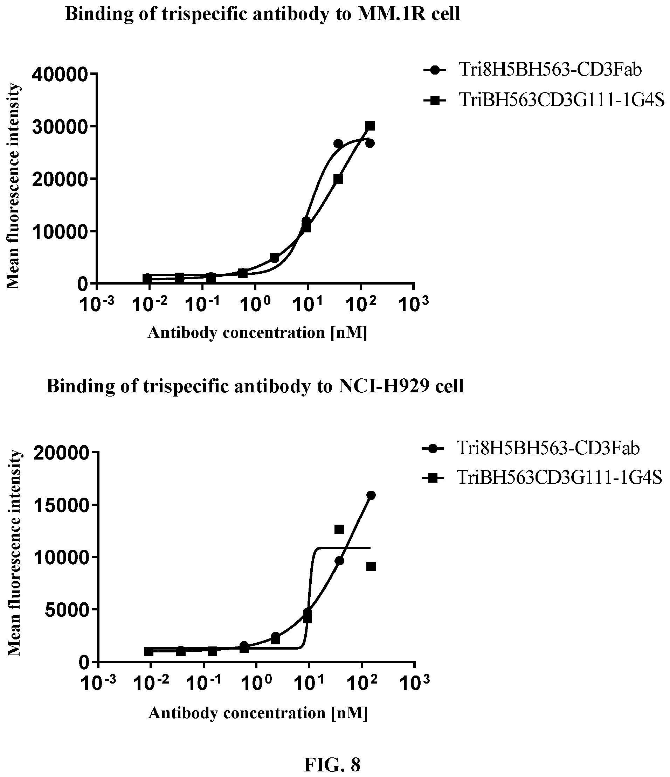

Single-cell suspensions of MM.1R (Cell Bank of Type Culture Collection Committee of the Chinese Academy of Sciences, SCSP-5050) and NCI-H929 (Nanjing Cobioer Biosciences Co., Ltd., CBP60243) cells were collected, and centrifuged at 400× g at room temperature, and the culture medium was then discarded. The cell pellet was washed once with PBS and then resuspended in the serially diluted anti-CD3/BCMA/GPRC5D trispecific antibodies (at an initial concentration of 150 nM, and serially 4-fold diluted to obtain 8 concentration points in total), and the cells were incubated at 4 °C for 30 min. The cells were washed once with PBS, and then fluorescent secondary antibody R-PE-conjugated AffiniPure Goat Anti-Human IgG, Fcγ Fragment Specific (Jackson ImmunoResearch, Cat#: 109-116-098) diluted in a 1:200 ratio was added. The cells were incubated in the dark at 4 °C for 30 min. The cells were washed twice with PBS and then resuspended, and finally, two-channel fluorescence signals were detected using a Beckman Cytoflex flow cytometer. As shown in the results in FIG. 8, all the test samples exhibited good binding activity to MM.1R and NCI-H929 tumor cells.

### 6.3. Detection of killing activity of anti-BCMA×GPPRC5D×CD3 trispecific antibodies targeting tumor cells and markers of activation and exhaustion of T cells

To detect the activity of the anti-BCMA×GPPRC5D×CD3 trispecific antibodies in mediating T cell killing of tumor cells, we established a primary T cell-dependent cytotoxicity assay system using tumor cells endogenously expressing human BCMA and GPRC5D at different levels: MM.1R (high) (Cell Bank of Type Culture Collection Committee of the Chinese Academy of Sciences, SCSP-5050), NCI-H929 (medium) (Nanjing Cobioer Biosciences Co., Ltd., CBP60243), and MOLP-8 (low) (Nanjing Cobioer Biosciences Co., Ltd., CBP60243) as the target cells, and human peripheral blood mononuclear cells (PBMCs) (obtained from healthy human blood) as the effector cells.

The PBMCs were thawed, and adjusted to a cell density of 5-10 × 10⁶ cells/mL using a 1640 complete medium (supplemented with 10% FBS). The cells were activated overnight with IL2 (Jiangsu Kingsley Pharmaceutical Co., Ltd.) at a final concentration of 100 IU/mL. The target cells MM.1R, NCI-H929, and MOLP-8 were collected by centrifugation at 400× g for 5 min, and the supernatant was discarded. The target cells were adjusted to a density of 4 × 10⁵ cells/mL using an MEM-α (Gibco, Cat#: 41061-029) assay buffer containing 1% FBS (Gibco, Cat#: TCHu44) and 100 IU/mL IL2, and seeded into a 96-well plate (Corning, Cat#: 3599) at 50 µL/well (i.e., 20,000 cells/well).

A serially diluted solution of the test antibodies (the maximum assay concentration of the test antibodies was 10 µg/mL, and the test antibodies were serially 5-fold diluted to obtain 9 concentration points in total) was prepared, and 50 µL of the prepared antibody solution was added to each well. In this study, the target cell maximal killing (2% Triton100 lysis solution was added to the target cells), minimal killing (the assay buffer was added to the target cells), and natural killing (the effector cells were added to the target cells) controls were set at 50 µL/well. The ratio of the effector cells E to the target cells T was finally 3:1, and then the cells were incubated in a cell incubator for another 48 h. After the incubation was completed, the experimental plate was taken out and centrifuged at 400× g for 3 min to allow all cells to settle to the bottom of the plate. 50 µL of supernatant was carefully pipetted into a new 96-well plate, 50 µL of LDH detection solution (Roche, Cat#: 11644793001) was added to each well, and the plate was incubated at room temperature. When the color changed, the plate was read on an F50 microplate reader. The detection wavelength was 492 nm, the reference wavelength was 650 nm, and the detection analysis was performed when the OD492 value of the maximum killing well was between 0.4 and 1.0.

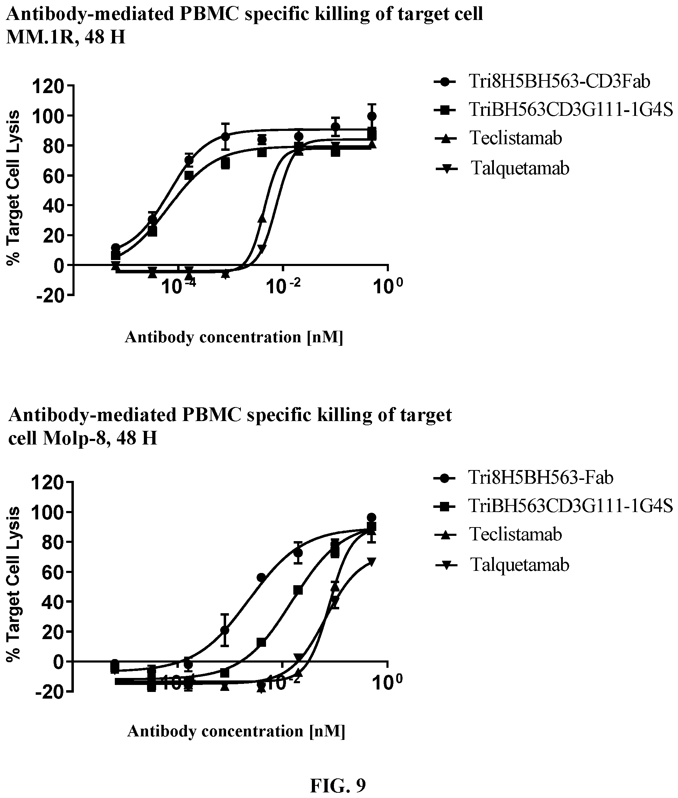

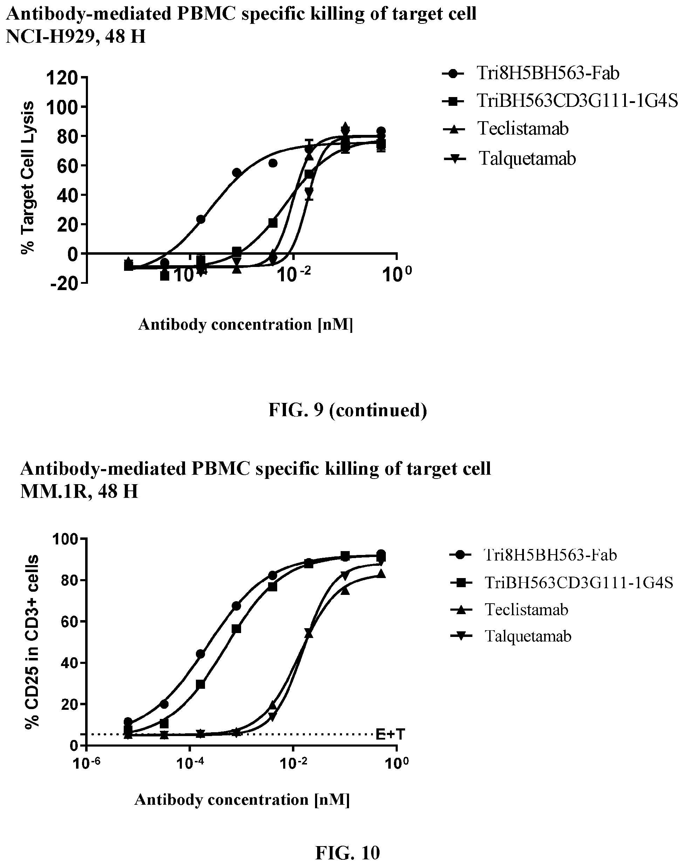

The results, shown in FIG. 9, indicated that the anti-BCMA×GPPRC5D×CD3 trispecific antibodies were capable of specifically inducing T cell killing of tumor cells, exhibiting higher killing activity compared to the control antibodies.

To detect the effects of the anti-BCMA×GPPRC5D×CD3 trispecific antibodies on T cell function in the TDCC killing system, we tested the markers of the expression of CD25 (Biolegend, Cat#: 302612), CD69 (Biolegend, Cat#: 310904), PD1 (Biolegend, Cat#: 329908), and TIM3 (Biolegend, Cat#: 345006) in T cells, as well as T cell apoptosis (Biolegend, Cat#: 423114).

The remaining cells in the sample plate of the killing experiment described above were resuspended in PBS. The cells were transferred to a 96-well V-bottom plate (NEST, Taobao) and collected by centrifugation at 500× g for 5 min, and the supernatant was discarded. The cells were resuspended in PBS containing a detection antibody. After incubation at 4 °C for 30 min, the plate was taken out and centrifuged at 500× g for 5 min, and the supernatant was discarded. The cells were resuspended in PBS for flow cytometry to test the changes of the markers of T cell activation (the expression of CD25 and CD69), T cell exhaustion (the expression of PD1 and TIM3), as well as T cell apoptosis.

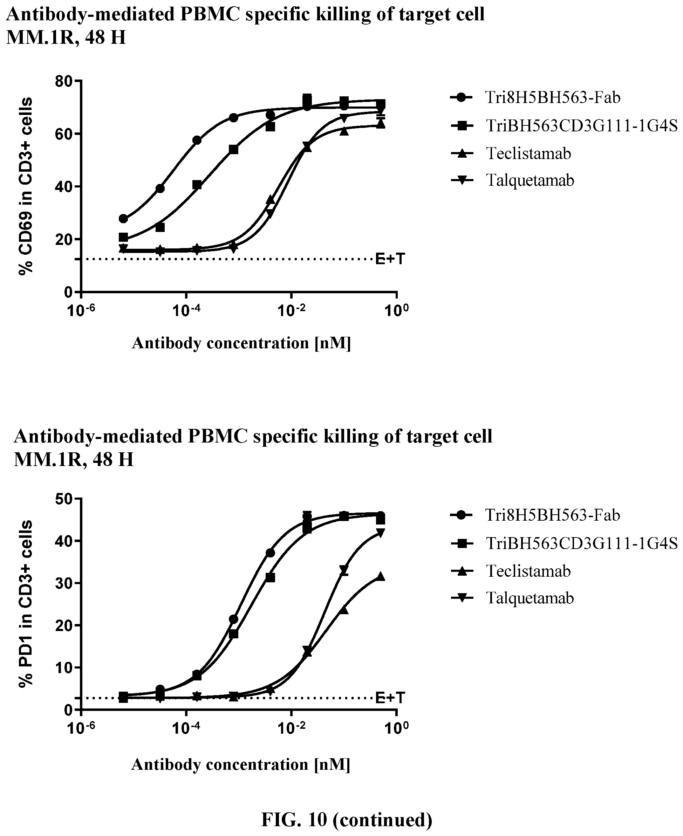

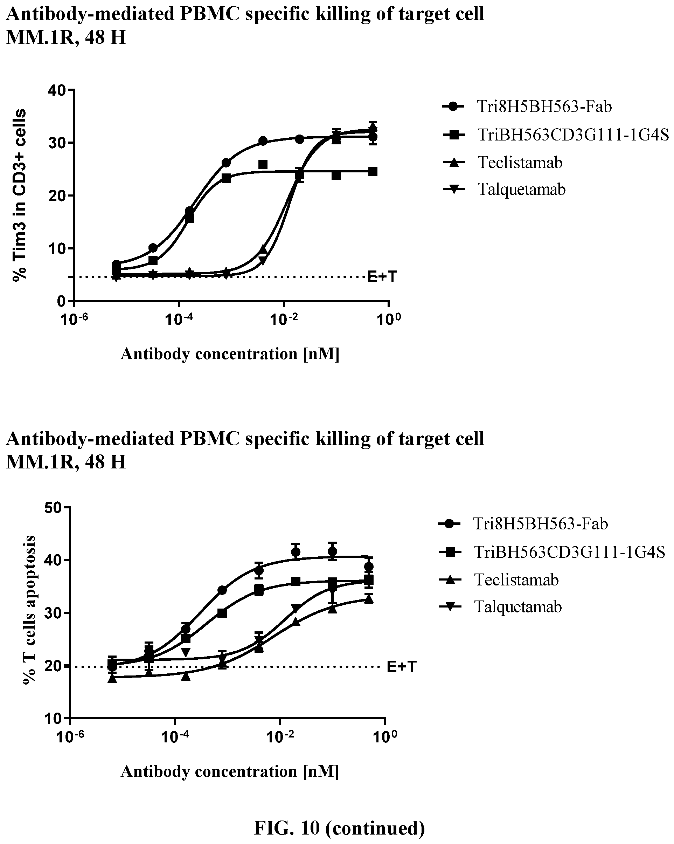

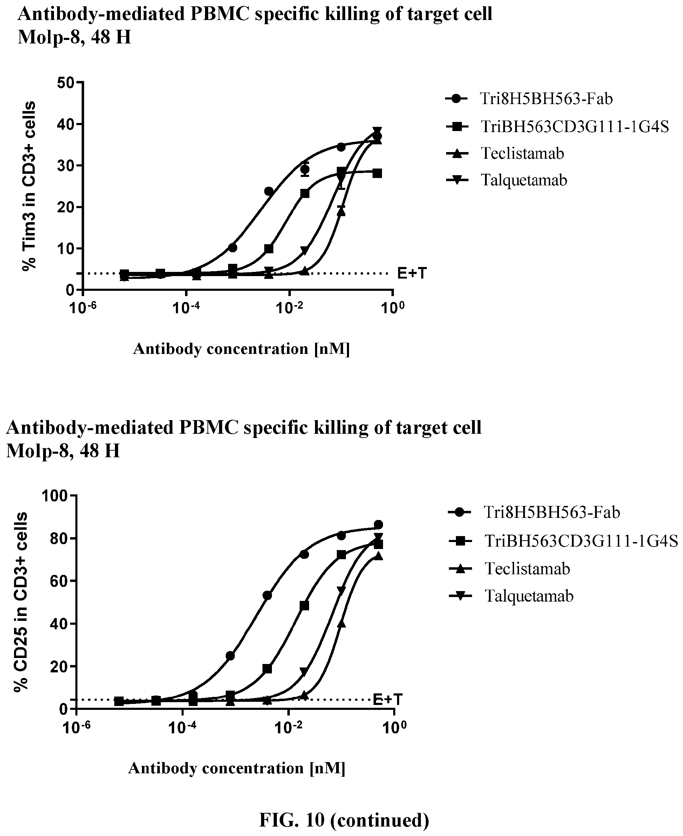

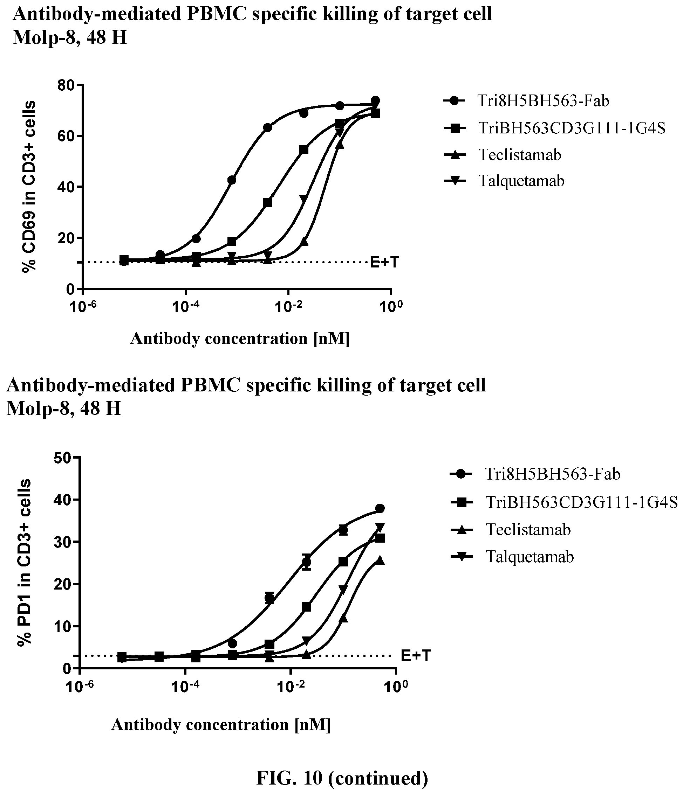

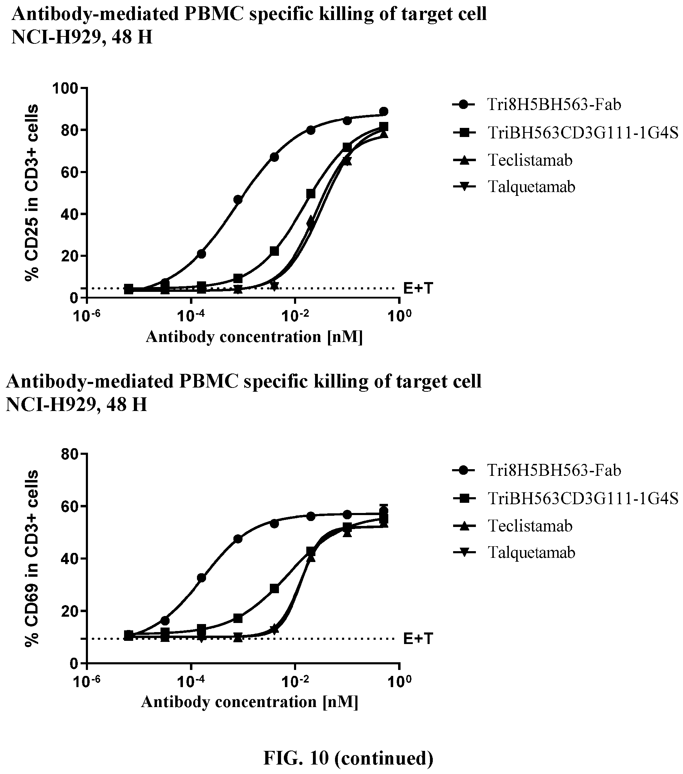

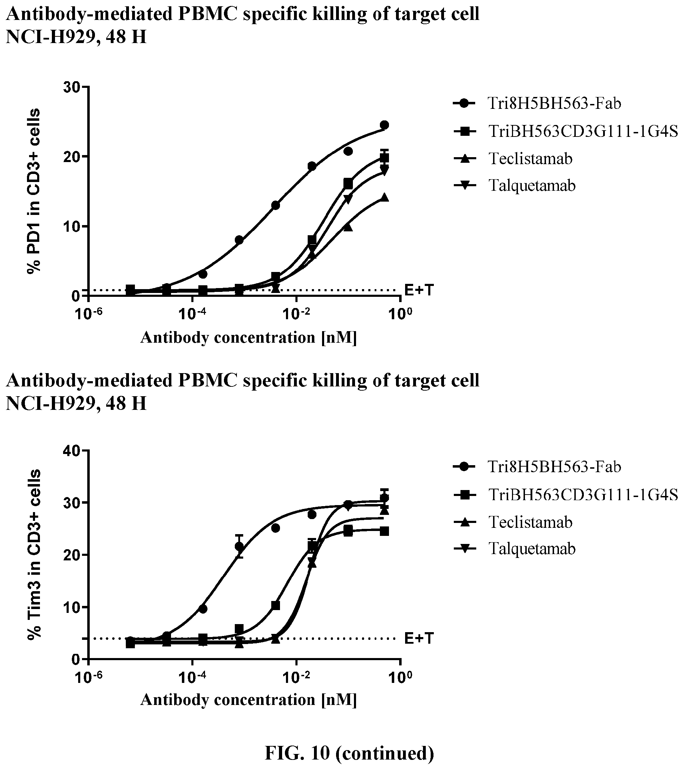

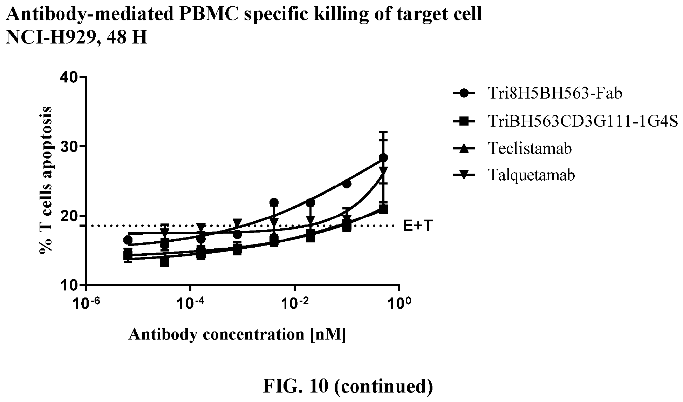

The results, shown in FIG. 10, indicated that the detection results for the markers of the activation and exhaustion of T cells were substantially consistent with the killing trend, which demonstrated that the anti-BCMA×GPPRC5D×CD3 trispecific antibodies had a stronger effect on the activation of T cells compared to the control antibodies, and also promoted greater T cell apoptosis.

### 6.4. Induction of cytokine release after activation of T cells by anti-BCMA×GPPRC5D×CD3 trispecific antibodies

To detect the induction of cytokine secretion by the anti-BCMA×GPPRC5D×CD3 trispecific antibodies while mediating T cell killing of tumor cells, we established a primary T cell-dependent cytotoxicity assay system using MM.1R, NCI-H929, and MOLP-8 cells endogenously expressing human BCMA and GPRC5D as the target cells, and human peripheral blood mononuclear cells (PBMCs) as the effector cells. The killing and incubation were performed as shown in 5.8. At the endpoint of the killing, the supernatant was collected for the detection of IL-6, TNF-α, and IFN-γ in the system.

After the killing and incubation were completed, the experimental plate was taken out and centrifuged at 500× g for 5 min to allow all cells to settle to the bottom of the plate. 100 µL of the supernatant was carefully pipetted into a new 96-well plate, and the levels of human IFNγ (detection kit: Cisbio, Cat#: 62HIFNGPEH), human TNFα (detection kit: R&D, Cat#: DY210), and human IL-6 (detection kit: R&D, Cat#: DY206) in the supernatant were detected, separately.

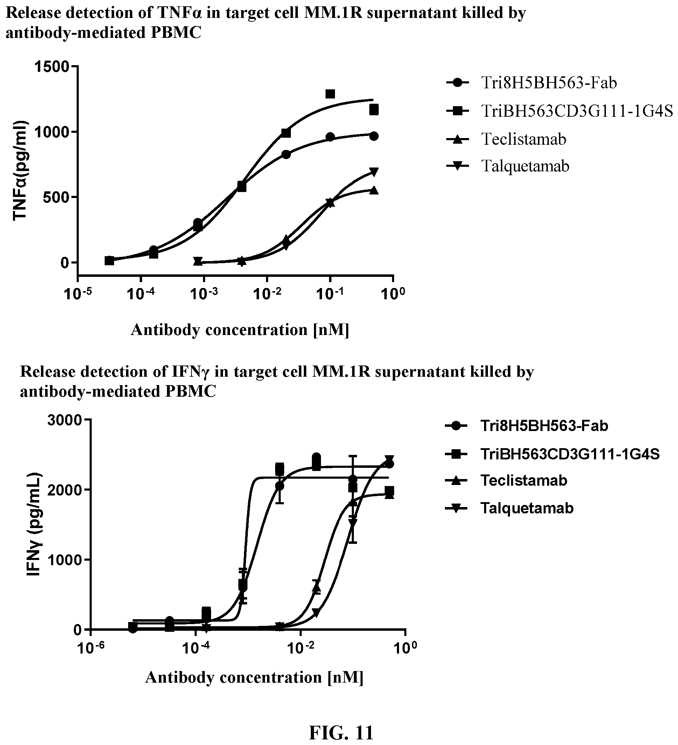

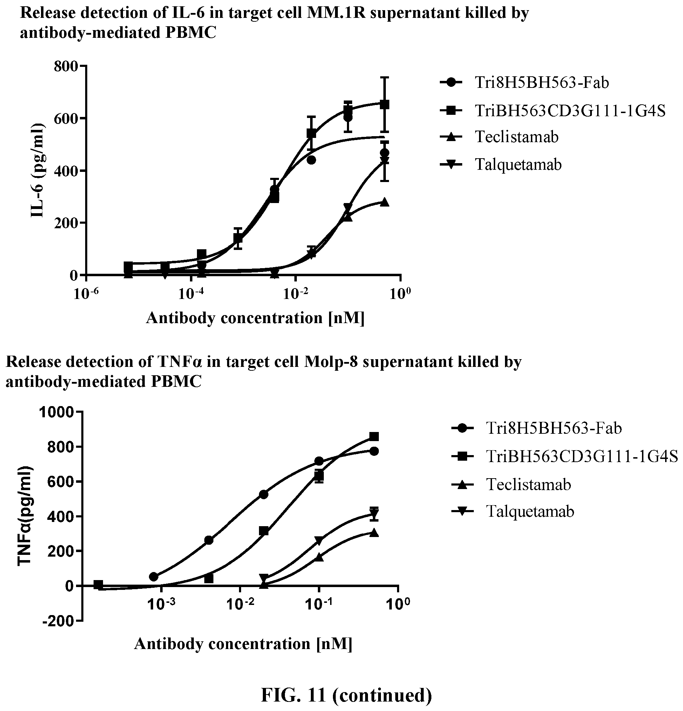

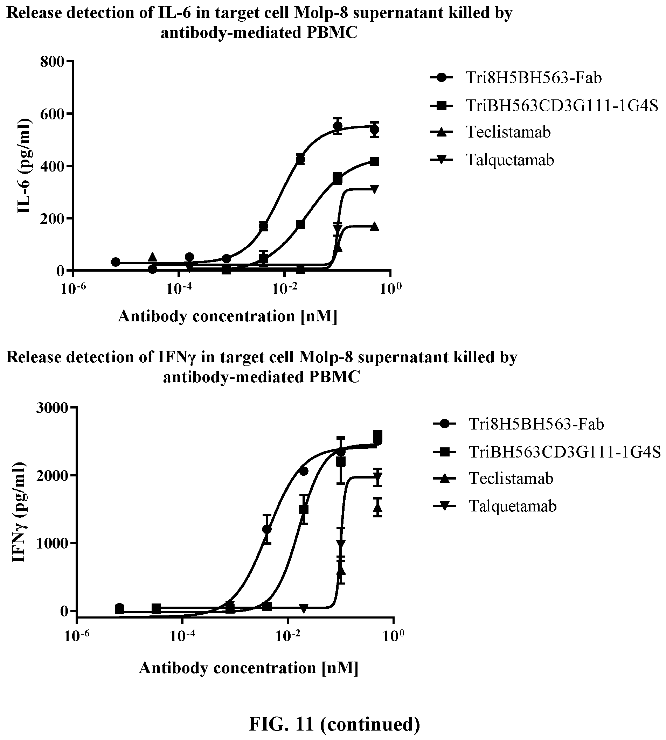

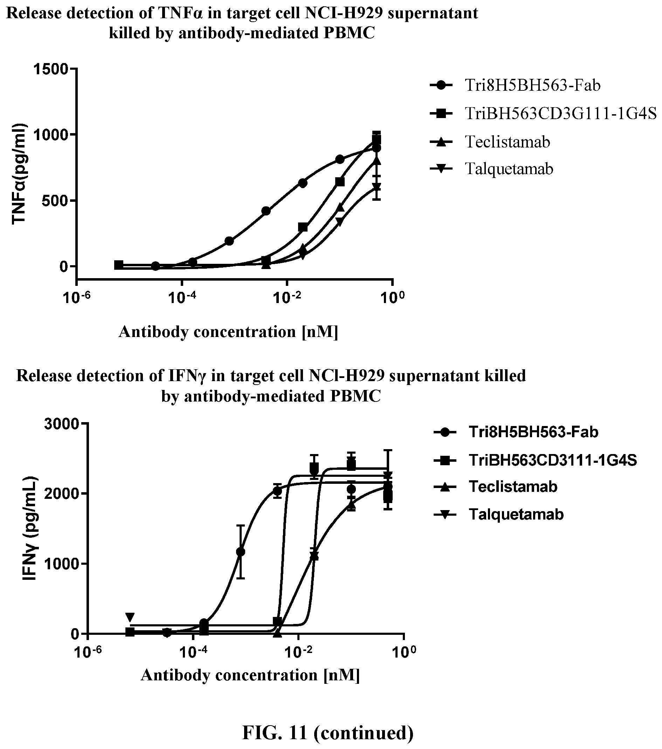

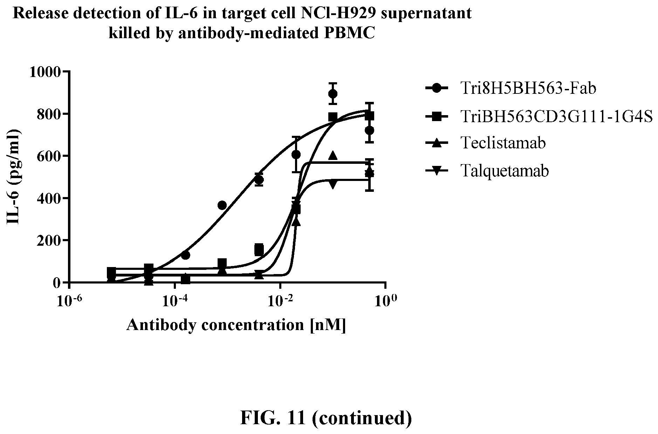

The results, shown in FIG. 11, indicated that the anti-BCMA×GPPRC5D×CD3 trispecific antibodies were capable of inducing T cells to release human IFNγ (FIG.), human TNFα (FIG.), and human IL-6 (FIG.) in the MM.1R, NCI-H929, and MOLP-8 systems, and the cytokine release trend was consistent with the killing results: the stronger the killing effect, the higher the cytokine release level.

### 6.5. Comparison of killing activity of anti-CD3×BCMA×GPRC5D trispecific antibodies to CD3×BCMA and CD3×GPRC5D diabodies in combination on tumor cells and negative cells

To compare the activity of anti-CD3×BCMA×GPRC5D trispecific antibodies as well as Teclistamab and Talquetamab diabodies in combination in mediating T cell killing of tumor cells, we established a primary T cell-dependent cytotoxicity assay system using MOLP-8 (Nanjing Cobioer Biosciences Co., Ltd., CBP60243) tumor cells and 293T (Cell Bank of Type Culture Collection Committee of the Chinese Academy of Sciences, GNHu17) negative cell lines both endogenously expressing human BCMA and GPRC5D at low levels as the target cells, and human peripheral blood mononuclear cells (PBMCs) (obtained from healthy human blood) as the effector cells.

The PBMCs were thawed, and adjusted to a cell density of 5-10 × 10⁶ cells/mL using a 1640 complete medium (supplemented with 10% FBS). The cells were activated overnight with IL2 (Jiangsu Kingsley Pharmaceutical Co., Ltd.) at a final concentration of 100 IU/mL. The target cells MOLP-8 and 293T were collected by centrifugation at 400× g for 5 min, and the supernatant was discarded. The cells were washed once with PBS, and then a dye CellTrace^{™} Violet (1 × 10⁷ cells/µL) (Invitrogen, Cat#: C34557) was added to label the cells. The mixture was incubated at 37 °C for 15 min, and subsequently, an equal volume of serum was added to stop the staining. The mixture was incubated at 37 °C for another 5 min. The cells were centrifuged at 400 g for 4 min, and the supernatant was then removed. The labeled target cells were adjusted to a density of 4 × 10⁵ cells/mL using an MEM-α (Gibco, Cat#: 41061-029) assay buffer containing 1% FBS (Gibco, Cat#: TCHu44) and 100 IU/mL IL2, and seeded into a 96-well plate (Corning, Cat#: 3599) at 50 µL per well (i.e., 20000 cells per well).

A serially diluted solution of the test antibodies (the maximum assay concentration of the test antibodies was 2 nM, and the test antibodies were serially 5-fold diluted to obtain 8 concentration points in total) was prepared, and 50 µL of the prepared antibody solution was added to each well. In this study, the target cell maximal killing (2% Triton100 lysis solution was added to the target cells), minimal killing (the assay buffer was added to the target cells), and natural killing (the effector cells were added to the target cells) controls were set at 50 µL/well. The ratio of the effector cells E to the target cells T was finally 3:1, and then the cells were incubated in a cell incubator for another 48 h. After the incubation was completed, the experimental plate was taken out and centrifuged at 400× g for 3 min to allow all cells to settle to the bottom of the plate. 100 µL of the supernatant was carefully pipetted into a new 96-well plate for subsequent use. The remaining cells were resuspended in PBS and then transferred to a 96-well V-bottom plate (NEST, Cat#: 701201). The cells were collected by centrifugation at 500× g for 5 min, and the supernatant was discarded. The cells were resuspended in PBS containing a detection antibody with Fixable Viability Stain 780 (Bioscience, Cat#: 565388) (1:2000 dilution). After incubation at 4 °C for 30 min, the experimental plate was taken out and centrifuged at 500× g for 5 min, and the supernatant was discarded. The cells were resuspended in PBS for flow cytometry to detect the killing of the target cells.

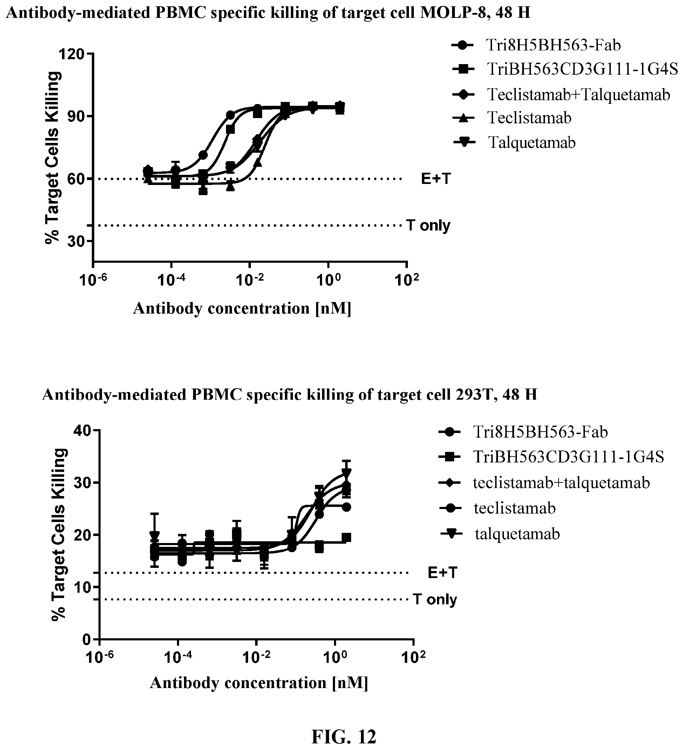

The results, shown in FIG. 12, indicated that the effect of T cell killing of tumor cells mediated by the anti-CD3/BCMA/GPRC5D trispecific antibodies was stronger than the killing effect of the control antibodies Teclistamab and Talquetamab in combination. In addition, the TriBH563CD3G111-1G4S trispecific antibody exhibited no non-specific killing effect on 293T negative cells.

### 6.6. Induction of cytokine release after activation of T cells by anti-CD3×BCMA×GPRC5D trispecific antibodies and CD3×BCMA and CD3×GPRC5D diabodies in combination on tumor cells and negative cells

To compare the induction of cytokine secretion by the **anti-CD3×BCMA×GPRC5D** trispecific antibodies as well as Teclistamab and Talquetamab diabodies in combination while mediating T cell killing of tumor cells, we established a primary T cell-dependent cytotoxicity assay system using MOLP-8 (Nanjing Cobioer Biosciences Co., Ltd., CBP60243) tumor cells and 293T (Cell Bank of Type Culture Collection Committee of the Chinese Academy of Sciences, GNHu17) negative cell lines both endogenously expressing human BCMA and GPRC5D at low levels as the target cells, and human peripheral blood mononuclear cells (PBMCs) (obtained from healthy human blood) as the effector cells. The killing and incubation were performed as shown in Example 6.3. At the endpoint of the killing, the supernatant was collected for detection of TNF-α release in the system.

After the killing and incubation were completed, the experimental plate was taken out and centrifuged at 500× g for 5 min to allow all cells to settle to the bottom of the plate. 100 µL of the supernatant was carefully pipetted into a new 96-well plate, and the release levels of human TNFα in the supernatant were detected (detection kit: R&D, Cat#: DY210), separately.

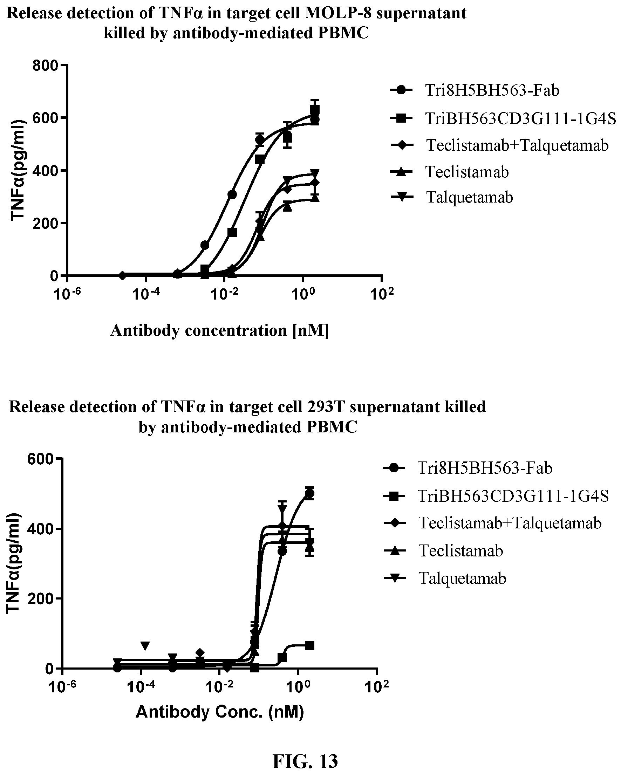

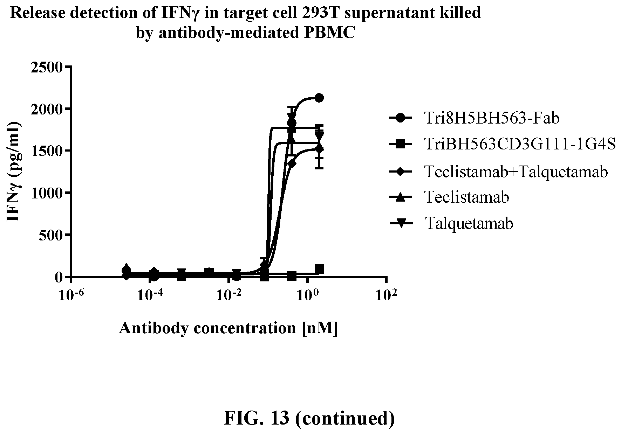

The results, shown in FIG. 13, indicated that the cytokine release trend was consistent with the killing results: the stronger the killing effect, the higher the cytokine release level. In addition, the TriBH563CD3G111-1G4S trispecific antibody exhibited no significant effect of promoting T cell cytokine release in the 293T negative cell killing experiment.

### 6.7. Binding experiment of anti-CD3×BCMA×GPRC5D trispecific antibodies to tumor cells endogenously expressing human BCMA and human GPRC5D proteins

Whether the trispecific antibodies of the present disclosure could bind to proteins on the surface of tumor cells endogenously expressing human BCMA and human GPRC5D proteins was determined by cell binding experiments.

Single-cell suspensions of AMO-1 (Nanjing Cobioer Biosciences Co., Ltd., CBP60242) and ARD (Wuhan Pricella Biotechnology Co., Ltd., CL-0778) were collected, and centrifuged at 400× g at room temperature, and the culture medium was then discarded. The cell pellet was washed once with PBS and then resuspended in the serially diluted anti-CD3/BCMA/GPRC5D trispecific antibodies (at an initial concentration of 200 nM, and serially 5-fold diluted to obtain 8 concentration points in total), and the cells were incubated at 4 °C for 30 min. The cells were washed once with PBS, and then fluorescent secondary antibody R-PE-conjugated AffiniPure Goat Anti-Human IgG, Fcγ Fragment Specific (Jackson ImmunoResearch, Cat#: 109-116-098) diluted in a 1:200 ratio was added. The cells were incubated in the dark at 4 °C for 30 min. The cells were washed twice with PBS and then resuspended, and finally, two-channel fluorescence signals were detected using a Beckman Cytoflex flow cytometer.

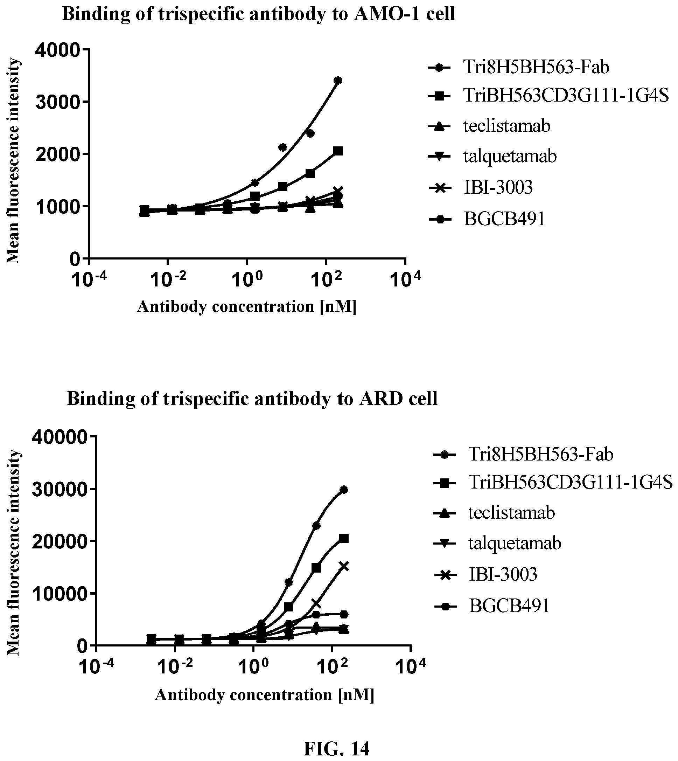

As shown in the results in FIG. 14, the test trispecific antibodies exhibited good binding activity to AMO-1 and ARD tumor cells, and the binding activity was stronger than that of the control diabodies Teclistamab and Talquetamab and the control trispecific antibodies IBI3003 and BGCB491.

### 6.8. Killing activity of anti-CD3×BCMA×GPRC5D trispecific antibodies targeting tumor cells

To detect the activity of the anti-CD3×BCMA×GPRC5D trispecific antibodies in mediating T cell killing of tumor cells, we established a primary T cell-dependent cytotoxicity assay system using AMO-1 (Nanjing Cobioer Biosciences Co., Ltd., CBP60242) and ARD (Wuhan Pricella Biotechnology Co., Ltd., CL-0778) tumor cells endogenously expressing human BCMA and GPRC5D at different levels as the target cells, and human peripheral blood mononuclear cells (PBMCs) (obtained from healthy human blood) as the effector cells.

The PBMCs were thawed, and adjusted to a cell density of 5-10 × 10⁶ cells/mL using a 1640 complete medium (supplemented with 10% FBS). The cells were activated overnight with IL2 (Jiangsu Kingsley Pharmaceutical Co., Ltd.) at a final concentration of 100 IU/mL. The target cells AMO-1 and ARD were collected by centrifugation at 400× g for 5 min, and the supernatant was discarded. The cells were washed once with PBS, and then a dye CellTrace^{™} Violet (1 × 10⁷ cells/µL) (Invitrogen, Cat#: C34557) was added to label the cells. The mixture was incubated at 37 °C for 15 min, and subsequently, an equal volume of serum was added to stop the staining. The mixture was incubated at 37 °C for another 5 min. The cells were centrifuged at 400 g for 4 min, and the supernatant was then removed. The labeled target cells were adjusted to a density of 4 × 10⁵ cells/mL using an MEM-α (Gibco, Cat#: 41061-029) assay buffer containing 1% FBS (Gibco, Cat#: TCHu44) and 100 IU/mL IL2, and seeded into a 96-well plate (Corning, Cat#: 3599) at 50 µL per well (i.e., 20,000 cells per well).

A serially diluted solution of the test antibodies (the maximum assay concentration of the test antibodies was 0.5 nM, and the test antibodies were serially 5-fold diluted to obtain 8 concentration points in total) was prepared, and 50 µL of the prepared antibody solution was added to each well. In this study, the target cell maximal killing (2% Triton100 lysis solution was added to the target cells), minimal killing (the assay buffer was added to the target cells), and natural killing (the effector cells were added to the target cells) controls were set at 50 µL/well. The ratio of the effector cells E to the target cells T was finally 3:1, and then the cells were incubated in a cell incubator for another 48 h. After the incubation was completed, the experimental plate was taken out and centrifuged at 400× g for 3 min to allow all cells to settle to the bottom of the plate. 100 µL of the supernatant was carefully pipetted into a new 96-well plate for subsequent use. The remaining cells were resuspended in PBS and then transferred to a 96-well V-bottom plate (NEST, Taobao). The cells were collected by centrifugation at 500× g for 5 min, and the supernatant was discarded. The cells were resuspended in PBS containing a detection antibody with Fixable Viability Stain 780 (Bioscience, Cat#: 565388) (1:2000 dilution). After incubation at 4 °C for 30 min, the experimental plate was taken out and centrifuged at 500× g for 5 min, and the supernatant was discarded. The cells were resuspended in PBS for flow cytometry to detect the killing of the target cells.

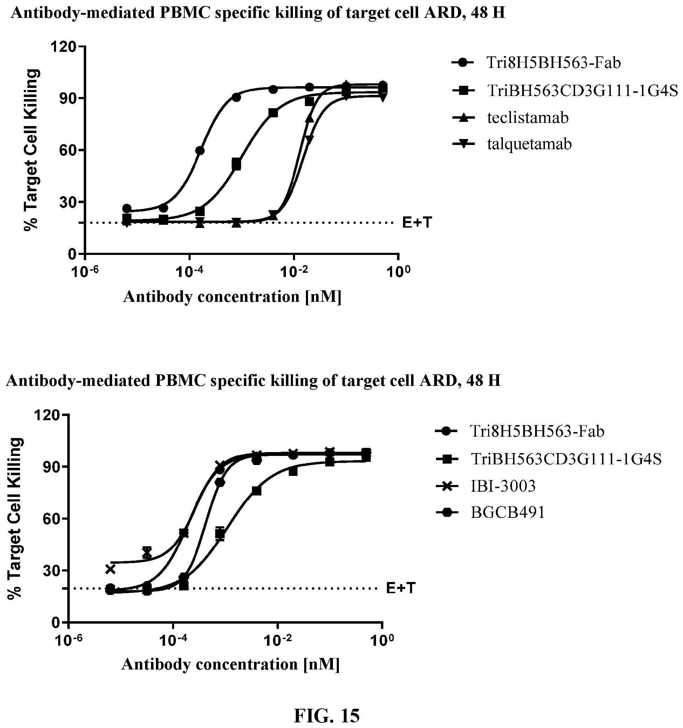

The results, shown in FIG. 15, indicated that the anti-CD3×BCMA×GPRC5D trispecific antibodies were capable of specifically inducing T cell killing of tumor cells, and the killing activity of trispecific antibody Tri8H5BH563-Fab was stronger than that of the control diabodies Teclistamab and Talquetamab and the control trispecific antibodies IBI3003 and BGCB491.

### 6.9. Induction of cytokine release after activation of T cells by anti-CD3×BCMA×GPRC5D trispecific antibodies

To detect the induction of cytokine secretion by the anti-CD3×BCMA×GPRC5D trispecific antibodies while mediating T cell killing of tumor cells, we established a primary T cell-dependent cytotoxicity assay system using AMO-1 and ARD cells endogenously expressing human BCMA and GPRC5D as the target cells, and human peripheral blood mononuclear cells (PBMCs) as the effector cells. The killing and incubation were performed as shown in Supplementary Example 2. At the endpoint of the killing, the supernatant was collected for detection of TNF-α release in the system.

After the killing and incubation were completed, the experimental plate was taken out and centrifuged at 500× g for 5 min to allow all cells to settle to the bottom of the plate. 100 µL of the supernatant was carefully pipetted into a new 96-well plate, and the release levels of human TNFα in the supernatant were detected (detection kit: R&D, Cat#: DY210), separately.

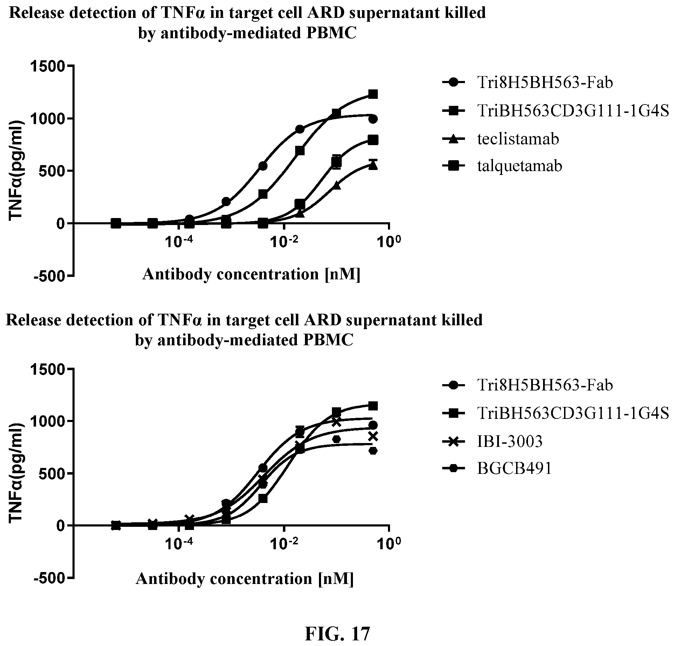

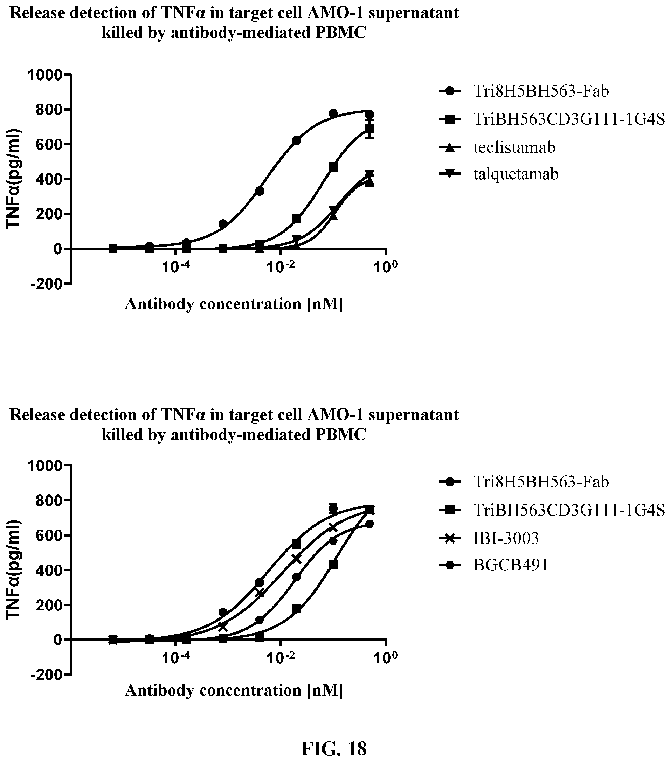

The results, shown in FIGs. 17 and 18, indicated that the anti-CD3×BCMA×GPRC5D trispecific antibodies were capable of inducing T cells to release human TNFα in the AMO-1 and ARD systems (FIGs. 17 and 18). Moreover, the cytokine release trend was consistent with the killing results: the stronger the killing effect, the higher the cytokine release level.

### Example 7: In vivo efficacy of anti-BCMA×GPRC5D×CD3 trispecific antibody in hPBMC-B-NDG mouse NCI-H929 tumor model

The *in vivo* anti-tumor effect of the anti-BCMA×GPRC5D×CD3 trispecific antibody was studied in an hPBMC-B-NDG mouse NCI-H929 tumor model.

The severe immunodeficiency B-NDG mice were purchased from Biocytogen Pharmaceuticals (Beijing) Co., Ltd., and the human peripheral blood mononuclear cells (PBMCs) were purchased from Milecell Biotechnology Inc.

The NCI-H929 human myeloma cells were purchased from Nanjing Cobioer Biosciences Co., Ltd. The cells were cultured in an incubator containing 5% CO₂ at 37 °C, and the culture medium was a RPMI 1640 medium containing 10% inactivated fetal bovine serum.

On Day-8, 18 severe immunodeficiency B-NDG mice were first inoculated with 5.5 × 10⁶ human peripheral blood mononuclear cells in 0.2 mL of RPMI 1640 per mouse via the tail vein. Subsequently, on day 0, 5 × 10⁶ NCI-H929 cells in 0.2 mL of RPMI 1640 + Matrigel were subcutaneously implanted into the right flank of each mouse. When the average tumor volume reached about 147.7 mm³, 12 appropriate mice were selected for study and randomly divided into 2 experimental groups (6 mice per group) based on mouse tumor volume and PBMC reconstitution (hCD 45% proportion). On days 0, 3, 7, and 10 after grouping, the blank control PBS, Talquetamab (0.3 mg/kg), and the trispecific antibody Tri8H5BH563-Fab (0.3 mg/kg) were administered intraperitoneally to the mice, separately. During the experiment, the tumor volume was monitored and measured twice a week using a vernier caliper.

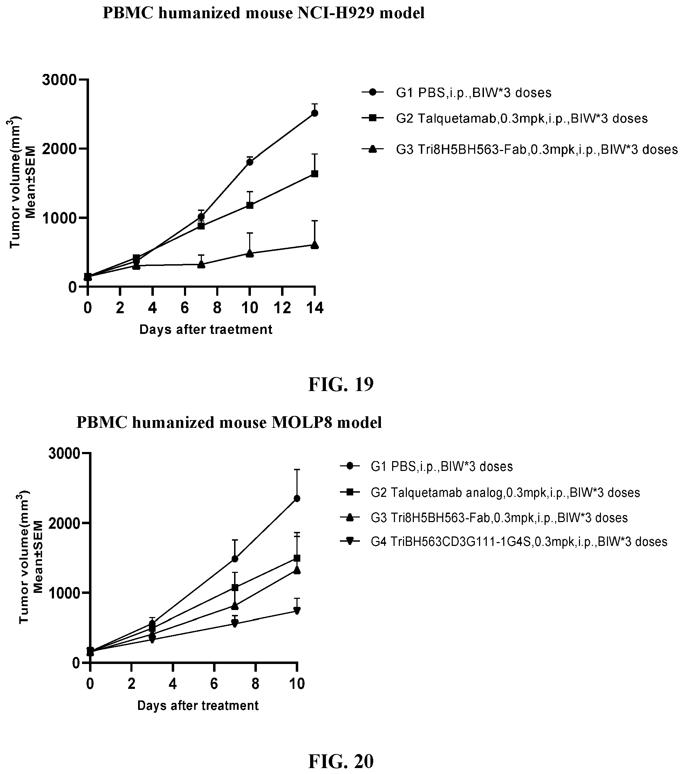

The treatment with the anti-GPRC5D×CD3 bispecific antibody Talquetamab resulted in inhibition of tumor growth compared to the PBS group, but there was no significant difference, with a TGI of 37%; the treatment with the anti-BCMA×GPRC5D×CD3 trispecific antibody Tri8H5BH563-Fab resulted in significant inhibition of tumor growth compared to the PBS group, with a TGI of 80.4%. The results are shown in FIG. 19 and Table 7 below.

**Table 10. Growth inhibition of NCI-H929 tumors by test substances**

| Group | Dose | Number of animals (N) | Tumor volume (mm³)^{a} (on day 14 after first administration) | TGI(%)^{b} | P^{c} |
|---|---|---|---|---|---|
| Blank control (PBS) | / | 6 | 2513.49±146.83 | - | / |
| Talquetamab | 0.3 mg/kg | 6 | 1637.6±309.63 | 37.0 | 0.0673 |
| Tri8H5BH563-Fab | 0.3 mg/kg | 6 | 608.90±384.46 | 80.4 | ***0.0005 |

| | | | | | |
|---|---|---|---|---|---|
| Note: a. Mean ± standard error; b. TGI = (1 - change in tumor volume of administration group/change in tumor volume of control group) × 100%; c. Tumor volumes in the administration group and the PBS control group were statistically analyzed (t tests) on day 14 of group administration,***: P < 0.001. | | | | | |

Throughout the course of the experiment, the animals maintained good mobility and feeding status during the administration period, the body weight of the animals in the G1 group increased to some extent, and the body weight of the animals in the G2 group did not significantly decrease, indicating that the animals were well tolerated by the test substances. The body weight changes of all animals are shown in Table 11.

**Table 11. Effect of test substance on mouse body weight**

| Group | Body weight (g)^{a} | | | Day 14 after administration Body weight change (%) |
|---|---|---|---|---|
| | Before administration | Day 14 of group administration | p^{b} | |
| Blank control (PBS) | 21.6±0.8 | 22.8±1.5 | - | +5.3 |
| Talquetamab | 22.2±0.6 | 22.5±1.0 | 0.9778 | +1.4 |
| Tri8H5BH563-Fab | 22.0±0.6 | 21.7±1.1 | 0.5460 | -0.9 |

| | | | | |
|---|---|---|---|---|
| Note: a. Mean ± standard error; b. Body weight in the administration group and the PBS control group were statistically analyzed (t tests) on day 14 of group administration. | | | | |

### Example 8: In vivo efficacy of anti-BCMA×GPRC5D×CD3 trispecific antibodies in hPBMC-NCG mouse MOLP8 tumor model

The *in vivo* anti-tumor effect of the anti-BCMA×GPRC5D×CD3 trispecific antibodies was studied in an hPBMC-NCG mouse MOLP8 tumor model.

The severe immunodeficiency NCG mice were purchased from GemPharmatech Co., Ltd., and the human peripheral blood mononuclear cells (PBMCs) were purchased from Milecell Biotechnology Inc.

The MOLP8 human myeloma cells were purchased from Nanjing Cobioer Biosciences Co., Ltd. The cells were cultured in an incubator containing 5% CO₂ at 37 °C, and the culture medium was a RPMI 1640 medium containing 20% inactivated fetal bovine serum.

On Day-8, 36 severe immunodeficiency NCG mice were first inoculated with 5 × 10⁶ human peripheral blood mononuclear cells in 0.2 mL of RPMI 1640 per mouse via the tail vein. Subsequently, on day 0, 5 × 10⁶ MOLP8 cells in 0.2 mL of RPMI 1640 + Matrigel were subcutaneously implanted into the right flank of each mouse. When the average tumor volume reached about 160.6 mm³, 24 appropriate mice were selected for study and randomly divided into 4 experimental groups (6 mice per group) based on mouse tumor volume and PBMC reconstitution (hCD 45% proportion). On days 0, 3, and 7 after grouping, the blank control PBS, the control antibody Talquetamab, and the trispecific antibodies Tri8H5BH563-Fab and TriBH563CD3G111-1G4S (0.3 mg/kg) were administered intraperitoneally to the mice, separately. During the experiment, the tumor volume was monitored and measured twice a week using a vernier caliper.

The treatment with the anti-GPRC5D×CD3 control antibody Talquetamab and the anti-BCMA×GPRC5D×CD3 trispecific antibody Tri8H5BH563-Fab resulted in inhibition of tumor growth compared to the PBS group, but there was no significant difference, with TGIs of 39% and 46.4%, respectively; the treatment with TriBH563CD3G111-1G4S resulted in significant inhibition of tumor growth compared to the PBS group, with TGIs of 46.4% and 73.8%, respectively. The results are shown in FIG. 20 and Table 12 below.

**Table 12. Growth inhibition of MOLP8 tumor by test substance**

| Group | Dose | Number of animals (N) | Tumor volume (mm³)^{a} (on day 10 after first administration) | TGI(%)^{b} | P^{c} |
|---|---|---|---|---|---|
| Blank control (PBS) | / | 6 | 2354.12±453.87 | - | / |
| Talquetamab | 0.3 mg/kg | 6 | 1497.29±400.95 | 39.0 | 0.2798 |
| Tri8H5BH563-Fab | 0.3 mg/kg | 6 | 1328.98±524.71 | 46.4 | 0.1331 |
| TriBH563CD3G1 11-1G4S | 0.3 mg/kg | 6 | 739.03±199.86 | 73.8 | *0.0166 |

| | | | | | |
|---|---|---|---|---|---|
| Note: a. Mean ± standard error; b. TGI = (1 - change in tumor volume of administration group/change in tumor volume of control group) × 100%; c. Tumor volumes in the administration group and the PBS control group were statistically analyzed (One-way ANOVA) on day 10 of group administration,*: *P* < 0.05. | | | | | |

Throughout the course of the experiment, the animals maintained good mobility and feeding status during the administration period, and the body weight of the animals in the G1-G3 groups increased to some extent, indicating that the animals were well tolerated by the test substances. The body weight changes of all animals are shown in Table 13.

**Table 13. Effect of test substance on mouse body weight**

| Group | Body weight (g) ^{a} | | | Day 10 after administration Body weight change (%) |
|---|---|---|---|---|
| | Before administration | Day 10 of group administration | p^{b} | |
| Blank control (PBS) | 22.5±0.9 | 25.2±1.2 | - | +11.9 |
| Talquetamab | 22.3±0.8 | 24.0±0.8 | 0.6202 | +7.6 |
| Tri8H5BH563-Fab | 22.1±0.3 | 23.2±0.9 | 0.2125 | +5.4 |
| TriBH563CD3G111-1G4S | 20.6±1.0 | 21.9±0.6 | *0.0283 | +7.2 |

| | | | | |
|---|---|---|---|---|
| Note: a. Mean ± standard error; b. Body weight in the administration group and the PBS control group were statistically analyzed (One-way ANOVA) on day 10 of group administration, *: P < 0.05. | | | | |

All publications, patent applications, patents, and other references mentioned herein are incorporated by reference in their entireties. Any or all of the features described above and throughout the present application may be combined in various embodiments of the present disclosure. In addition, the materials, methods, and examples described herein are illustrative only and are not intended to be limiting. Other features, objectives, and advantages of the present disclosure will be apparent from this description and drawings, and from the appended claims.

### Sequence Information

| SEQ NO. | Sequence information | Specific sequence |
|---|---|---|
| 1 | BH56-VHH>HCDR1 | PSAMS |
| 2 | BH56-VHH>HCDR2 | GIYGDGRTTYADSVKG |
| 3 | BH56-VHH>HCDR3 | GIRLLSESWAQAL |
| 4 | BH56-VHH>VH | EVQVVESGGGLVQAGESLTLSCAASGFTFAPSAMSWARQPPGKEVEWVSGIYGDG RTTYADSVKGRFTISRDNAKKTVTLQMNNLEPEDSGNYICVVGIRLLSESWAQALR GQGTQVTVSS |
| 5 | HA68-VHH>HCDR1 | PSAMN |
| 6 | HA68-VHH>HCDR2 | GIYGDGKAEYADSVKG |
| 7 | HA68-VHH>HCDR3 | GIRPTDAPWAASL |
| 8 | HA68-VHH>VH | QVQLVETGGGLVQPGGSLRLSCAASGFTFSPSAMNWARQAPGKGLEWVAGIYGD GKAEYADSVKGRFTISRDNAKNMVYLQMVNLRPEDTAVYSCAVGIRPTDAPWAA SLRGQGTQVTVSS |
| 1 | huBH56-1>HCDR1 | SEQ ID NO:1 |
| 9 | huBH56-1>HCDR2 | VIYGDGRTYYADSVKG |
| 3 | huBH56-1>HCDR3 | SEQ ID NO:3 |
| 10 | huBH56-1>VH | EVQLVESGGGLIQPGGSLRLSCAASGFTFAPSAMSWVRQPPGKGLEWVSVIYGDG RTYYADSVKGRFTISRDNSKNTLYLQMNSLRAEDTAVYYCVVGIRLLSESWAQAL WGQGTLVTVSS |
| 1 | huBH56-3>HCDR1 | SEQ ID NO:1 |
| 2 | huBH56-3>HCDR2 | SEQ ID NO:2 |
| 3 | huBH56-3>HCDR3 | SEQ ID NO:3 |
| 12 | huBH56-3>VH | EVQVVESGGGLIQPGGSLRLSCAASGFTFAPSAMSWARQPPGKEVEWVSGIYGDG RTTYADSVKGRFTISRDNSKNTVYLQMNSLRAEDTAVYYCVVGIRLLSESWAQAL RGQGTQVTVSS |
| 1 | huBH56-4>HCDR1 | SEQ ID NO:1 |
| 13 | huBH56-4>HCDR2 | GIYGDGRTYYADSVKG |
| 3 | huBH56-4>HCDR3 | SEQ ID NO:3 |
| 14 | huBH56-4>VH | EVQLVESGGGLIQPGGSLRLSCAASGFTFAPSAMSWARQPPGKEVEWVSGIYGDG RTYYADSVKGRFTISRDNSKNTVYLQMNSLRAEDTAVYYCVVGIRLLSESWAQAL RGQGTQVTVSS |
| 1 | huBH56-5>HCDR1 | SEQ ID NO:1 |
| 2 | huBH56-5>HCDR2 | SEQ ID NO:2 |
| 3 | huBH56-5>HCDR3 | SEQ ID NO:3 |
| 15 | huBH56-5>VH | EVQVVESGGGLIQPGGSLRLSCAASGFTFAPSAMSWARQPPGKEVEWVSGIYGDG RTTYADSVKGRFTISRDNSKNTVYLQMNSLRAEDTAVYICVVGIRLLSESWAQALR GQGTQVTVSS |
| 1 | huBH56-6>HCDR1 | SEQ ID NO:1 |
| 13 | huBH56-6>HCDR2 | SEQ ID NO:13 |
| 3 | huBH56-6>HCDR3 | SEQ ID NO:3 |
| 16 | huBH56-6>VH | EVQLVESGGGLIQPGGSLRLSCAASGFTFAPSAMSWARQPPGKGLEWVSGIYGDG RTYYADSVKGRFTISRDNSKNTVYLQMNSLRAEDTAVYYCVVGIRLLSESWAQAL RGQGTQVTVSS |
| 1 | huHA68-1>HCDR1 | SEQ ID NO:1 |
| 17 | huHA68-1>HCDR2 | VIYGDGKAYYADSVKG |
| 7 | huHA68-1>HCDR3 | SEQ ID NO:7 |
| 18 | huHA68-1>VH | EVQLVETGGGLIQPGGSLRLSCAASGFTFSPSAMSWVRQAPGKGLEWVSVIYGDG KAYYADSVKGRFTISRDNSKNTLYLQMNSLRAEDTAVYYCAVGIRPTDAPWAASL WGQGTLVTVSS |
| 1 | huHA68-2>HCDR1 | SEQ ID NO:1 |
| 17 | huHA68-2>HCDR2 | SEQ ID NO:17 |
| 7 | huHA68-2>HCDR3 | SEQ ID NO:7 |
| 21 | huHA68-2>VH | EVQLVETGGGLIQPGGSLRLSCAASGFTFSPSAMSWARQAPGKGLEWVSVIYGDG KAYYADSVKGRFTISRDNSKNTLYLQMNSLRAEDTAVYYCAVGIRPTDAPWAASL RGQGTLVTVSS |
| 1 | huHA68-3>HCDR1 | SEQ ID NO:1 |
| 6 | huHA68-3>HCDR2 | SEQ ID NO:6 |
| 7 | huHA68-3>HCDR3 | SEQ ID NO:7 |
| 22 | huHA68-3>VH | EVQLVETGGGLIQPGGSLRLSCAASGFTFSPSAMSWARQAPGKGLEWVSGIYGDG KAEYADSVKGRFTISRDNSKNTVYLQMNSLRAEDTAVYYCAVGIRPTDAPWAASL RGQGTLVTVSS |
| 1 | huHA68-4>HCDR1 | SEQ ID NO:1 |
| 6 | huHA68-4>HCDR2 | SEQ ID NO:6 |
| 7 | huHA68-4>HCDR3 | SEQ ID NO:7 |
| 23 | huHA68-4>VH | EVQLVETGGGLIQPGGSLRLSCAASGFTFSPSAMSWARQAPGKGLEWVSGIYGDG KAEYADSVKGRFTISRDNSKNTVYLQMNSLRAEDTAVYSCAVGIRPTDAPWAASL RGQGTLVTVSS |
| 24 | 8H5>HCDR1 | ASVMS |
| 25 | 8H5>HCDR2 | SMLNSNTNYADSVKG |
| 26 | 8H5>HCDR3 | ERSGVY |
| 27 | 8H5>VH | EVQLLESGGGLVQPGGSLRLSCAASGIFLSASVMSWYRQAPGKQRELVASMLNSN TNYADSVKGRFTISRDNSKNTMYLQMNSLRAEDTAVYYCNAERSGVYWGQGTQ VTVSS |
| 28 | CD3>HCDR1 | TYAMN |
| 29 | CD3>HCDR2 | RIRSKYNNYATYYADSVKD |
| 30 | CD3>HCDR3 | HGNFGNSYVSWFAY |
| 31 | CD3>LCDR1 | RSSTGAVTTSNYAN |
| 32 | CD3>LCDR2 | GTNKRAP |
| 33 | CD3>LCDR3 | ALWYSNLWV |
| 34 | CD3>VH | EVQLVESGGGLVQPGGSLKLSCAASGFTFSTYAMNWVRQAPGKGLEWVGRIRSK YNNYATYYADSVKDRFTISRDDSKNTLYLQMNSLKTEDTAVYYCTRHGNFGNSYV SWFAYWGQGTTVTVSS |
| 35 | CD3>VL | QAVVTQEPSLTVSPGGTVTLTCRSSTGAVTTSNYANWVQQKPGQAPRGLIGGTNK RAPGVPARFSGSLLGGKAALTLSGAQPEDEAEYFCALWYSNLWVFGGGTKLTVLG |
| 36 | 29H6>HCDR1 | THYMY |
| 37 | 29H6>HCDR2 | GINPSNEATNFNEKFKT |
| 38 | 29H6>HCDR3 | VGGLSYTMDY |
| 39 | 29H6>LCDR1 | KSSQSLLYSSNQKNYLA |
| 40 | 29H6>LCDR2 | WASTRES |
| 41 | 29H6>LCDR3 | QQYYSYPRT |
| 42 | 29H6>VH | QVQLVQSGAEVKKPGASVKVSCKASGYTFTTHYMYWVRQAPGQGLEWIGGINPS NEATNFNEKFKTKATLTVDKSTSTAYMELSSLRSEDTAVYYCTRVGGLSYTMDYW GQGTLVTVSS |
| 43 | 29H6>VL | DIVMTQSPDSLAVSLGERVTMNCKSSQSLLYSSNQKNYLAWYQQKPGQPPKLLIY WASTRESGVPDRFSGSGYGTDFTLTISSLQAEDVAVYYCQQYYSYPRTFGGGTKLE IK |
| 44 | BH56-IgG (FC with mutations (L234A, | EVQVVESGGGLVQAGESLTLSCAASGFTFAPSAMSWARQPPGKEVEWVSGIYGDG RTTYADSVKGRFTISRDNAKKTVTLQMNNLEPEDSGNYICVVGIRLLSESWAQALR |
| | L235A, D265A, and P329A)) > HC | GQGTQVTVSSEPKSSDKTHTCPPCPAPEAAGGPSVFLFPPKPKDTLMISRTPEVTCV VVAVSHEDPEVKFNWYVDGVEVHNAKTKPREEQYNSTYRVVSVLTVLHQDWLN GKEYKCKVSNKALAAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSV MHEALHNHYTQKSLSLSPGK |
| 45 | BH68-IgG (FC with mutations (L234A, L235A, D265A, and P329A)) > HC | QVQLVETGGGLVQPGGSLRLSCAASGFTFSPSAMNWARQAPGKGLEWVAGIYGD GKAEYADSVKGRFTISRDNAKNMVYLQMVNLRPEDTAVYSCAVGIRPTDAPWAA SLRGQGTQVTVSSEPKSSDKTHTCPPCPAPEAAGGPSVFLFPPKPKDTLMISRTPEV TCVVVAVSHEDPEVKFNWYVDGVEVHNAKTKPREEQYNSTYRVVSVLTVLHQD WLNGKEYKCKVSNKALAAPIEKTISKAKGQPREPQVYTLPPSRDELTKNQVSLTCL VKGFYPSDIAVEWESNGQPENNYKTTPPVLDSDGSFFLYSKLTVDKSRWQQGNVF SCSVMHEALHNHYTQKSLSLSPGK |
| 46 | huBH56-1-IgG (FC with mutations (L234A, L235A, D265A, and P329A)) > HC | EVQLVESGGGLIQPGGSLRLSCAASGFTFAPSAMSWVRQPPGKGLEWVSVIYGDG RTYYADSVKGRFTISRDNSKNTLYLQMNSLRAEDTAVYYCVVGIRLLSESWAQAL WGQGTLVTVSSEPKSSDKTHTCPPCPAPEAAGGPSVFLFPPKPKDTLMISRTPEVTC VVVAVSHEDPEVKFNWYVDGVEVHNAKTKPREEQYNSTYRVVSVLTVLHQDWL NGKEYKCKVSNKALAAPIEKTISKAKGQPREPQVYTLPPSRDELTKNQVSLTCLVK GFYPSDIAVEWESNGQPENNYKTTPPVLDSDGSFFLYSKLTVDKSRWQQGNVFSCS VMHEALHNHYTQKSLSLSPGK |
| 48 | huBH56-3-IgG (FC with mutations (L234A, L235A, D265A, and P329A)) > HC | EVQVVESGGGLIQPGGSLRLSCAASGFTFAPSAMSWARQPPGKEVEWVSGIYGDG RTTYADSVKGRFTISRDNSKNTVYLQMNSLRAEDTAVYYCVVGIRLLSESWAQAL RGQGTQVTVSSEPKSSDKTHTCPPCPAPEAAGGPSVFLFPPKPKDTLMISRTPEVTC VVVAVSHEDPEVKFNWYVDGVEVHNAKTKPREEQYNSTYRVVSVLTVLHQDWL NGKEYKCKVSNKALAAPIEKTISKAKGQPREPQVYTLPPSRDELTKNQVSLTCLVK GFYPSDIAVEWESNGQPENNYKTTPPVLDSDGSFFLYSKLTVDKSRWQQGNVFSCS VMHEALHNHYTQKSLSLSPGK |
| 49 | huBH56-4-IgG (with mutations (L234A, L235A, D265A, and P329A)) > HC | EVQLVESGGGLIQPGGSLRLSCAASGFTFAPSAMSWARQPPGKEVEWVSGIYGDG RTYYADSVKGRFTISRDNSKNTVYLQMNSLRAEDTAVYYCVVGIRLLSESWAQAL RGQGTQVTVSSEPKSSDKTHTCPPCPAPEAAGGPSVFLFPPKPKDTLMISRTPEVTC VVVAVSHEDPEVKFNWYVDGVEVHNAKTKPREEQYNSTYRVVSVLTVLHQDWL NGKEYKCKVSNKALAAPIEKTISKAKGQPREPQVYTLPPSRDELTKNQVSLTCLVK GFYPSDIAVEWESNGQPENNYKTTPPVLDSDGSFFLYSKLTVDKSRWQQGNVFSCS VMHEALHNHYTQKSLSLSPGK |
| 50 | huBH56-5-IgG (FC with mutations (L234A, L235A, D265A, and P329A)) > HC | EVQVVESGGGLIQPGGSLRLSCAASGFTFAPSAMSWARQPPGKEVEWVSGIYGDG RTTYADSVKGRFTISRDNSKNTVYLQMNSLRAEDTAVYICVVGIRLLSESWAQALR GQGTQVTVSSEPKSSDKTHTCPPCPAPEAAGGPSVFLFPPKPKDTLMISRTPEVTCV VVAVSHEDPEVKFNWYVDGVEVHNAKTKPREEQYNSTYRVVSVLTVLHQDWLN GKEYKCKVSNKALAAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSV MHEALHNHYTQKSLSLSPGK |
| 51 | huBH56-6-IgG (FC with mutations (L234A, L235A, D265A, and P329A)) > HC | EVQLVESGGGLIQPGGSLRLSCAASGFTFAPSAMSWARQPPGKGLEWVSGIYGDG RTYYADSVKGRFTISRDNSKNTVYLQMNSLRAEDTAVYYCVVGIRLLSESWAQAL RGQGTQVTVSSEPKSSDKTHTCPPCPAPEAAGGPSVFLFPPKPKDTLMISRTPEVTC VVVAVSHEDPEVKFNWYVDGVEVHNAKTKPREEQYNSTYRVVSVLTVLHQDWL NGKEYKCKVSNKALAAPIEKTISKAKGQPREPQVYTLPPSRDELTKNQVSLTCLVK GFYPSDIAVEWESNGQPENNYKTTPPVLDSDGSFFLYSKLTVDKSRWQQGNVFSCS VMHEALHNHYTQKSLSLSPGK |
| 52 | huHA68-1-IgG (FC with mutations (L234A, L235A, D265A, and P329A)) > HC | EVQLVETGGGLIQPGGSLRLSCAASGFTFSPSAMSWVRQAPGKGLEWVSVIYGDG KAYYADSVKGRFTISRDNSKNTLYLQMNSLRAEDTAVYYCAVGIRPTDAPWAASL WGQGTLVTVSSEPKSSDKTHTCPPCPAPEAAGGPSVFLFPPKPKDTLMISRTPEVTC VVVAVSHEDPEVKFNWYVDGVEVHNAKTKPREEQYNSTYRVVSVLTVLHQDWL NGKEYKCKVSNKALAAPIEKTISKAKGQPREPQVYTLPPSRDELTKNQVSLTCLVK GFYPSDIAVEWESNGQPENNYKTTPPVLDSDGSFFLYSKLTVDKSRWQQGNVFSCS VMHEALHNHYTQKSLSLSPGK |
| 53 | huHA68-2-IgG (FC with mutations (L234A, L235A, D265A, and P329A)) > HC | EVQLVETGGGLIQPGGSLRLSCAASGFTFSPSAMSWARQAPGKGLEWVSVIYGDG KAYYADSVKGRFTISRDNSKNTLYLQMNSLRAEDTAVYYCAVGIRPTDAPWAASL RGQGTLVTVSSEPKSSDKTHTCPPCPAPEAAGGPSVFLFPPKPKDTLMISRTPEVTC VVVAVSHEDPEVKFNWYVDGVEVHNAKTKPREEQYNSTYRVVSVLTVLHQDWL NGKEYKCKVSNKALAAPIEKTISKAKGQPREPQVYTLPPSRDELTKNQVSLTCLVK GFYPSDIAVEWESNGQPENNYKTTPPVLDSDGSFFLYSKLTVDKSRWQQGNVFSCS VMHEALHNHYTQKSLSLSPGK |
| 54 | huHA68-3-IgG (FC with mutations (L234A, L235A, D265A, and P329A)) > HC | EVQLVETGGGLIQPGGSLRLSCAASGFTFSPSAMSWARQAPGKGLEWVSGIYGDG KAEYADSVKGRFTISRDNSKNTVYLQMNSLRAEDTAVYYCAVGIRPTDAPWAASL RGQGTLVTVSSEPKSSDKTHTCPPCPAPEAAGGPSVFLFPPKPKDTLMISRTPEVTC VVVAVSHEDPEVKFNWYVDGVEVHNAKTKPREEQYNSTYRVVSVLTVLHQDWL NGKEYKCKVSNKALAAPIEKTISKAKGQPREPQVYTLPPSRDELTKNQVSLTCLVK |
| | | GFYPSDIAVEWESNGQPENNYKTTPPVLDSDGSFFLYSKLTVDKSRWQQGNVFSCS VMHEALHNHYTQKSLSLSPGK |
| 55 | huHA68-4-IgG (FC with mutations (L234A, L235A, D265A, and P329A)) > HC | EVQLVETGGGLIQPGGSLRLSCAASGFTFSPSAMSWARQAPGKGLEWVSGIYGDG KAEYADSVKGRFTISRDNSKNTVYLQMNSLRAEDTAVYSCAVGIRPTDAPWAASL RGQGTLVTVSSEPKSSDKTHTCPPCPAPEAAGGPSVFLFPPKPKDTLMISRTPEVTC VVVAVSHEDPEVKFNWYVDGVEVHNAKTKPREEQYNSTYRVVSVLTVLHQDWL NGKEYKCKVSNKALAAPIEKTISKAKGQPREPQVYTLPPSRDELTKNQVSLTCLVK GFYPSDIAVEWESNGQPENNYKTTPPVLDSDGSFFLYSKLTVDKSRWQQGNVFSCS VMHEALHNHYTQKSLSLSPGK |
| 56 | huBH56-3-hole-IgG1-mut (hole) (with mutations (L234A, L235A, D265A, P329A, Y407V, T366S, L368A, and Y349C)) > HC | EVQVVESGGGLIQPGGSLRLSCAASGFTFAPSAMSWARQPPGKEVEWVSGIYGDG RTTYADSVKGRFTISRDNSKNTVYLQMNSLRAEDTAVYYCVVGIRLLSESWAQAL RGQGTQVTVSSEPKSSDKTHTCPPCPAPEAAGGPSVFLFPPKPKDTLMISRTPEVTC VVVAVSHEDPEVKFNWYVDGVEVHNAKTKPREEQYNSTYRVVSVLTVLHQDWL NGKEYKCKVSNKALAAPIEKTISKAKGQPREPQVCTLPPSRDELTKNQVSLSCAVK GFYPSDIAVEWESNGQPENNYKTTPPVLDSDGSFFLVSKLTVDKSRWQQGNVFSCS VMHEALHNHYTQKSLSLSPGK |
| 57 | CD3ScFv-knob-IgG1-mut (knob) (with mutations (L234A, L235A, D265A, P329A, S354C, and T366W)) > HC | EVQLVESGGGLVQPGGSLKLSCAASGFTFSTYAMNWVRQAPGKGLEWVGRIRSK YNNYATYYADSVKDRFTISRDDSKNTLYLQMNSLKTEDTAVYYCTRHGNFGNSYV SWFAYWGQGTTVTVSSGGGGSGGGGSGGGGSQAVVTQEPSLTVSPGGTVTLTCRS STGAVTTSNYANWVQQKPGQAPRGLIGGTNKRAPGVPARFSGSLLGGKAALTLSG AQPEDEAEYFCALWYSNLWVFGGGTKLTVLGEPKSSDKTHTCPPCPAPEAAGGPS VFLFPPKPKDTLMISRTPEVTCVVVAVSHEDPEVKFNWYVDGVEVHNAKTKPREE QYNSTYRVVSVLTVLHQDWLNGKEYKCKVSNKALAAPIEKTISKAKGQPREPQV YTLPPCRDELTKNQVSLWCLVKGFYPSDIAVEWESNGQPENNYKTTPPVLDSDGSF FLYSKLTVDKSRWQQGNVFSCSVMHEALHNHYTQKSLSLSPGK |
| 58 | hu8H5-huBH56-3-hole-IgG1-mut (hole) (with mutations (L234A, L235A, D265A, P329A, Y407V, T366S, L368A, Y349C)) > HC | EVQLLESGGGLVQPGGSLRLSCAASGIFLSASVMSWYRQAPGKQRELVASMLNSN TNYADSVKGRFTISRDNSKNTMYLQMNSLRAEDTAVYYCNAERSGVYWGQGTQ VTVSSGGSGGEVQVVESGGGLIQPGGSLRLSCAASGFTFAPSAMSWARQPPGKEV EWVSGIYGDGRTTYADSVKGRFTISRDNSKNTVYLQMNSLRAEDTAVYYCVVGIR LLSESWAQALRGQGTQVTVSSEPKSSDKTHTCPPCPAPEAAGGPSVFLFPPKPKDT LMISRTPEVTCVVVAVSHEDPEVKFNWYVDGVEVHNAKTKPREEQYNSTYRVVS VLTVLHQDWLNGKEYKCKVSNKALAAPIEKTISKAKGQPREPQVCTLPPSRDELT KNQVSLSCAVKGFYPSDIAVEWESNGQPENNYKTTPPVLDSDGSFFLVSKLTVDKS RWQQGNVFSCSVMHEALHNHYTQKSLSLSPGK |
| 59 | CD3-Fab-HC-knob-IgG1-mut (knob) (with mutations (L234A, L235A, D265A, P329A, S354C, and T366W)) > HC | EVQLVESGGGLVQPGGSLKLSCAASGFTFSTYAMNWVRQAPGKGLEWVGRIRSK YNNYATYYADSVKDRFTISRDDSKNTLYLQMNSLKTEDTAVYYCTRHGNFGNSYV SWFAYWGQGTTVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTCVVVAVSHED PEVKFNWYVDGVEVHNAKTKPREEQYNSTYRVVSVLTVLHQDWLNGKEYKCKV SNKALAAPIEKTISKAKGQPREPQVYTLPPCRDELTKNQVSLWCLVKGFYPSDIAVE WESNGQPENNYKTTPPVLDSDGSFFLYSKLTVDKSRWQQGNVFSCSVMHEALHN HYTQKSLSLSPGK |
| 60 | CD3Fab>LC | QAVVTQEPSLTVSPGGTVTLTCRSSTGAVTTSNYANWVQQKPGQAPRGLIGGTNK RAPGVPARFSGSLLGGKAALTLSGAQPEDEAEYFCALWYSNLWVFGGGTKLTVLG QPKAAPSVTLFPPSSEELQANKATLVCLISDFYPGAVTVAWKADSSPVKAGVETTTP SKQSNNKYAASSYLSLTPEQWKSHRSYSCQVTHEGSTVEKTVAPTEC |
| 61 | 29H6-CD3ScFv-knob-IgG1-mut (knob) (with mutations (L234A, L235A, D265A, P329A, S354C, and T366W)) > HC | QVQLVQSGAEVKKPGASVKVSCKASGYTFTTHYMYWVRQAPGQGLEWIGGINPS NEATNFNEKFKTKATLTVDKSTSTAYMELSSLRSEDTAVYYCTRVGGLSYTMDYW GQGTLVTVSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKVEPKSC GGGGSEVQLVESGGGLVQPGGSLKLSCAASGFTFSTYAMNWVRQAPGKGLEWVG RIRSKYNNYATYYADSVKDRFTISRDDSKNTLYLQMNSLKTEDTAVYYCTRHGNF GNSYVSWFAYWGQGTTVTVSSGGGGSGGGGSGGGGSQAVVTQEPSLTVSPGGTV TLTCRSSTGAVTTSNYANWVQQKPGQAPRGLIGGTNKRAPGVPARFSGSLLGGKA ALTLSGAQPEDEAEYFCALWYSNLWVFGGGTKLTVLGEPKSSDKTHTCPPCPAPE AAGGPSVFLFPPKPKDTLMISRTPEVTCVVVAVSHEDPEVKFNWYVDGVEVHNAK TKPREEQYNSTYRVVSVLTVLHQDWLNGKEYKCKVSNKALAAPIEKTISKAKGQP REPQVYTLPPCRDELTKNQVSLWCLVKGFYPSDIAVEWESNGQPENNYKTTPPVL DSDGSFFLYSKLTVDKSRWQQGNVFSCSVMHEALHNHYTQKSLSLSPGK |
| 62 | 29H6Fab>LC | DIVMTQSPDSLAVSLGERVTMNCKSSQSLLYSSNQKNYLAWYQQKPGQPPKLLIY WASTRESGVPDRFSGSGYGTDFTLTISSLQAEDVAVYYCQQYYSYPRTFGGGTKLE IKRTVAAPSVFIFPPSDEQLKSGTASVVCLLNNFYPREAKVQWKVDNALQSGNSQE SVTEQDSKDSTYSLSSTLTLSKADYEKHKVYACEVTHQGLSSPVTKSFNRGEC |
| 63 | Teclistamab-analog-BCMA>HC | QLQLQESGPGLVKPSETLSLTCTVSGGSISSGSYFWGWIRQPPGKGLEWIGSIYYSG ITYYNPSLKSRVTISVDTSKNQFSLKLSSVTAADTAVYYCARHDGAVAGLFDYWGQ |
| | | GTLVTVSSASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKRVESKYGPP CPPCPAPEAAGGPSVFLFPPKPKDTLMISRTPEVTCVVVDVSQEDPEVQFNWYVDG VEVHNAKTKPREEQFNSTYRVVSVLTVLHQDWLNGKEYKCKVSNKGLPSSIEKTI SKAKGQPREPQVYTLPPSQEEMTKNQVSLTCLVKGFYPSDIAVEWESNGQPENNY KTTPPVLDSDGSFFLYSRLTVDKSRWQEGNVFSCSVMHEALHNHYTQKSLSLSLG K |
| 64 | Teclistamab-analog-BCMA>LC | SYVLTQPPSVSVAPGQTARITCGGNNIGSKSVHWYQQPPGQAPVVVVYDDSDRPS GIPERFSGSNSGNTATLTISRVEAGDEAVYYCQVWDSSSDHVVFGGGTKLTVLGQP KAAPSVTLFPPSSEELQANKATLVCLISDFYPGAVTVAWKGDSSPVKAGVETTTPSK QSNNKYAASSYLSLTPEQWKSHRSYSCQVTHEGSTVEKTVAPTEC |
| 65 | Teclistamab-analog-CD3>HC | EVQLVESGGGLVQPGGSLRLSCAASGFTFNTYAMNWVRQAPGKGLEWVARIRSK YNNYATYYAASVKGRFTISRDDSKNSLYLQMNSLKTEDTAVYYCARHGNFGNSYV SWFAYWGQGTLVTVSSASTKGPSVFPLAPCSRSTSESTAALGCLVKDYFPEPVTVS WNSGALTSGVHTFPAVLQSSGLYSLSSVVTVPSSSLGTKTYTCNVDHKPSNTKVDK RVESKYGPPCPPCPAPEAAGGPSVFLFPPKPKDTLMISRTPEVTCVVVDVSQEDPEV QFNWYVDGVEVHNAKTKPREEQFNSTYRVVSVLTVLHQDWLNGKEYKCKVSNK GLPSSIEKTISKAKGQPREPQVYTLPPSQEEMTKNQVSLTCLVKGFYPSDIAVEWES NGQPENNYKTTPPVLDSDGSFLLYSKLTVDKSRWQEGNVFSCSVMHEALHNHYT QKSLSLSLGK |
| 66 | Teclistamab-analog-CD3>LC | QTVVTQEPSLTVSPGGTVTLTCRSSTGAVTTSNYANWVQQKPGQAPRGLIGGTNK RAPGTPARFSGSLLGGKAALTLSGVQPEDEAEYYCALWYSNLWVFGGGTKLTVLG QPKAAPSVTLFPPSSEELQANKATLVCLISDFYPGAVTVAWKADSSPVKAGVETTTP SKQSNNKYAASSYLSLTPEQWKSHRSYSCQVTHEGSTVEKTVAPTEC |
| 67 | Talquetamab-GPRC5D>HC GC5B596 HC | QVQLVQSGAEVKKPGASVKVSCKASGYSFTGYTMNWVRQAPGQGLEWMGLINP YNSDTNYAQKLQGRVTMTTDTSTSTAYMELRSLRSDDTAVYYCARVALRVALDY WGQGTLVTVSSASTKGPSVFPLAPCSRSTSESTAALGCLVKDYFPEPVTVSWNSGA LTSGVHTFPAVLQSSGLYSLSSVVTVPSSSLGTKTYTCNVDHKPSNTKVDKRVESK YGPPCPPCPAPEAAGGPSVFLFPPKPKDTLMISRTPEVTCVVVDVSQEDPEVQFNW YVDGVEVHNAKTKPREEQFNSTYRVVSVLTVLHQDWLNGKEYKCKVSNKGLPSS IEKTISKAKGQPREPQVYTLPPSQEEMTKNQVSLTCLVKGFYPSDIAVEWESNGQPE NNYKTTPPVLDSDGSFFLYSRLTVDKSRWQEGNVFSCSVMHEALHNHYTQKSLSL SLGK |
| 68 | Talquetamab - GPRC5D>LC GC5B596LC | DIQMTQSPSSLSASVGDRVTITCKASQNVATHVGWYQQKPGKAPKRLIYSASYRYS GVPSRFSGSGSGTEFTLTISNLQPEDFATYYCQQYNRYPYTFGQGTKLEIKRTVAAP SVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC |
| 69 | BGCB491>HC1 | QVQLQQSGPRLVRPSQTLSLTCAISGDSVFNNNAAWSWIRQSPSRGLEWLGRTYY RSKWLYDYAVSVKSRITVNPDTSRNQFTLQLNSVTPEDTALYYCARGYSSSFDYW GQGTLVTVSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKVEPKSC DKTHTCPPCPAPEAAGGPSVFLFPPKPKDTLMISRTPEVTCVVVSVSHEDPEVKFN WYVDGVEVHNAKTKPREEQYNSTYRVVSVLTVLHQDWLNGKEYKCKVSNKALP APIEKTISKAKGQPREPQVYTLPPSREEMTKNQVSLSCAVKGFYPSDIAVEWESNG QPENNYKTTPPVLDSDGSFFLVSKLTVDKSRWQQGNVFSCSVMHEALHNRFTQKS LSLSPGKGGGGSGGGGSGGGGSGGGGSDIVMTQTPLSSPVTLGQPASISCRSSQSL VHSDGNTYLSWLQQRPGQPPRLLIYKISNRFFGVPDRFSGSGAGTDFTLKISRVEAE DVGVYYCMQATQFPHTFGQGTKLEIKGGSEGKSSGSGSESKSTGGSQVTLKESGP VLVKPTETLTLTCTVSGFSLTNIRMSVSWIRQPPGKALEWLAHIFSNDEKSYSTSLK SRLTISRDTSKSQVVLTLTNVDPVDTATYYCARMRLPYGMDVWGQGTTVTVSS |
| 70 | BGCB491>HC2 | EIVLTQSPGTLSLSPGERATLSCRASQSISSSFLTWYQQKPGQAPRLLIYGASSRATGI PDRFSGGGSGTDFTLTISRLEPEDFAVYYCQHYGSSPMYTFGQGTKLEIKGGSEGKS SGSGSESKSTGGSEVQLLESGGGLVQPGGSLRLSCAASGFTFSSYAMSWVRQAPG KGLEWVSAISGSGGSTYYADSVKGRFTISRDNSKNTLYLQMNSLRAEDTAVYYCA KDEGYSSGHYYGMDVWGQGTTVTVSSEPKSSDKTHTCPPCPAPEAAGGPSVFLFP PKPKDTLMISRTPEVTCVVVSVSHEDPEVKFNWYVDGVEVHNAKTKPREEQYNS TYRVVSVLTVLHQDWLNGKEYKCKVSNKALPAPIEKTISKAKGQPREPQVYTLPPS REEMTKNQVSLWCLVKGFYPSDIAVEWESNGQPENNYKTTPPVLDSDGSFFLYSK LTVDKSRWQQGNVFSCSVMHEALHNHYTQKSLSLSPGK |
| 71 | BGCB491>LC | ALTQPASVSGSPGQSITISCTGTSSNIGTYKFVSWYQQHPDKAPKVLLYEVSKRPSG VSSRFSGSKSGNTASLTISGLQAEDQADYHCVSYAGSGTLLFGGGTKLTVLGQPKA APSVTLFPPSSEELQANKATLVCLISDFYPGAVTVAWKADSSPVKAGVETTTPSKQS NNKYAASSYLSLTPEQWKSHRSYSCQVTHEGSTVEKTVAPTECS |
| 72 | IBI3003>HC1 | QVQLVQSGAEVKKPGASVKVSCKASGYTFIGYVMHWVRQAPGQGLEWMGYINP YNDVSQHQGKFKGRVTMTSDTSTSTVYMELSSLRSEDTAVYYCAREGRLGLRVLA YWGQGTLVTVSSASTKGPSVFPLAPSSKSTSGGTAALGCLVKDYFPEPVTVSWNS |
| | | GALTSGVHTFPAVLQSSGLYSLSSVVTVPSSSLGTQTYICNVNHKPSNTKVDKKVEP KSCDKTHTCPPCPAPEAAGGPSVFLFPPKPKDTLMISRTPEVTCVVVDVSHEDPEV KFNWYVDGVEVHNAKTKPREEQYNSTYRVVSVLTVLHQDWLNGKEYKCKVSNK ALPAPIEKTISKAKGQPREPQVYTLPPSRDELTKNQVSLWCLVKGFYPSDIAVEWES NGQPENNYKTTPPVLDSDGSFFLYSKLTVDKSRWQQGNVFSCSVMHEALHNHYT QKSLSLSPGK |
| 73 | IBI3003>HC2 | EVQLVESGGGLVQPGGSLKLSCAASGFTFNTYAMNWVRQASGKGLEWVGRIRSK YNNYATYYADSVKDRFTISRDDSKSTLYLQMNSLKTEDTAVYYCARHGNFGQSYV SWFAYWGQGTTVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDK KVEPKSSDKTHTCPPCPAPEAAGGPSVFLFPPKPKDTLMISRTPEVTCVVVDVSHE DPEVKFNWYVDGVEVHNAKTKPREEQYNSTYRVVSVLTVLHQDWLNGKEYKCK VSNKALPAPIEKTISKAKGQPREPQVYTLPPSRDELTKNQVSLSCAVKGFYPSDIAV EWESNGQPENNYKTTPPVLDSDGSFFLVSKLTVDKSRWQQGNVFSCSVMHEALH NHYTQKSLSLSPGKGGGGSGGGGSEIVLTQSPATLSLSPGERATLSCRASQSVSSYL AWYQQKPGQAPRLLIYDASKRATGIPARFSGSGSGTDFTLTISSLEPEDFAVYYCQQ ASALPLTFGCGTKVEIKGGGGSGGGGSGGGGSEVQLVESGGGLVQPGRSLRLSCA ASGFTFGDYAIHWVRQAPGKCLEWVSGISWDSSSIAYADSVKGRFTISRDNAKNSL YLQMNSLRAEDTAVYYCAKDSPVRDSFGSIAFDIWGQGTMVTVSS |
| 74 | IBI3003>LC1 | QAVVTQEPSLTVSPGGTVTLTCRSSTGAVTTSNYANWVQQKPGQAPRGLIGGTNK RAPGVPARFSGSLLGDKAALTLLGAQPEDEAEYYCALWYSNLWVFGQGTKLTVL GQPKAAPSVTLFPPSSEELQANKATLVCLISDFYPGAVTVAWKADSSPVKAGVETT TPSKQSNNKYAASSYLSLTPEQWKSHRSYSCQVTHEGSTVEKTVAPTEC |
| 75 | IBI3003>LC2 | DIVMTQSPDSLAVSLGERATINCRASESVDSYGITFMNWYQQKPGQPPKLLIYAAS NQGSGVPDRFSGSGSGTDFTLTISSLQAEDVAVYYCQQSKEVPWTFGQGTKLEIKR TVAAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC |
| 76 | 269B094>HC | DVQLVESGGGLVQPGGSLRLSCAASGRTFSSIVMGWFRQAPGKEREFVGAIMWND GITYLQDSVKGRFTIFRDNAKNTVYLQMNSLKLEDTAVYYCAASKGRYSEYEYW GQGTQVTVSSEPKSSDKTHTCPPCPAPELLGGPSVFLFPPKPKDTLMISRTPEVTCV VVDVSHEDPEVKFNWYVDGVEVHNAKTKPREEQYNSTYRVVSVLTVLHQDWLN GKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSV MHEALHNHYTQKSLSLSPGK |
| 77 | huBCMA-huFc | MLQMAGQCSQNEYFDSLLHACIPCQLRCSSNTPPLTCQRYCNASVTNSVKGTNAE PKSCDKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPSDIAVEWES NGQPENNYKTTPPVLDSDGSFFLYSKLTVDKSRWQQGNVFSCSVMHEALHNHYT QKSLSLSPGK |
| 78 | cynoBCMA-huFc | MLQMARQCSQNEYFDSLLHDCKPCQLRCSSTPPLTCQRYCNASMTNSVKGMNAE PKSCDKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPSDIAVEWES NGQPENNYKTTPPVLDSDGSFFLYSKLTVDKSRWQQGNVFSCSVMHEALHNHYT QKSLSLSPGK |
| 79 | huBCMA-his | MLQMAGQCSQNEYFDSLLHACIPCQLRCSSNTPPLTCQRYCNASVTNSVKGTNAH HHHHH |
| 80 | IgG1 Fc (with hinge region, no mutation, CH2-CH3) | EPKSSDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCKVSN KALPAPIEKTISKAKGQPREPQVYTLPPSRDELTKNQVSLTCLVKGFYPSDIAVEWE SNGQPENNYKTTPPVLDSDGSFFLYSKLTVDKSRWQQGNVFSCSVMHEALHNHYT QKSLSLSPGK |
| 81 | HuGPRC5A | MATTVPDGCRNGLKSKYYRLCDKAEAWGIVLETVATAGVVTSVAFMLTLPILVCK VQDSNRRKMLPTQFLFLLGVLGIFGLTFAFIIGLDGSTGPTRFFLFGILFSICFSCLLA HAVSLTKLVRGRKPLSLLVILGLAVGFSLVQDVIAIEYIVLTMNRTNVNVFSELSAPR RNEDFVLLLTYVLFLMALTFLMSSFTFCGSFTGWKRHGAHIYLTMLLSIAIWVAWI TLLMLPDFDRRWDDTILSSALAANGWVFLLAYVSPEFWLLTKQRNPMDYPVEDA FCKPQLVKKSYGVENRAYSQEEITQGFEETGDTLYAPYSTHFQLQNQPPQKEFSIPR AHAWPSPYKDYEVKKEGS |
| 82 | HuGPRC5B | MFVASERKMRAHQVLTFLLLFVITSVASENASTSRGCGLDLLPQYVSLCDLDAIWG IVVEAVAGAGALITLLLMLILLVRLPFIKEKEKKSPVGLHFLFLLGTLGLFGLTFAFII QEDETICSVRRFLWGVLFALCFSCLLSQAWRVRRLVRHGTGPAGWQLVGLALCLM LVQVIIAVEWLVLTVLRDTRPACAYEPMDFVMALIYDMVLLVVTLGLALFTLCGKF KRWKLNGAFLLITAFLSVLIWVAWMTMYLFGNVKLQQGDAWNDPTLAITLAASG |
| | | WVFVIFHAIPEIHCTLLPALQENTPNYFDTSQPRMRETAFEEDVQLPRAYMENKAF SMDEHNAALRTAGFPNGSLGKRPSGSLGKRPSAPFRSNVYQPTEMAVVLNGGTIPT APPSHTGRHLW |
| 83 | HuGPRC5C | MAIHKALVMCLGLPLFLFPGAWAQGHVPPGCSQGLNPLYYNLCDRSGAWGIVLEA VAGAGIVTTFVLTIILVASLPFVQDTKKRSLLGTQVFFLLGTLGLFCLVFACVVKPDF STCASRRFLFGVLFAICFSCLAAHVFALNFLARKNHGPRGWVIFTVALLLTLVEVIIN TEWLIITLVRGSGEGGPQGNSSAGWAVASPCAIANMDFVMALIYVMLLLLGAFLG AWPALCGRYKRWRKHGVFVLLTTATSVAIWVVWIVMYTYGNKQHNSPTWDDPTL AIALAANAWAFVLFYVIPEVSQVTKSSPEQSYQGDMYPTRGVGYETILKEQKGQS MFVENKAFSMDEPVAAKRPVSPYSGYNGQLLTSVYQPTEMALMHKVPSEGAYDII LPRATANSQVMGSANSTLRAEDMYSAQSHQAATPPKDGKNSQVFRNPYVWD |
| 84 | Multispecific antibody Fc-hole (CH2-CH3 hole) | DKTHTCPPCPAPEAAGGPSVFLFPPKPKDTLMISRTPEVTCVVVAVSHEDPEVKFN WYVDGVEVHNAKTKPREEQYNSTYRVVSVLTVLHQDWLNGKEYKCKVSNKAL AAPIEKTISKAKGQPREPQVCTLPPSRDELTKNQVSLSCAVKGFYPSDIAVEWESNG QPENNYKTTPPVLDSDGSFFLVSKLTVDKSRWQQGNVFSCSVMHEALHNHYTQK SLSLSPGK |
| 85 | Fc-hole (hinge region-CH2-CH3 hole) | EPKSSDKTHTCPPCPAPEAAGGPSVFLFPPKPKDTLMISRTPEVTCVVVAVSHEDPE VKFNWYVDGVEVHNAKTKPREEQYNSTYRVVSVLTVLHQDWLNGKEYKCKVSN KALAAPIEKTISKAKGQPREPQVCTLPPSRDELTKNQVSLSCAVKGFYPSDIAVEWE SNGQPENNYKTTPPVLDSDGSFFLVSKLTVDKSRWQQGNVFSCSVMHEALHNHY TQKSLSLSPGK |
| 86 | Multispecific antibody Fc-knob (hinge region-CH2-CH3 knob) | EPKSCDKTHTCPPCPAPEAAGGPSVFLFPPKPKDTLMISRTPEVTCVVVAVSHEDPE VKFNWYVDGVEVHNAKTKPREEQYNSTYRVVSVLTVLHQDWLNGKEYKCKVSN KALAAPIEKTISKAKGQPREPQVYTLPPCRDELTKNQVSLWCLVKGFYPSDIAVEW ESNGQPENNYKTTPPVLDSDGSFFLYSKLTVDKSRWQQGNVFSCSVMHEALHNHY TQKSLSLSPGK |
| 87 | Multispecific antibody Fc-knob (CH2-CH3 knob) | DKTHTCPPCPAPEAAGGPSVFLFPPKPKDTLMISRTPEVTCVVVAVSHEDPEVKFN WYVDGVEVHNAKTKPREEQYNSTYRVVSVLTVLHQDWLNGKEYKCKVSNKAL AAPIEKTISKAKGQPREPQVYTLPPCRDELTKNQVSLWCLVKGFYPSDIAVEWESN GQPENNYKTTPPVLDSDGSFFLYSKLTVDKSRWQQGNVFSCSVMHEALHNHYTQ KSLSLSPGK |
| 88 | CH1 | ASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKKV |
| 89 | IgG1 constant region (LALA mutation + D265A + P329A) | ASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSCDKTHTCPPC PAPEAAGGPSVFLFPPKPKDTLMISRTPEVTCVVVAVSHEDPEVKFNWYVDGVEV HNAKTKPREEQYNSTYRVVSVLTVLHQDWLNGKEYKCKVSNKALAAPIEKTISK AKGQPREPQVYTLPPSRDELTKNQVSLTCLVKGFYPSDIAVEWESNGQPENNYKTT PPVLDSDGSFFLYSKLTVDKSRWQQGNVFSCSVMHEALHNHYTQKSLSLSPGK (constant region Fc sequence is underlined) |
| 90 | Fc (without hinge region, CH2-CH3 + LALA mutation + D265A + P329A) | DKTHTCPPCPAPEAAGGPSVFLFPPKPKDTLMISRTPEVTCVVVAVSHEDPEVKFN WYVDGVEVHNAKTKPREEQYNSTYRVVSVLTVLHQDWLNGKEYKCKVSNKAL AAPIEKTISKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHNHYTQKS LSLSPGK |
| 91 | Lambda light chain constant region | GQPKAAPSVTLFPPSSEELQANKATLVCLISDFYPGAVTVAWKADSSPVKAGVETT TPSKQSNNKYAASSYLSLTPEQWKSHRSYSCQVTHEGSTVEKTVAPTEC |
| 92 | IgG1 constant region (no mutation) |  (constant region Fc sequence is underlined) |
| 93 | Kappa light chain constant region | |
| 94 | Hinge region | EPKSS |
| 95 | Linker peptide | GGGGSGGGGSGGGGSGGGGS |
| 96 | Hinge region | EPKSC |
| 97 | Fc (with hinge region, CH2-CH3 + LALA mutation + D265A + P329A) | EPKSSDKTHTCPPCPAPEAAGGPSVFLFPPKPKDTLMISRTPEVTCVVVAVSHEDPE VKFNWYVDGVEVHNAKTKPREEQYNSTYRVVSVLTVLHQDWLNGKEYKCKVSN KALAAPIEKTISKAKGQPREPQVYTLPPSRDELTKNQVSLTCLVKGFYPSDIAVEWE SNGQPENNYKTTPPVLDSDGSFFLYSKLTVDKSRWQQGNVFSCSVMHEALHNHYT QKSLSLSPGK |
| 98 | IgG1 Fc (without hinge region, no mutation, CH2-CH3) | DKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALP APIEKTISKAKGQPREPQVYTLPPSRDELTKNQVSLTCLVKGFYPSDIAVEWESNGQ PENNYKTTPPVLDSDGSFFLYSKLTVDKSRWQQGNVFSCSVMHEALHNHYTQKSL SLSPGK |
| 99 | Linker peptide | GGGGS |
| 100 | Linker peptide | GGGGSGGGGSGGGGS |
| 101 | Linker peptide | GGSGG |
| 102 | huHA68-4-hole-IgG1-mut (hole) (with mutations (L234A, L235A, D265A, P329A, Y407V, T366S, L368A, and Y349C)) > HC | EVQLVETGGGLIQPGGSLRLSCAASGFTFSPSAMSWARQAPGKGLEWVSGIYGDG KAEYADSVKGRFTISRDNSKNTVYLQMNSLRAEDTAVYSCAVGIRPTDAPWAASL RGQGTLVTVSSEPKSSDKTHTCPPCPAPEAAGGPSVFLFPPKPKDTLMISRTPEVTC VVVAVSHEDPEVKFNWYVDGVEVHNAKTKPREEQYNSTYRVVSVLTVLHQDWL NGKEYKCKVSNKALAAPIEKTISKAKGQPREPQVCTLPPSRDELTKNQVSLSCAVK GFYPSDIAVEWESNGQPENNYKTTPPVLDSDGSFFLVSKLTVDKSRWQQGNVFSCS VMHEALHNHYTQKSLSLSPGK |
| 103 | 2×huHA68-4-hole-IgG1-mut (hole) (with mutations (L234A, L235A, D265A, P329A, Y407V, T366S, L368A, and Y349C)) > HC | EVQLVETGGGLIQPGGSLRLSCAASGFTFSPSAMSWARQAPGKGLEWVSGIYGDG KAEYADSVKGRFTISRDNSKNTVYLQMNSLRAEDTAVYSCAVGIRPTDAPWAASL RGQGTLVTVSSGGGGSEVQLVETGGGLIQPGGSLRLSCAASGFTFSPSAMSWARQ APGKGLEWVSGIYGDGKAEYADSVKGRFTISRDNSKNTVYLQMNSLRAEDTAVY SCAVGIRPTDAPWAASLRGQGTLVTVSSEPKSSDKTHTCPPCPAPEAAGGPSVFLFP PKPKDTLMISRTPEVTCVVVAVSHEDPEVKFNWYVDGVEVHNAKTKPREEQYNST YRVVSVLTVLHQDWLNGKEYKCKVSNKALAAPIEKTISKAKGQPREPQVCTLPPS RDELTKNQVSLSCAVKGFYPSDIAVEWESNGQPENNYKTTPPVLDSDGSFFLVSKL TVDKSRWQQGNVFSCSVMHEALHNHYTQKSLSLSPGK |
| 104 | 2×huBH56-3-hole-IgG1-mut (hole) (with mutations (L234A, L235A, D265A, P329A, Y407V, T366S, L368A, and Y349C)) > HC | EVQVVESGGGLIQPGGSLRLSCAASGFTFAPSAMSWARQPPGKEVEWVSGIYGDG RTTYADSVKGRFTISRDNSKNTVYLQMNSLRAEDTAVYYCVVGIRLLSESWAQAL RGQGTQVTVSSGGGGSEVQVVESGGGLIQPGGSLRLSCAASGFTFAPSAMSWARQ PPGKEVEWVSGIYGDGRTTYADSVKGRFTISRDNSKNTVYLQMNSLRAEDTAVYY CVVGIRLLSESWAQALRGQGTQVTVSSEPKSSDKTHTCPPCPAPEAAGGPSVFLFPP KPKDTLMISRTPEVTCVVVAVSHEDPEVKFNWYVDGVEVHNAKTKPREEQYNST YRVVSVLTVLHQDWLNGKEYKCKVSNKALAAPIEKTISKAKGQPREPQVCTLPPS RDELTKNQVSLSCAVKGFYPSDIAVEWESNGQPENNYKTTPPVLDSDGSFFLVSKL TVDKSRWQQGNVFSCSVMHEALHNHYTQKSLSLSPGK |
| 105 | Humanized anti-GPRC5D VHH 8H5-Fc (humanized anti-GPRC5D HC) | EVQLLESGGGLVQPGGSLRLSCAASGIFLSASVMSWYRQAPGKQRELVASMLNSN TNYADSVKGRFTISRDNSKNTMYLQMNSLRAEDTAVYYCNAERSGVYWGQGTQ VTVSSEPKSSDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEYK CKVSNKALPAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCSVMHEAL HNHYTQKSLSLSPGK |
| 106 | Camelid-derived anti-GPRC5D VHH 8 | EVQLQESGGGLVQAGGSLRLSCAASGIFLSASVMRWYRRAPGKQRELVASMLNSN TNYADSVKGRFTISRDNTKNTMYLQMNSLKPEDTAVYYCNAERSGVYWGQGTQ VTVSS |
| 107 | Camelid-derived anti-GPRC5D VHH8-Fc (camelid-derived anti-GPRC5D HC) | EVQLQESGGGLVQAGGSLRLSCAASGIFLSASVMRWYRRAPGKQRELVASMLNSN TNYADSVKGRFTISRDNTKNTMYLQMNSLKPEDTAVYYCNAERSGVYWGQGTQ VTVSSEPKSSDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEYK CKVSNKALPAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCSVMHEAL HNHYTQKSLSLSPGK |
| 108 | Human GPRC5D protein | MYKDCIESTGDYFLLCDAEGPWGIILESLAILGIVVTILLLLAFLFLMRKIQDCSQW NVLPTQLLFLLSVLGLFGLAFAFIIELNQQTAPVRYFLFGVLFALCFSCLLAHASNLV KLVRGCVSFSWTTILCIAIGCSLLQIIIATEYVTLIMTRGMMFVNMTPCQLNVDFVV LLVYVLFLMALTFFVSKATFCGPCENWKQHGRLIFITVLFSIIIWVVWISMLLRGNP QFQRQPQWDDPVVCIALVTNAWVFLLLYIVPELCILYRSCRQECPLQGNACPVTAY QHSFQVENQELSRARDSDGAEEDVALTSYGTPIQPQTVDPTQECFIPQAKLSPQQD AGGV |
| 109 | CD3-ScFv | EVQLVESGGGLVQPGGSLKLSCAASGFTFSTYAMNWVRQAPGKGLEWVGRIRSK YNNYATYYADSVKDRFTISRDDSKNTLYLQMNSLKTEDTAVYYCTRHGNFGNSYV SWFAYWGQGTTVTVSSGGGGSGGGGSGGGGSQAVVTQEPSLTVSPGGTVTLTCRS STGAVTTSNYANWVQQKPGQAPRGLIGGTNKRAPGVPARFSGSLLGGKAALTLSG |
| | | AQPEDEAEYFCALWYSNLWVFGGGTKLTVLG |
| 110 | 29H6-Fab>HC | QVQLVQSGAEVKKPGASVKVSCKASGYTFTTHYMYWVRQAPGQGLEWIGGINPS NEATNFNEKFKTKATLTVDKSTSTAYMELSSLRSEDTAVYYCTRVGGLSYTMDYW GQGTLVTVSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKVEPKSC |
| 11 | CD3-Fab>HC | EVQLVESGGGLVQPGGSLKLSCAASGFTFSTYAMNWVRQAPGKGLEWVGRIRSK YNNYATYYADSVKDRFTISRDDSKNTLYLQMNSLKTEDTAVYYCTRHGNFGNSYV SWFAYWGQGTTVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDK KVEPKSC |
| 47 | Multispecific antibody Fc-knob (hinge region-CH2-CH3 knob) (with mutations (L234A, L235A, D265A, P329A, S354C, and T366W) | EPKSSDKTHTCPPCPAPEAAGGPSVFLFPPKPKDTLMISRTPEVTCVVVAVSHEDPE VKFNWYVDGVEVHNAKTKPREEQYNSTYRVVSVLTVLHQDWLNGKEYKCKVSN KALAAPIEKTISKAKGQPREPQVYTLPPCRDELTKNQVSLWCLVKGFYPSDIAVEW ESNGQPENNYKTTPPVLDSDGSFFLYSKLTVDKSRWQQGNVFSCSVMHEALHNHY TQKSLSLSPGK |
| 111 | Multispecific antibody Fc-hole (hinge region-CH2-CH3 hole) (with mutations (L234A, L235A, D265A, P329A, Y407V, T366S, L368A, and Y349C)) | EPKSCDKTHTCPPCPAPEAAGGPSVFLFPPKPKDTLMISRTPEVTCVVVAVSHEDPE VKFNWYVDGVEVHNAKTKPREEQYNSTYRVVSVLTVLHQDWLNGKEYKCKVSN KALAAPIEKTISKAKGQPREPQVCTLPPSRDELTKNQVSLSCAVKGFYPSDIAVEWE SNGQPENNYKTTPPVLDSDGSFFLVSKLTVDKSRWQQGNVFSCSVMHEALHNHY TQKSLSLSPGK |

## Claims

1. A VHH antibody specifically binding to BCMA, comprising:
three complementarity determining regions (CDRs) contained in a VHH set forth in any one of SEQ ID NOs: 4, 8, 10, 12, 14, 15, 16, 18, 21, 22, and 23, wherein
preferably, sequences of the CDRs are defined according to IMGT.

2. The VHH antibody according to claim 1, comprising complementarity determining regions (CDRs) VHH CDR1, VHH CDR2, and VHH CDR3, wherein
(i) the VHH CDR1 comprises or consists of the amino acid sequence set forth in SEQ ID NO: 1, the VHH CDR2 comprises or consists of an amino acid sequence set forth in SEQ ID NO: 2, 9, or 13, and the VHH CDR3 comprises or consists of the amino acid sequence set forth in SEQ ID NO: 3;
(ii) the VHH CDR1 comprises or consists of an amino acid sequence set forth in SEQ ID NO: 1 or 5, the VHH CDR2 comprises or consists of the amino acid sequence set forth in SEQ ID NO: 6, and the VHH CDR3 comprises or consists of the amino acid sequence set forth in SEQ ID NO: 7; or
(iii) the VHH CDR1 comprises or consists of the amino acid sequence set forth in SEQ ID NO: 1, the VHH CDR2 comprises or consists of the amino acid sequence set forth in SEQ ID NO: 17, and the VHH CDR3 comprises or consists of the amino acid sequence set forth in SEQ ID NO: 7.

3. The VHH antibody according to claim 1, comprising or consisting of a heavy chain variable region, wherein the heavy chain variable region
(i) comprises or consists of an amino acid sequence having at least 90%, 91%, 92%, 93%, 94%, 95%, 96%, 97%, 98%, or 99% identity to an amino acid sequence selected from any one of SEQ ID NOs: 4, 8, 10, 12, 14, 15, 16, 18, 21, 22, and 23;
(ii) comprises or consists of an amino acid sequence selected from any one of SEQ ID NOs: 4, 8, 10, 12, 14, 15, 16, 18, 21, 22, and 23; or
(iii) comprises an amino acid sequence having one or more (preferably not more than 10, and more preferably not more than 5, 4, 3, 2, or 1) amino acid modifications (preferably amino acid substitutions, and more preferably conservative amino acid substitutions) compared to an amino acid sequence selected from any one of SEQ ID NOs: 4, 8, 10, 12, 14, 15, 16, 18, 21, 22, and 23, wherein preferably, the amino acid modifications do not occur in the CDRs.

4. A heavy-chain antibody specifically binding to BCMA, comprising the VHH antibody according to any one of claims 1-3.

5. The heavy-chain antibody according to claim 4, comprising the VHH antibody according to any one of claims 1-3 linked to an antibody constant region or an Fc region, wherein preferably, the antibody constant region or the Fc region is from human IgG1, human IgG2, human IgG3, or human IgG4, optionally, the VHH antibody is linked to the Fc region by a hinge region or a portion thereof, and optionally, an amino acid sequence of the hinge region portion is EPKSS (SEQ ID NO: 94) or EPKSC (SEQ ID NO: 96).

6. The heavy-chain antibody according to claim 4, comprising the VHH antibody according to any one of claims 1-3 linked to an antibody Fc region, wherein the Fc region is an Fc region from human IgG1, IgG2, IgG3, or IgG4, optionally the Fc region comprises an L234A/L235A mutation, a D265A mutation and a P329A mutation, and preferably the Fc region
(i) comprises or consists of an amino acid sequence having at least 85%, 90%, 91%, 92%, 93%, 94%, 95%, 96%, 97%, 98%, or 99% identity to an amino acid sequence set forth in SEQ ID NO: 80, 90, 97, or 98;
(ii) comprises or consists of an amino acid sequence set forth in SEQ ID NO: 80, 90, 97, or 98; or
(iii) comprises an amino acid sequence having one or more (preferably not more than 10, and more preferably not more than 5, 4, 3, 2, or 1) amino acid modifications (preferably amino acid substitutions, and more preferably conservative amino acid substitutions) compared to an amino acid sequence set forth in SEQ ID NO: 80, 90, 97, or 98.

7. The heavy-chain antibody according to claim 4,
(i) comprising or consisting of an amino acid sequence having at least 85%, 90%, 91%, 92%, 93%, 94%, 95%, 96%, 97%, 98%, or 99% identity to an amino acid sequence selected from any one of SEQ ID NOs: 44-46 and 48-55;
(ii) comprising or consisting of an amino acid sequence selected from any one of SEQ ID NOs: 44-46 and 48-55; or
(iii) comprising an amino acid sequence having one or more (preferably not more than 10, and more preferably not more than 5, 4, 3, 2, or 1) amino acid modifications (preferably amino acid substitutions, and more preferably conservative amino acid substitutions) compared to an amino acid sequence selected from any one of SEQ ID NOs: 44-46 and 48-55, wherein preferably, the amino acid modifications do not occur in the CDRs.

8. The VHH antibody according to any one of claims 1-3 or the heavy-chain antibody according to any one of claims 4-7, wherein the antibody is a chimeric antibody or a humanized antibody.

9. A multispecific antibody, comprising a first antigen-binding region and a second antigen-binding region, and optionally a third antigen-binding region, wherein the second antigen-binding region specifically binds to BCMA, and comprises the VHH antibody according to any one of claims 1-3 and 8, or the heavy-chain antibody according to any one of claims 4-8, and preferably the multispecific antibody being a bispecific antibody or a trispecific antibody.

10. The multispecific antibody according to claim 9, wherein the first antigen-binding region specifically binds to CD3.

11. The multispecific antibody according to claim 10, wherein the first antigen-binding region comprises a VH and a VL, wherein the VH comprises three complementarity determining regions from a heavy chain variable region (HCDRs): HCDR1, HCDR2, and HCDR3, and the VL comprises three complementarity determining regions from a light chain variable region (LCDRs): LCDR1, LCDR2, and LCDR3, wherein
(i) the HCDR1, the HCDR2, and the HCDR3 are three complementarity determining regions HCDR1, HCDR2, and HCDR3 contained in a VH set forth in SEQ ID NO: 34, respectively; and the LCDR1, the LCDR2, and the LCDR3 are three complementarity determining regions LCDR1, LCDR2, and LCDR3 contained in a VL set forth in SEQ ID NO: 35, respectively; or
(ii) the HCDR1 consists of the amino acid sequence set forth in SEQ ID NO: 28, the HCDR2 consists of the amino acid sequence set forth in SEQ ID NO: 29, the HCDR3 consists of the amino acid sequence set forth in SEQ ID NO: 30, the LCDR1 consists of the amino acid sequence set forth in SEQ ID NO: 31, the LCDR2 consists of the amino acid sequence set forth in SEQ ID NO: 32, and the LCDR3 consists of the amino acid sequence set forth in SEQ ID NO: 33.

12. The multispecific antibody according to claim 11, wherein the first antigen-binding region comprises a VH and a VL, wherein
the VH comprises or consists of the amino acid sequence set forth in SEQ ID NO: 34, or an amino acid sequence having at least 90%, 91%, 92%, 93%, 94%, 95%, 96%, 97%, 98%, or 99% identity thereto, and/or
the VL comprises or consists of the amino acid sequence set forth in SEQ ID NO: 35, or an amino acid sequence having at least 90%, 91%, 92%, 93%, 94%, 95%, 96%, 97%, 98%, or 99% identity thereto.

13. The multispecific antibody according to any one of claims 10-12, wherein the first antigen-binding region is a Fab or an scFv specifically binding to CD3.

14. The multispecific antibody according to claim 13, wherein the Fab comprises a VH and a CH1 of the first antigen-binding region, and optionally a hinge region portion (e.g., EPKSS and EPKSC), wherein the CH1 is a CH1 from IgG1, IgG2, IgG3, or IgG4, preferably a CH1 from IgG1.

15. The multispecific antibody according to claim 14, wherein the CH1
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 88; or
(ii) comprises or consists of the amino acid sequence selected from SEQ ID NO: 88.

16. The multispecific antibody according to claim 15, wherein the Fab heavy chain of the first antigen-binding region
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 11;
(ii) comprises or consists of the amino acid sequence selected from SEQ ID NO: 11; or
(iii) comprises or consists of an amino acid sequence having one or more (preferably not more than 10 or 10, and more preferably not more than 5, 4, 3, 2, or 1) amino acid modifications (preferably amino acid substitutions, and more preferably conservative amino acid substitutions) compared to the amino acid sequence selected from SEQ ID NO: 11; and/or
the Fab light chain of the first antigen-binding region
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 60;
(ii) comprises or consists of the amino acid sequence selected from SEQ ID NO: 60; or
(iii) comprises or consists of an amino acid sequence having one or more (preferably not more than 10 or 10, and more preferably not more than 5, 4, 3, 2, or 1) amino acid modifications (preferably amino acid substitutions, and more preferably conservative amino acid substitutions) compared to the amino acid sequence selected from SEQ ID NO: 60.

17. The multispecific antibody according to claim 13, wherein the scFv comprises, from N-terminus to C-terminus, a heavy chain variable region VH of the first antigen-binding region, a linker, and a light chain variable region of the first antigen-binding region.

18. The multispecific antibody according to claim 17, wherein the scFv comprises the sequence set forth in SEQ ID NO: 109 or a sequence having at least 90%, 91%, 92%, 93%, 94%, 95%, 96%, 97%, 98%, 99% or more sequence identity and the same CDRs as compared to SEQ ID NO: 109.

19. The multispecific antibody according to any one of claims 9-18, wherein the second antigen-binding region is the VHH according to any one of claims 1-3 and 8.

20. The multispecific antibody according to any one of claims 9-19, wherein the multispecific antibody is a trispecific antibody and comprises a third antigen-binding region specifically binding to GPRC5D.

21. The multispecific antibody according to claim 20, wherein the third antigen-binding region comprises or consists of an anti-GPRC5D VHH.

22. The multispecific antibody according to claim 21, wherein the anti-GPRC5D VHH comprises or consists of a heavy chain variable region comprising
(i) three complementarity determining regions (CDRs) contained in a VH set forth in any one of SEQ ID NOs: 27 and 106, or
(ii) complementarity determining regions (CDRs) VHH CDR1, VHH CDR2, and VHH CDR3, wherein the VHH CDR1 comprises or consists of the amino acid sequence set forth in SEQ ID NO: 24, the VHH CDR2 comprises or consists of the amino acid sequence set forth in SEQ ID NO: 25, and the VHH CDR3 comprises or consists of the amino acid sequence set forth in SEQ ID NO: 26.

23. The multispecific antibody according to claim 22, wherein the heavy chain variable region
(i) comprises or consists of an amino acid sequence having at least 90%, 91%, 92%, 93%, 94%, 95%, 96%, 97%, 98%, or 99% identity to an amino acid sequence selected from any one of SEQ ID NOs: 27 and 106;
(ii) comprises or consists of an amino acid sequence selected from any one of SEQ ID NOs: 27 and 106; or
(iii) comprises an amino acid sequence having one or more (preferably not more than 10, and more preferably not more than 5, 4, 3, 2, or 1) amino acid modifications (preferably amino acid substitutions, and more preferably conservative amino acid substitutions) compared to an amino acid sequence selected from any one of SEQ ID NOs: 27 and 106, wherein preferably, the amino acid modifications do not occur in the CDRs.

24. The multispecific antibody according to claim 20, wherein the third antigen-binding region comprises a VH and a VL, wherein the VH comprises three complementarity determining regions from a heavy chain variable region (HCDRs): HCDR1, HCDR2, and HCDR3, and the VL comprises three complementarity determining regions from a light chain variable region (LCDRs): LCDR1, LCDR2, and LCDR3, wherein
(i) the HCDR1, the HCDR2, and the HCDR3 are three complementarity determining regions HCDR1, HCDR2, and HCDR3 contained in a VH set forth in SEQ ID NO: 42, respectively; and the LCDR1, the LCDR2, and the LCDR3 are three complementarity determining regions LCDR1, LCDR2, and LCDR3 contained in a VL set forth in SEQ ID NO: 43, respectively; or
(ii) the HCDR1 comprises or consists of the amino acid sequence set forth in SEQ ID NO: 36, the HCDR2 comprises or consists of the amino acid sequence set forth in SEQ ID NO: 37, the HCDR3 comprises or consists of the amino acid sequence set forth in SEQ ID NO: 38, the LCDR1 comprises or consists of the amino acid sequence set forth in SEQ ID NO: 39, the LCDR2 comprises or consists of the amino acid sequence set forth in SEQ ID NO: 40, and the LCDR3 comprises or consists of the amino acid sequence set forth in SEQ ID NO: 41.

25. The multispecific antibody according to claim 24, wherein the third antigen-binding region comprises a VH and a VL, wherein
the VH comprises or consists of the amino acid sequence set forth in SEQ ID NO: 42, or an amino acid sequence having at least 90%, 91%, 92%, 93%, 94%, 95%, 96%, 97%, 98%, or 99% identity thereto, and/or
the VL comprises or consists of the amino acid sequence set forth in SEQ ID NO: 43, or an amino acid sequence having at least 90%, 91%, 92%, 93%, 94%, 95%, 96%, 97%, 98%, or 99% identity thereto.

26. The multispecific antibody according to claim 24 or 25, wherein the third antigen-binding region is a Fab specifically binding to GPRC5D.

27. The multispecific antibody according to claim 26, wherein the Fab comprises a VH and a CH1 of the third antigen-binding region, and optionally the CH1 comprises a hinge region portion (e.g., EPKSS and EPKSC), wherein the CH1 is a CH1 from IgG1, IgG2, IgG3, or IgG4, preferably a CH1 from IgG1.

28. The multispecific antibody according to claim 27, wherein the CH1
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 88; or
(ii) comprises or consists of the amino acid sequence selected from SEQ ID NO: 88.

29. The multispecific antibody according to claim 28, wherein the Fab heavy chain of the third antigen-binding region
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 110;
(ii) comprises or consists of the amino acid sequence selected from SEQ ID NO: 110; or
(iii) comprises or consists of an amino acid sequence having one or more (preferably not more than 10 or 10, and more preferably not more than 5, 4, 3, 2, or 1) amino acid modifications (preferably amino acid substitutions, and more preferably conservative amino acid substitutions) compared to the amino acid sequence selected from SEQ ID NO: 110; and/or
the Fab light chain of the third antigen-binding region
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 62;
(ii) comprises or consists of the amino acid sequence selected from SEQ ID NO: 62; or
(iii) comprises or consists of an amino acid sequence having one or more (preferably not more than 10 or 10, and more preferably not more than 5, 4, 3, 2, or 1) amino acid modifications (preferably amino acid substitutions, and more preferably conservative amino acid substitutions) compared to the amino acid sequence selected from SEQ ID NO: 62.

30. The multispecific antibody according to any one of claims 9-29, wherein the multispecific antibody is an IgG-like bispecific antibody comprising an Fc dimer, wherein two Fc regions constituting the Fc dimer are identical or different.

31. The multispecific antibody according to claim 30, wherein the two Fc regions are different, and preferably, a corresponding knob mutation(s) and a corresponding hole mutation(s) are introduced into the two Fc regions, respectively.

32. The multispecific antibody according to claim 31, wherein
a) one Fc-region polypeptide comprises a knob mutation T366W, and the other Fc-region polypeptide comprises hole mutations T366S, L368A, and Y407V, or
b) one Fc-region polypeptide comprises knob mutations T366W and Y349C, and the other Fc-region polypeptide comprises hole mutations T366S, L368A, Y407V, and S354C, or
c) one Fc-region polypeptide comprises knob mutations T366W and S354C, and the other Fc-region polypeptide comprises hole mutations T366S, L368A, Y407V, and Y349C; and
optionally, the Fc region further comprises a mutation(s) that reduces binding to an Fcγ receptor, e.g., one or more of an L234A/L235A mutation, a D265A mutation, and a P329A mutation, for example, an L234A/L235A mutation, a D265A mutation, and a P329A mutation.

33. The bispecific antibody according to any one of claims 30-32, wherein one or both of the Fc regions comprise a hinge region, e.g., EPKSS (SEQ ID NO: 94) or EPKSC (SEQ ID NO: 96).

34. The multispecific antibody according to claim 32, wherein
(i) the Fc region comprising a knob mutation(s)
a) comprises or consists of an amino acid sequence set forth in SEQ ID NO: 47, 86, or 87; or
b) comprises or consists of an amino acid sequence having at least 90% identity, e.g., 95%, 96%, 97%, 99% or higher identity, to SEQ ID NO: 47, 86, or 87 and comprising a knob mutation(s) (e.g., S354C and T366W); and/or
(ii) the Fc region comprising a hole mutation(s)
a) comprises or consists of an amino acid sequence set forth in SEQ ID NO: 84, 85, or 111; or
b) comprises or consists of an amino acid sequence having at least 90% identity, e.g., 95%, 96%, 97%, 99% or higher identity, to SEQ ID NO: 84, 85, or 111 and comprising a hole mutation(s) (e.g., Y349C, T366S, L368A, and Y407V).

35. The multispecific antibody according to any one of claims 9-34, being a bispecific antibody and comprising a first antigen-binding region, a second antigen-binding region, and an Fc dimer, wherein the first antigen-binding region is an scFv fragment specifically binding to CD3, and the second antigen-binding region is a VHH specifically binding to BCMA, e.g., the VHH according to any one of claims 1-3 and 8.

36. The multispecific antibody according to claim 35, comprising one or two VHHs specifically binding to BCMA, one anti-CD3 scFv, and an Fc heterodimer, wherein
the scFv comprises a VH-VL (optionally the VH and VL are fused via a linker), and the VHH comprises or consists of a heavy chain variable region,
wherein the C-terminus of the VL of the scFv is fused to a CH2 or a hinge region of a first Fc region (e.g., comprising a knob mutation(s) or comprising a hole mutation(s)) to form a first heavy chain; and
the C-terminus of the one anti-BCMA VHH or of the two anti-BCMA VHHs in tandem is fused to a second Fc region (e.g., comprising a hole mutation(s) or comprising a knob mutation(s)) to form a second heavy chain (e.g., the C-terminus of the VHH is fused to a CH2 or a hinge region of the second Fc region).

37. The multispecific antibody according to claim 36, wherein
the first heavy chain comprises or consists of the amino acid sequence set forth in SEQ ID NO: 57,
or an amino acid sequence having at least 85%, 90%, 91%, 92%, 93%, 94%, 95%, 96%, 97%, 98%, or 99% identity thereto; and/or
the second heavy chain comprises or consists of an amino acid sequence set forth in any one of SEQ ID NOs: 56, 102, 103, and 104, or an amino acid sequence having at least 85%, 90%, 91%, 92%, 93%, 94%, 95%, 96%, 97%, 98%, or 99% identity thereto.

38. The multispecific antibody according to any one of claims 9-34, being a trispecific antibody and comprising a first antigen-binding region, a second antigen-binding region, a third antigen-binding region, and an Fc dimer, wherein the first antigen-binding region is an scFv fragment or a Fab fragment specifically binding to CD3, the second antigen-binding region is a VHH specifically binding to BCMA, e.g., the VHH according to any one of claims 1-3 and 8, and the third antigen-binding region is a VHH or a Fab fragment specifically binding to GPRC5D.

39. The multispecific antibody according to claim 38, comprising one anti-GPRC5D VHH, one anti-BCMA VHH, one anti-CD3 Fab fragment, and an Fc dimer, wherein
the Fab fragment comprises a VH-CH1 and a VL-CL, the VHH comprises a heavy chain variable region, and the trispecific antibody comprises or consists of a first heavy chain, a second heavy chain, and a light chain, wherein
the first heavy chain comprises, from N-terminus to C-terminus: the anti-GPRC5D VHH, the anti-BCMA VHH, and a first Fc region, which are fused with or without a linker(s); or the anti-BCMA VHH, the anti-GPRC5D VHH, and a first Fc region, which are fused with or without a linker(s);
the second heavy chain comprises, from N-terminus to C-terminus: the heavy chain of the anti-CD3 Fab fragment and a second Fc region, which are fused with or without a linker; and
the light chain comprises: the light chain of the anti-CD3 Fab fragment.

40. The multispecific antibody according to claim 39, wherein
the first heavy chain comprises or consists of the amino acid sequence set forth in SEQ ID NO: 58, or an amino acid sequence having at least 85%, 90%, 91%, 92%, 93%, 94%, 95%, 96%, 97%, 98%, or 99% identity thereto;
the second heavy chain comprises or consists of the amino acid sequence set forth in SEQ ID NO: 59, or an amino acid sequence having at least 85%, 90%, 91%, 92%, 93%, 94%, 95%, 96%, 97%, 98% or 99% identity thereto; and/or
the light chain comprises or consists of the amino acid sequence set forth in SEQ ID NO: 60, or an amino acid sequence having at least 85%, 90%, 91%, 92%, 93%, 94%, 95%, 96%, 97%, 98%, or 99% identity thereto.

41. The multispecific antibody according to claim 38, comprising one anti-GPRC5D Fab fragment, one anti-BCMA VHH, one anti-CD3 scFv, and an Fc dimer, wherein
the Fab fragment comprises a VH-CH1 and a VL-CL, the VHH comprises a heavy chain variable region, the scFv comprises a VH-VL, and the trispecific antibody comprises or consists of a first heavy chain, a second heavy chain, and a light chain, wherein
the first heavy chain comprises, from N-terminus to C-terminus: the anti-BCMA VHH and a first Fc region, which are fused with or without a linker;
the second heavy chain comprises, from N-terminus to C-terminus: the heavy chain of the anti-GPRC5D Fab fragment, the anti-CD3 scFv, and a second Fc region, which are fused with or without a linker; and
the light chain comprises: the light chain of the anti-GPRC5D Fab fragment.

42. The multispecific antibody according to claim 41, wherein
the first heavy chain comprises or consists of the amino acid sequence set forth in SEQ ID NO: 56, or an amino acid sequence having at least 85%, 90%, 91%, 92%, 93%, 94%, 95%, 96%, 97%, 98%, or 99% identity thereto;
the second heavy chain comprises or consists of an amino acid sequence set forth in SEQ ID NO: 61, or an amino acid sequence having at least 85%, 90%, 91%, 92%, 93%, 94%, 95%, 96%, 97%, 98%, or 99% identity thereto; and/or
the light chain comprises or consists of the amino acid sequence set forth in SEQ ID NO: 62, or an amino acid sequence having at least 85%, 90%, 91%, 92%, 93%, 94%, 95%, 96%, 97%, 98%, or 99% identity thereto.

43. A nucleic acid molecule, encoding the VHH antibody according to any one of claims 1-3 and 8, the heavy-chain antibody according to any one of claims 4-8 or any chain of the multispecific antibody according to any one of claims 9-42, or consisting of a nucleic acid sequence.

44. An expression vector, comprising the nucleic acid molecule according to claim 43.

45. A host cell, comprising the nucleic acid molecule according to claim 43 or the expression vector according to claim 44, wherein preferably, the host cell is prokaryotic or eukaryotic, e.g., a 293 cell or a CHO cell, such as a 293F cell or a 293T cell or a CHO-S cell.

46. A method for preparing the VHH antibody according to any one of claims 1-3 and 8, or the heavy-chain antibody according to any one of claims 4-8, or the multispecific antibody according to any one of claims 9-42, the method comprising culturing a host cell comprising the nucleic acid molecule according to claim 43 or the expression vector according to claim 44 under a condition suitable for expressing the chains of the antibody, and optionally recovering the antibody from the host cell (or a host cell culture medium).

47. An immunoconjugate, comprising the VHH antibody according to any one of claims 1-3 and 8, or the heavy-chain antibody according to any one of claims 4-8, or the multispecific antibody according to any one of claims 9-42.

48. A pharmaceutical composition or a medicament or a formulation, comprising the VHH antibody according to any one of claims 1-3 and 8, or the heavy-chain antibody according to any one of claims 4-8, or the multispecific antibody according to any one of claims 9-42, or the immunoconjugate according to claim 47, and optionally a pharmaceutical supplementary material.

49. A pharmaceutical combination product, comprising the VHH antibody according to any one of claims 1-3 and 8, or the heavy-chain antibody according to any one of claims 4-8, or the multispecific antibody according to any one of claims 9-42, or the immunoconjugate according to claim 47, and one or more additional therapeutic agents (e.g., a chemotherapeutic agent, a cytokine, a cytotoxic agent, an additional antibody, a small molecule drug, or an immunomodulatory agent).

50. A method for preventing or treating a cancer in a subject, comprising administering to the subject an effective amount of the VHH antibody according to any one of claims 1-3 and 8, or the heavy-chain antibody according to any one of claims 4-8, or the bispecific antibody according to any one of claims 9-42, or the immunoconjugate according to claim 47, or the pharmaceutical composition or the medicament or the formulation according to claim 48, or the pharmaceutical combination product according to claim 49.

51. The method according to claim 50, wherein tumor cells of the cancer have an elevated protein level and/or nucleic acid level (e.g., elevated expression) of BCMA.

52. The method according to claim 50 or 51, wherein the cancer is a solid tumor or a hematological tumor, such as myeloma, for example, multiple myeloma (e.g., metastatic multiple myeloma), colon cancer, rectal cancer, or colorectal cancer.

53. The method according to any one of claims 50-52, further comprising administering in combination with an additional therapy such as a therapeutic modality (e.g., surgical therapy or radiotherapy) and/or an additional therapeutic agent (e.g., a chemotherapeutic agent, a cytokine, a cytotoxic agent, an additional antibody, a small molecule drug, or an immunomodulatory agent).

54. A method for detecting the presence of BCMA in a biological sample, comprising
(i) contacting the biological sample with the VHH antibody according to any one of claims 1-3 and 8, or the heavy-chain antibody according to any one of claims 4-8, or the multispecific antibody according to any one of claims 9-42 under a condition which allows the antibody to bind to BCMA, and
(ii) detecting whether a complex is formed by the antibody or the bispecific antibody and the BCMA, wherein the formation of the complex indicates the presence of BCMA.
